# EUROPÄISCHE PATENTANMELDUNG

(11) **EP 4 393 581 A2**
(43) Veröffentlichungstag der Anmeldung: **03.07.2024**
(21) Anmeldenummer: 24170337.0
(22) Anmeldetag: 06.08.2020
(51) Int. Cl.: B01J 13/20

(54) **BIOABBAUBARE POLYHARNSTOFF/POLYURETHAN- MIKROKAPSELN**

(62) Teilanmeldung aus: 20754224.2
(71) Anmelder: Symrise AG, 37603 Holzminden (DE)
(72) Erfinder: GEORGI, Julian Alexander, 37603 Holzminden (DE); RAABE, Britta, 37603 Holzminden (DE); ROST, Benjamin, 37619 Bodenwerder (DE); VOGEL, Andreas, 37627 Stadtoldendorf (DE); BERTRAM, Ralf, 37603 Holzminden (DE); GREGOR, Daniela, 37639 Bevern (DE); KOEPKE, Christina, 37603 Holzminden (DE); BARGSTEN, Stefanie, 32839 Steinheim (DE)
(74) Vertreter: Global IP Europe Patentanwaltskanzlei

(57) **Zusammenfassung**

Die vorliegende Erfindung betrifft ein Verfahren zur Herstellung von bioabbaubaren Polyharnstoff/Polyurethan-Mikrokapseln, vorzugsweise von parfümhaltigen Polyharnstoff/Polyurethan-Mikrokapseln, welche eine verbesserte Bioabbaubarkeit gegenüber Mikrokapseln des Stands der Technik aufweisen. Darüber hinaus betrifft die vorliegende Erfindung bioabbaubare Polyharnstoff/Polyurethan-Mikrokapseln, umfassend mindestens einen lipophilen Wirkstoff, die nach dem erfindungsgemäßen Verfahren erhältlich sind. In einem weiteren Aspekt betrifft die hierin beschriebene Erfindung die Verwendung derartiger Mikrokapseln oder Mikrokapsel-Dispersionen, die die erfindungsgemäßen Mikrokapseln umfassen, zur Herstellung von Haushaltsprodukten, Textilpflegeprodukten, Waschmitteln, Weichspülern, Reinigungsmitteln, Scent Boostern, Scent Lotions oder Duftverstärkern, Kosmetika, Körperpflegeprodukten, Agrarprodukten, pharmazeutischen Produkten oder Druckbeschichtungen für Papier. Letztendlich betrifft die vorliegende Erfindung Verbraucherprodukte, die derartige Mikrokapseln oder Mikrokapsel-Dispersionen umfassen.

## Beschreibung

Die vorliegende Erfindung betrifft ein Verfahren zur Herstellung von bioabbaubaren Polyharnstoff/Polyurethan-Mikrokapseln, die mindestens einen lipophilen Wirkstoff einschließen, vorzugsweise von parfüm- oder aromahaltigen Polyharnstoff/Polyurethan-Mikrokapseln, welche eine ausgewogene Balance aus Bioabbaubarkeit, Stabilität und Performance gegenüber Mikrokapseln des Stands der Technik aufweisen. Darüber hinaus betrifft die vorliegende Erfindung bioabbaubare Polyharnstoff/Polyurethan-Mikrokapseln, umfassend mindestens einen lipophilen Wirkstoff, die nach dem erfindungsgemäßen Verfahren erhältlich sind. In einem weiteren Aspekt betrifft die hierin beschriebene Erfindung die Verwendung derartiger Mikrokapseln oder Mikrokapsel-Dispersionen, die die erfindungsgemäßen Mikrokapseln umfassen, zur Herstellung von Haushaltsprodukten, Textilpflegeprodukten, Waschmitteln, Weichspülern, Reinigungsmitteln, Scent Boostern, Scent Lotions oder Duftverstärkern, Kosmetika, Körperpflegeprodukten, Agrarprodukten, pharmazeutischen Produkte oder Druckbeschichtungen für Papier. Letztendlich betrifft die vorliegende Erfindung Verbraucherprodukte, die derartige Mikrokapseln oder Mikrokapsel-Dispersionen umfassen.

Mikrokapseln sind Partikel, die aus einem Kern und einem den Kern umgebenden Wandmaterial bestehen, wobei der Kern eine feste, flüssige oder gasförmige Substanz sein kann, der von einem polymeren dichten, permeablen oder semipermeablen Wandmaterial umgeben ist. Bei der Herstellung schlagen sich die Polymer aus den Ausgangsbestandteilen nach Emulgierung und Koazervation oder Grenzflächenpolymerisation auf den zu verkapslenden Substanzen nieder, welche dadurch immobilisiert werden. Der Kern wird auch als innere Phase bezeichnet. Für die Wand werden auch Namen wie äußere Phase, Hülle oder Überzug verwendet. Der Durchmesser der Mikrokapseln schwankt typischerweise im Bereich von 1 bis 1000 µm. Die Wandstärke beträgt typischerweise 0,5 bis 150 µm. Typischerweise sind Beladungen von 25 bis 95 Gew.-%, aber auch solche von 1 bis 99 Gew.-% möglich.

Ziel der Verkapselung ist unter anderem, die eingekapselten Substanzen bzw.Wirkstoffe zu schützen, sie zu einem bestimmten Zeitpunkt gezielt freizusetzen, die Überführung von Flüssigkeiten in eine handhabbare Pulverform, die Verzögerung von Verlusten flüchtiger Komponenten (z.B. bei Duft- oder Aromastoffen), die Verhinderung von vorzeitigen chemischen Reaktionen mit anderen Mischungskomponenten oder ein besseres Handling vor oder während der Verarbeitung zu gewährleisten. Lipophile bzw. hydrophobe Wirkstoffe, wie beispielsweise Duft- oder Aromastoffe, lassen sich durch Verkapselung leicht in zahlreiche und verschiedene Anwendungsformulierungen einarbeiten.

Der Inhalt von Mikrokapseln kann auf verschiedene Weisen freigesetzt werden und beruht insbesondere auf einem der nachfolgend beschriebenen Mechanismen: mechanische Zerstörung der Kapsel durch Zerdrücken oder Scherung; Zerstörung der Kapsel durch Schmelzen des Wandmaterials, Zerstörung der Kapseln durch Auflösen des Wandmaterials oder Diffusion der Wirkstoffe durch die Kapselwand.

Für die Herstellung von Mikrokapseln ist eine Vielzahl von Schalenmaterialien bekannt. Die Schale kann entweder aus natürlichen, halbsynthetischen oder synthetischen Materialien bestehen. Natürliche Schalenmaterialien sind beispielsweise Gummi arabicum, Agar-Agar, Agarose, Maltodextrine, Alginsäure oder deren Salze, z.B. Natriumalginat oder Calciumalginat, Fette und Fettsäuren, Cetylalkohol, Kollagen, Chitosan, Lecithine, Gelatine, Albumin, Schellack, Polysaccharide wie Stärke oder Dextran, Polypeptide, Proteinhydrolysate, Saccharose und Wachse. Semisynthetische Hüllenmaterialien sind unter anderem chemisch modifizierte Cellulosen, insbesondere Celluloseester und Celluloseether, z.B. Celluloseacetat, Ethylcellulose, Hydroxypropylcellulose, Hydroxypropylmethylcellulose und Carboxymethylcellulose sowie Stärkederivate, insbesondere Stärkeether und Stärkeester. Synthetische Schalenmaterialien sind beispielsweise Polymere wie Polyacrylate, Polyamide, Polyvinylalkohol oder Polyvinylpyrrolidon.

Je nach Art des Schalenmaterials und Herstellungsverfahrens entstehen jeweils Mikrokapseln mit unterschiedlichen Eigenschaften hinsichtlich Durchmesser, Größenverteilung sowie physikalischen und/oder chemischen Eigenschaften.

Polyharnstoff-Mikrokapseln oder Polyharnstoff/Polyurethan-Mikrokapseln, die durch Polymerisation zwischen einem Polyisocyanat und einem Polyamin und/oder einem Diol oder Polyol gebildet werden, sind bekannte Kapseln, die in einer Vielzahl von technischen Bereichen, einschließlich Parfümerie, verwendet werden.

Polyharnstoff-Mikrokapseln, die durch Reaktion von zwei Polyisocyanaten und einem Polyamin erhalten werden, sind beispielsweise in der WO 2011/161229 oder WO 2011/160733 beschrieben. Gemäß WO 2011/161229 oder WO 2011/160733 werden die Polyharnstoff-Mikrokapseln in Gegenwart von Polyvinylpyrrolidon (PVP) als Schutzkolloid hergestellt. WO 2012/107323 offenbart Polyharnstoff-Mikrokapseln mit einer Polyharnstoffhülle, die das Reaktionsprodukt eines Polyisocyanats mit Guanazol (3,5-Diamino)-1,2,4-triazol) und einer Aminosäure in Gegenwart von anionischen Stabilisatoren oder Tensiden wie anionischem Polyvinylalkohol umfasst. Die EP 0 537 467 B beschreibt Mikrokapseln, die aus Polyisocyanaten hergestellt wurden, die Polyethylenoxidgruppen enthalten, in Gegenwart von Stabilisatoren wie Polyvinylalkohol. Gemäß WO 2007/096592 kann die Mikroverkapselung in einer Ölphase erfolgen, die in einer kontinuierlichen wässrigen Phase emulgiert wird, die im Allgemeinen durch ein Tensidsystem wie Polyvinylalkohole oder carboxylierte und sulfonierte Derivate davon stabilisiert wird.

Die oben beschriebenen beispielhaften Abgabesysteme aus dem Stand der Technik weisen sowohl eine gute Stabilität, nämlich die Fähigkeit zur Retention des Wirkstoffs und damit die Fähigkeit der Kapseln, den Verlust der flüchtigen Bestandteile zu vermeiden, als auch eine gute Performance, beispielsweise Duftstofffreisetzung im Falle von Duftstoffkapseln, auf.

Die oben beschriebenen Mikrokapseln des Standes der Technik haben jedoch den Nachteil, dass das polymere Kapselwand- oder Kapselhüllmaterial einen großen Polymer-Anteil benötigt, um eine ausreichende Stabilität zu gewährleisten und keine allzu großen Wirkstoffverluste zu erleiden. Außerdem bringt die Mikroverkapselung Kunststoff in die Umwelt, der dort als "Mikroplastik" Probleme verursachen kann.

Weil Kunststoffpartikel hinsichtlich ihrer Umweltbelastung zunehmend in der öffentlichen Kritik stehen, und die Nachfrage nach biobasierten und biologisch abbaubaren Lösungen aufgrund des stetig steigenden gesellschaftlichen Drucks im Hinblick umwelttechnischer Gesichtspunkte wächst, besteht für die Mikroverkapselung ein Bedarf, neue Materialien zu entwickeln, um eine Reduzierung von Mikroplastik in der Umwelt zu erreichen. Biobasierte und bioabbaubare Werkstoffe stehen dabei im Fokus.

Vor diesem Hintergrund besteht daher ein Bedarf an der Bereitstellung von Polyharnstoff/Polyurethan-Mikrokapseln mit einer herausragenden Stabilität und herausragenden Freisetzungseigenschaften für die jeweiligen Anwendungen einerseits, und Polyharnstoff/Polyurethan-Mikrokapseln, die überwiegend oder nahezu vollständig bioabbaubar sind, andererseits.

Diese Aufgabe, mit Hilfe von bioabbaubaren Werkstoffen die Menge der Mikrokunststoffe in der Umwelt zu reduzieren, ist jedoch im Fall von Mikroverkapselungen nicht trivial, da die gewünschte Funktionalität der Mikrokapsel, wie beispielsweise olfaktorische Eigenschaften und positive sekundäre Eigenschaften wie beispielsweise hohe Stabilität, toxikologische Stabilität, in vielen Anwendungen mit den Anforderungen an eine schnelle biologische Abbaubarkeit im Widerspruch steht.

Es ist besonders schwierig, Mikrokapseln herzustellen, die sowohl eine gute Stabilität als auch eine gute Wirkstoff-Freisetzung aufweisen. Die Fähigkeit zur Wirkstoffretention und damit die Fähigkeit der Kapseln, den Verlust der flüchtigen Bestandteile zu vermeiden, hängt insbesondere von der Stabilität der Kapseln in der Produktbasis ab. Insbesondere Kapseln mit einer guten Stabilität weisen jedoch nicht automatisch eine gute Bioabbaubarkeit auf.

Mit steigendem Vernetzungsgrad erhöht sich die Stabilität der Mikrokapseln, gleichzeitig sinkt aber auch die Fähigkeit zum biologischen Abbau der Kapselhülle. Bei sehr stabilen Mikrokapseln ist die Performance, beispielsweise sensorische Performance, geringer, da die Anzahl der Mikrokapseln, welche durch Druck, Reibung, etc. aufbrechen und Wirkstoffe freigeben, sinkt. Sind sie zu instabil, werden sie bereits während der Lagerung zerstört und performen ebenfalls nicht.

Vor diesem Hintergrund lag der vorliegenden Erfindung die komplexe Aufgabe zugrunde, ein Verfahren zur Herstellung von Mikrokapseln bereitzustellen, welches es zum einen ermöglicht, mit einem geringeren Polymer-Anteil Mikrokapseln bereitzustellen, welche gleichzeitig eine hohe Stabilität als auch ein ausgezeichnetes Freisetzungsverhalten der verkapselten Wirkstoffe zeigen und möglichst bioabbaubare Eigenschaften aufweisen.

Überraschendenderweise wurde gefunden, dass diese Aufgabe dadurch gelöst werden kann, dass die Polyharnstoff/Polyurethan-Mikrokapseln unter Verwendung von Aminosäuren und eines Trennmittels, welches sich in die Mikrokapsel-Hülle einlagert, hergestellt werden.

### Zusammenfassung der Erfindung

Die vorliegende Problemstellung wird durch die Gegenstände der unabhängigen Patentansprüche gelöst. Bevorzugte Ausgestaltungen ergeben sich aus dem Wortlaut der abhängigen Patentansprüche sowie der nachfolgenden Beschreibung.

Ein erster Gegenstand der vorliegenden Erfindung betrifft daher ein Verfahren zur Herstellung einer bioabbaubaren Polyharnstoff/Polyurethan-Mikrokapsel, welches die folgenden Schritte in dieser Reihenfolge umfasst:
(a) Durchführung eines ersten Polymerisations- und/oder Vernetzungsschrittes, umfassend:
   (a1) Bereitstellen einer internen nicht-wässrigen Phase, umfassend mindestens ein Polyisocyanat mit zwei oder mehreren Isocyanat-Gruppen und mindestens einen zu verkapselnden lipophilen Wirkstoff;
   (a2) Bereitstellen einer externen wässrigen Phase, umfassend mindestens ein Schutzkolloid und optional einen Emulgator;
   (a3) Vermischen der internen nicht-wässrigen Phase und der externen wässrigen Phase unter Erhalt einer Öl-in-Wasser-Emulsion;
   (a4) Zugabe mindestens einer ersten Aminosäure oder eines Aminosäure-Hydrochlorids und eines Katalysators;
(b) Durchführen eines zweiten Polymerisations- und/oder Vernetzungsschrittes durch Zugabe mindestens eines Hydroxylgruppen-Donors;
(c) Durchführen eines dritten Polymerisations- und/oder Vernetzungsschrittes durch Zugabe mindestens einer zweiten Aminosäure, insbesondere bei einer Temperatur von mindestens 60 °C, unter Erhalt einer Mikrokapsel-Dispersion;
(d) Aushärten der Mikrokapsel-Dispersion bei einer Temperatur von mindestens 60°C für eine Zeitdauer von mindestens 60 Minuten;
(e) Zugabe mindestens eines Trennmittels und Einlagerung des Trennmittels in die Mikrokapselhülle;
(f) Nach-Härten der in Schritt (e) erhaltenen Mikrokapsel;
und optional:
(g) Abtrennen der Mikrokapsel von der Mikrokapsel-Dispersion und gegebenenfalls Trocknen der Mikrokapsel oder Einstellen der Viskosität der Mikrokapsel-Slurry durch Zugabe eines Verdickungsmittels.

Außerdem ist Gegenstand der hierin beschriebenen Erfindung eine bioabbaubare Polyharnstoff/Polyurethan-Mikrokapsel, umfassend mindestens einen lipophilen Wirkstoff, die nach dem erfindungsgemäßen Verfahren herstellt wird.

Ein weiterer Aspekt der vorliegenden Erfindung ist eine bioabbaubare Polyharnstoff/Polyurethan-Mikrokapsel, umfassend
(i) einen Kern, umfassend mindestens einen hydrophoben Wirkstoff; und
(ii) eine Kapselhülle, umfassend:
   - ein Reaktionsprodukt aus einer Polymerisation und/oder Vernetzung von mindestens einem Polyisocyanat mit zwei oder mehreren Isocyanat-Gruppen, in Anwesenheit mindestens eines Schutzkolloids mit mindestens einer ersten Aminosäure oder eines Aminosäure-Hydrochlorids, einer weiteren Polymerisation und/oder Vernetzung mit mindestens einem Hydroxylgruppen-Donor, und einer noch weiteren Polymerisation und/oder Vernetzung mit mindestens einer zweiten Aminosäure; und
   - mindestens ein Trennmittel.

Letztlich betrifft die vorliegende Erfindung in einem weiteren Aspekt die Verwendung der bioabbaubaren Polyharnstoff/Polyurethan-Mikrokapsel oder einer Dispersion aus den erfindungsgemäßen Polyharnstoff/Polyurethan-Mikrokapseln zur Herstellung von Haushaltsprodukten, Textilpflegeprodukten, Waschmitteln, Weichspülern, Reinigungsmitteln, Scent Boostern, Scent Lotions oder Duftverstärkern, Kosmetika, Körperpflegeprodukten, Parfümzusammensetzungen, Agrarprodukten, pharmazeutischen Produkten oder Druckbeschichtungen für Papier sowie die daraus hergestellten Verbraucherprodukte.

Überraschend wurde im Rahmen der vorliegenden Erfindung gefunden, dass bei der Herstellung der Mikrokapseln die Kombination aus gezielter Polymerisation und/oder Vernetzung von Polyisocyanaten mit mindestens zwei oder mehreren Isocyanat-Gruppen mit einer ersten Aminosäure oder eines Aminosäure-Hydrochlorids, anschließende Polymerisation und/oder Vernetzung mit einem Hydroxylgruppen-Donor und einer noch weiteren Polymerisation und/oder Vernetzung mit einer zweiten Aminosäure und der Zusatz von einem Trennmittel und Einlagerung des Trennmittels in die Mikrokapselhülle zu stabilen Mikrokapseln führt und somit eine effiziente Verkapselung von Wirkstoffen mit anschließend gezielter Freisetzung dieser Wirkstoffe gewährleistet werden kann, während die Mikrokapeseln gleichzeitig eine gute biologische Abbaubarkeit aufgrund ihrer biobasierten und biologisch abbaubaren Bausteine, wie beispielsweise Aminosäuren und Trennmittel, aufweisen.

Durch die Verwendung von Aminosäuren und eines Trennmittels kann außerdem der Polymer-Anteil des Kapselwand- oder Kapselhüllmaterials reduziert werden, ohne dass darunter die Stabilität der Mikrokapselwand leidet. Durch die Einlagerung des Trennmittels in die Mikrokapsel-Hülle wird darüber hinaus die biologische Abbaubarkeit des Kapselwand- oder Kapselhüllmaterials erleichtert.

Diese und weitere Aspekte, Merkmale und Vorteile der vorliegenden Erfindung werden für den Fachmann aus dem Studium der folgenden detaillierten Beschreibung und der Patentansprüche ersichtlich. Dabei kann jedes Merkmal aus einem Aspekt der Erfindung in einem anderen Aspekt der Erfindung eingesetzt oder ausgetauscht werden. Die hierin enthaltenen Beispiele veranschaulichen die Erfindung, ohne diese einzuschränken.

Die Begriffe "mindestens ein" oder "wenigstens ein" oder "ein oder mehrere", wie hierin verwendet, bezieht sich auf 1 oder mehr, beispielsweise 2, 3, 4, 5, 6, 7, 8, 9 oder mehr.

Der Begriff "und/oder" drückt aus, dass eine Verknüpfung vorliegt oder eine Alternative angeboten wird.

Nummerische Beispiele, die in der Form "x bis y" angegeben sind, schließen die genannten Werte ein. Wenn mehrere bevorzugte numerische Bereiche in diesem Format angegeben sind, sind alle Bereiche, die durch die Kombination der verschiedenen Endpunkte entstehen, ebenfalls erfasst.

### Figuren

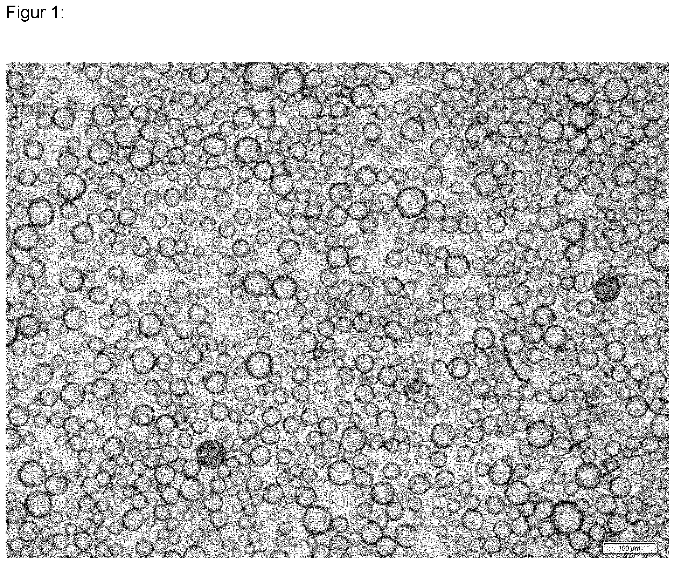

Figur 1 ist eine lichtmikroskopische Aufnahme der erfindungsgemäßen Mikrokapseln. Die Mikrokapseln wurden hergestellt aus Hexamethylendiisocyanat und 4,4'-Methyldiphenylendiisocyanat in einem Verhältnis von 75 : 25. Des Weiteren wurden als erste Aminosäure Lysin*HCl, als Hydroxylgruppen-Donor Glycerin und als zweite Aminosäure Arginin verwendet. Als Katalysator wurde DABCO verwendet und als Schutzkolloid eine modifizierte Stärke; dazu wurde Bienenwachs als Trennmittel gegeben. Für die lichtmikroskopische Aufnahme wurde ein Olympus BX51 verwendet. Der dargestellte Balken entspricht dabei 100 µm.

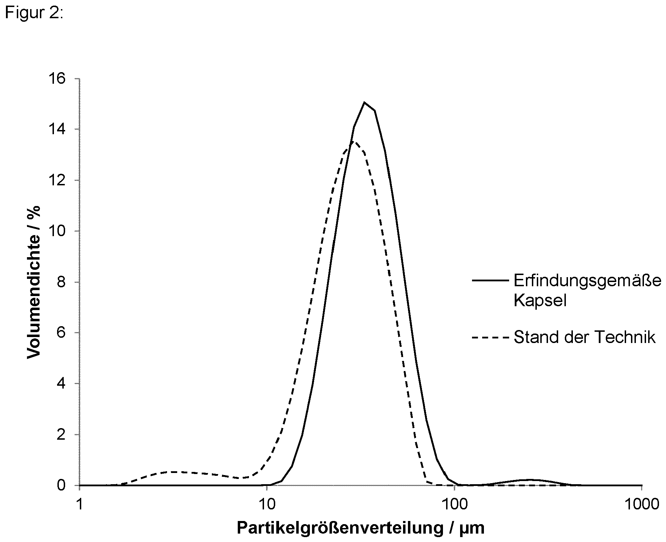

Figur 2 zeigt ein Diagramm der Partikelgrößenverteilung (d(0,5)-Wert) von erfindungsgemäßen Mikrokapseln und Mikrokapseln des Stands der Technik basierend auf Polyharnstoff/Polyurethan-Strukturen ohne Trennmittel. Für die Bestimmung der Partikelgrößenverteilung wurde ein MALVERN Mastersizer 3000 verwendet. Die entsprechende Berechnung basiert auf der Mie-Theorie.

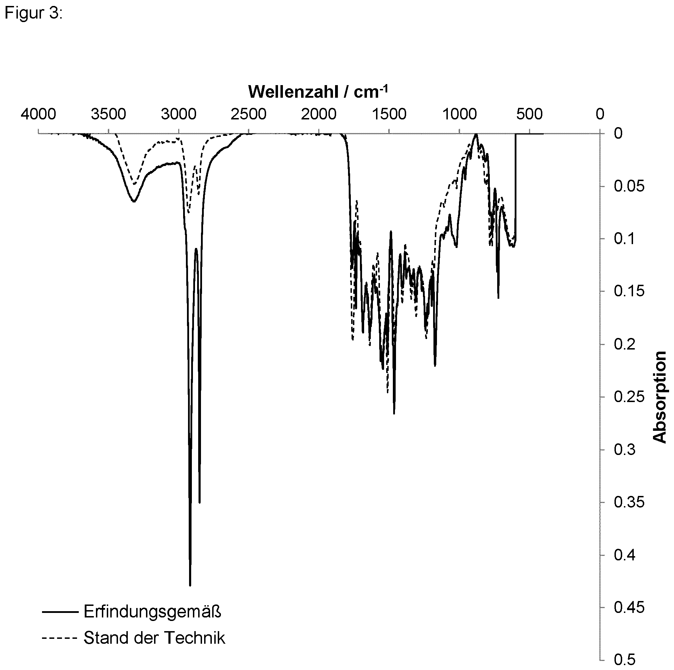

Figur 3 ist ein Diagramm, das die Ergebnisse einer IR-spektroskopischen Analyse von erfindungsgemäßen Mikrokapseln und Mikrokapseln des Standes der Technik, d. h. von Mikrokapseln basierend auf Polyharnstoff/Polyurethan-Strukturen ohne Trennmittel, zeigt. Die Analyse erfolgte mittels ATR-Infrarotspektroskopie (engl. Attenuated Total Reflection). N.B. In Figur 3 sind die Dezimalstellen in der Absorptionsachse mit einem Punkt statt Komma als Dezimaltrennzeichen gekennzeichnet.

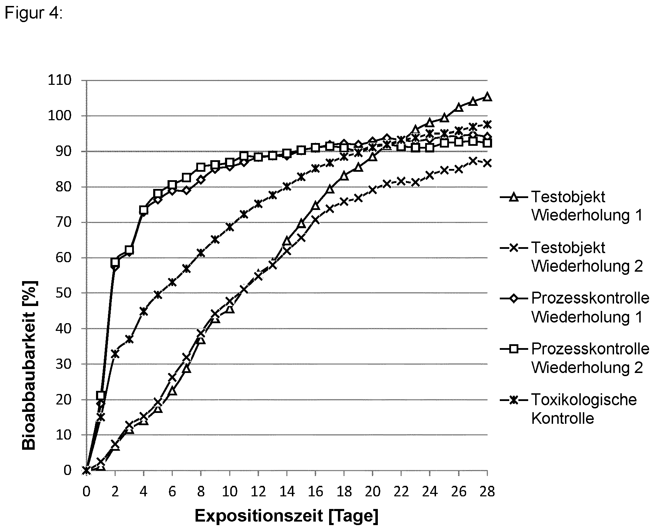

Figur 4 ist ein Diagramm, das die biologische Abbaubarkeit von erfindungsgemäßen Mikrokapseln gemäß OECD 301 F darstellt im Vergleich zu Natriumbenzoat und einer Toxizitätskontrolle (Mischung der erfindungsgemäßen Mikrokapseln und Natriumbenzoat).

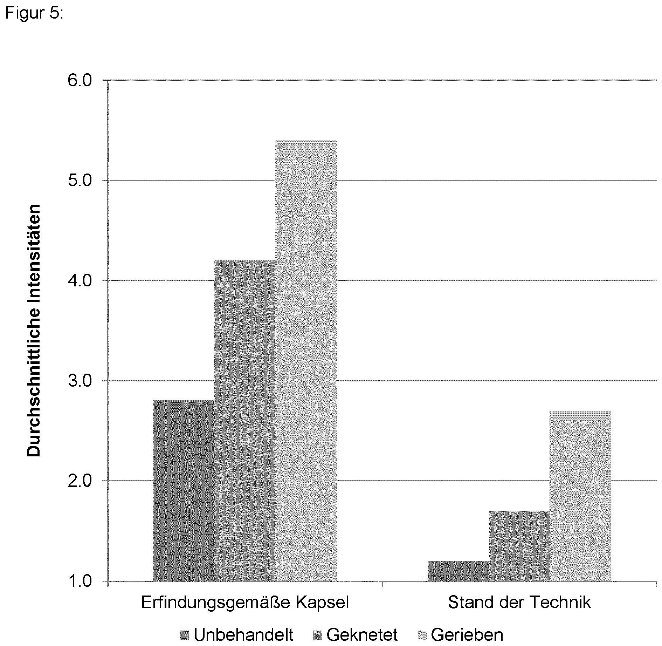

Figur 5 ist ein Diagramm, das die Ergebnisse einer sensorischen Evaluierung von erfindungsgemäßen Mikrokapseln und Mikrokapseln des Standes der Technik, d. h. von Mikrokapseln basierend auf Polyharnstoff/Polyurethan-Strukturen ohne Trennmittel, zeigt. N.B. In Figur 5 sind die Dezimalstellen in der Intensitäts-Achse mit einem Punkt statt Komma als Dezimaltrennzeichen gekennzeichnet.

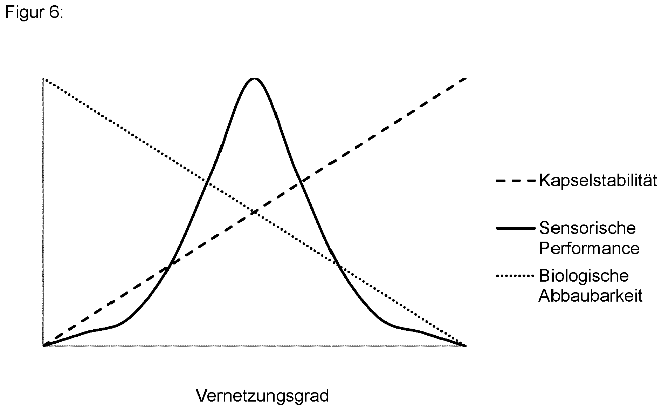

Figur 6 ist ein Diagramm, das allgemein die Korrelation zwischen Mikrokapselstabilität, Performance und Bioabbaubarkeit in Abhängigkeit vom Vernetzungsgrad zeigt.

### Detaillierte Beschreibung der Erfindung

In einem ersten Aspekt betrifft die vorliegende Erfindung ein Verfahren zur Herstellung einer bioabbaubaren Polyharnstoff/Polyurethan-Mikrokapsel, vorzugsweise ein Verfahren zur Herstellung einer Duftstoffkapsel, welches die folgenden Schritte in dieser Reihenfolge umfasst:
(a) Durchführung eines ersten Polymerisations- und/oder Vernetzungsschrittes, umfassend:
   (a1) Bereitstellen einer internen nicht-wässrigen Phase, umfassend mindestens ein Polyisocyanat mit zwei oder mehreren Isocyanat-Gruppen und mindestens einen zu verkapselnden lipophilen Wirkstoff;
   (a2) Bereitstellen einer externen wässrigen Phase, umfassend mindestens ein Schutzkolloid und optional einen Emulgator;
   (a3) Vermischen der internen nicht-wässrigen Phase und der externen wässrigen Phase unter Erhalt einer Öl-in-Wasser-Emulsion;
   (a4) Zugabe mindestens einer ersten Aminosäure oder eines Aminosäure-Hydrochlorids und eines Katalysators;
(b) Durchführen eines zweiten Polymerisations- und/oder Vernetzungsschrittes durch Zugabe mindestens eines Hydroxylgruppen-Donors;
(c) Durchführen eines dritten Polymerisations- und/oder Vernetzungsschrittes durch Zugabe mindestens einer zweiten Aminosäure, insbesondere bei einer Temperatur von mindestens 60 °C, unter Erhalt einer Mikrokapsel-Dispersion;
(d) Aushärten der Mikrokapsel-Dispersion bei einer Temperatur von mindestens 60 °C für eine Zeitdauer von mindestens 60 Minuten;
(e) Zugabe mindestens eines Trennmittels und Einlagerung des Trennmittels in die Mikrokapselhülle;
(f) Nach-Härten der in Schritt (e) erhaltenen Mikrokapsel;
und optional:
(g) Abtrennen der Mikrokapsel von der Mikrokapsel-Dispersion und gegebenenfalls Trocknen der Mikrokapsel oder Einstellen der Viskosität der Mikrokapsel-Slurry durch Zugabe eines Verdickungsmittels.

Unter Mikrokapseln werden im Kontext der vorliegenden Erfindung Mikropartikel verstanden, die eine Kapselhülle bzw. Kapselwand und mindestens einen oder mehrere Wirkstoffe als Kernmaterial im Inneren der Kapsel aufweisen. Bei den Wirkstoffen handelt es sich vorzugsweise um lipophile bzw. hydrophobe Wirkstoffe. Derartige Wirkstoffe sind nich oder schlecht in Wasser, hingegen gut in Fetten und Ölen löslich. Die Begriffe "Mikrokapsel" bzw. "Kapsel" und "lipophil" bzw. "hydrophob" werden im Sinne der vorliegenden Erfindung synonym verwendet.

Die Kapselhülle bzw. Kapselwand ist im Rahmen der vorliegenden Erfindung vorzugsweise aus mehreren Vernetzungsmatrices bzw. Vernetzungseinheiten aufgebaut, welche vorzugsweise unterschiedliche Zusammensetzungen aufweisen und durch mehrere Verfahrensschritte bzw. Verfahrenssequenzen, insbesondere Vernetzungsschritte, bei der Herstellung der erfindungsgemäßen Mikrokapsel generiert werden, so dass ein dreidimensionales Netzwerk entsteht.

Bei einer Vernetzungsmatrix bzw. Vernetzungseinheit im Kontext der vorliegenden Erfindung handelt es sich um einen Verbund oder ein Netzwerk aus Ausgangsbestandteilen zum Aufbau der Mikrokapsel-Hülle, der/das durch lineare oder dreidimensionale Polymerisation und/oder Vernetzung zwischen funktionellen Gruppen der Ausgangsbestandteile und/oder mit anderen Bestandteilen der Mikrokapsel-Hülle aufgebaut wird und/oder in den andere Bestandteile der Mikrokapsel-Hülle eingebettet sind. Mehrere Vernetzungsmatrices können im Verlauf des erfindungsgemäßen Verfahrens wiederum durch weitere Vernetzung miteinander vernetzt werden und eine dreidimensionale Struktur zum Aufbau der Mikrokapsel-Hülle bzw. Mikrokapsel-Wand ausbilden. Die Vernetzungseinheiten bzw. Vernetzungsmatrices bilden in ihrer Gesamtheit die Kapselhülle bzw. Kapselwand.

In einer noch mehr bevorzugten Variante der vorliegenden Erfindung umfasst die Kapselhülle bzw. Kapselwand wenigstens Polyharnstoff- und Polyurethan-Vernetzungsmatrices oder -Vernetzungseinheiten und ein Trennmittel, das in die Kapselhülle bzw. Kapselwand eingelagert ist.

In einem ersten Schritt (a) des erfindungsgemäßen Verfahrens wird eine erste Polymerisation und/oder Vernetzung (a) durchgeführt. Hierzu wird eine interne nicht-wässrige Phase bereitgestellt (a1), welche mindestens ein Isocyanat oder ein Polyisocyanat mit zwei oder mehreren Isocyanat-Gruppen und mindestens einen zu verkapselnden lipophilen Wirkstoff umfasst.

Die Polyharnstoff/Polyurethan-Mikrokapseln gemäß der vorliegenden Erfindung werden unter Verwendung mindestens eines oder mehrerer Polyisocyanate hergestellt.

Das mindestens eine Isocyanat oder Polyisocyanat mit zwei oder mehreren Isocyanat-Gruppe, welches in dem erfindungsgemäßen Verfahren zur Herstellung einer bioabbaubaren Polyharnstoff/Polyurethan-Mikrokapsel eingesetzt wird, weist mindestens zwei Isocyanat-Gruppen zur Ausbildung polymerer Netzwerke durch Polymerisation auf, die eine Kapselhülle bzw. Kapselwand ausbilden.

Polyisocyanate sind R-substituierte organische Derivate (R-N=C=O) von Isocyaninsäure (HN=C=O). Organische Isocyanate sind Verbindungen, bei denen die Isocyanat-Gruppe (-N=C=O) an ein organisches Radikal gebunden ist. Polyfunktionelle Isocyanate oder Polyisocyanate sind solche Verbindungen, die mindestens zwei oder mehrere, d.h. 3, 4, 5, 6, 7, 8, 9, 10, 11, 12, 13, 14, 15, 20, 50, 100, 200 oder noch mehr, Isocyanat-Gruppen (-N=C=O) im Molekül enthalten. Polyisocyanate mit zwei Isocyanat-Gruppen werden auch als Diisocyanate bezeichnet.

Polyisocyanate können in aliphatische, cycloaliphatische, hydroaromatische, aromatische oder heterocyclische Isocyanate bzw. Polyisocyanate eingeteilt werden. Daneben können die erfindungsgemäßen Polyisocyanate linear oder verzweigt sein.

Polyisocyanate, insbesondere aromatische Polyisocyanate, sind hochreaktive Verbindungen. Die Polyadditions-Reaktionen von Polyisocyanaten mit Diolen oder Polyolen sind die Grundlage der Polyurethan-Chemie und die Polyadditions-Reaktionen von Polyisocyanaten mit Aminen sind die Grundlage der Polyharnstoff-Chemie.

Erfindungsgemäß werden mindestens difunktionelle, vorzugsweise polyfunktionelle Polyisocyanate verwendet, d.h. alle aliphatischen, alicyclischen und aromatischen Isocyanate sind geeignet, sofern sie mindestens zwei reaktive Isocyanatgruppen aufweisen.

Besonders bevorzugt sind aliphatische, cycloaliphatische, hydroaromatische, aromatische oder heterocyclische Polyisocyanate, deren Substitutionsprodukte sowie Mischungen aus den vorgenannten monomeren oder oligomeren Verbindungen. Von den zuvor spezifizierten Polyisocyanaten finden aliphatische und/oder aromatische Verbindungen bevorzugt Verwendung.

In bevorzugten Ausführungsformen des erfindungsgemäßen Verfahrens enthält das Polyisocyanat durchschnittlich 2 bis 5 funktionelle -N=C=O-Gruppen. Hierzu zählen beispielsweise aliphatische, cycloaliphatische und aromatische Di-, Tri- und höhere Polyisocyanate.

Unter den oben genannten Polyisocyanaten sind Diisocyanate und Polyisocyanate mit drei funktionellen -N-C=O-Gruppen besonders bevorzugt und finden daher vorrangig Anwendung in der Durchführung der vorliegenden Erfindung. Bevorzugt verwendet werden Diisocyanate mit der allgemeinen Struktur O=C=N-R-N=C=O, wobei R für aliphatische, alicyclische oder aromatische Radikale stehen. Vorzugsweise weisen die Radikale fünf oder mehr Kohlenstoffatome auf.

In einer bevorzugten Ausführungsform des erfindungsgemäßen Verfahrens ist das mindestens eine Polyisocyanat mit zwei oder mehreren Isocyanat-Gruppen ausgewählt aus der Gruppe der aliphatischen Polyisocyanate und/oder der aromatischen Polyisocyanate. In einer noch mehr bevorzugten Variante des erfindungsgemäßen Verfahrens ist das mindestens eine Polyisocyanat eine Kombination aus zwei unterschiedlichen aliphatischen Polyisocyanaten oder eine Kombination aus einem aliphatischen und aromatischen Polyisocyanat.

Aufgrund der Anzahl von funktionellen Gruppen wird eine optimale Vernetzung oder ein Netzwerk der Kapselwand erreicht, wodurch Mikrokapseln bereitgestellt werden, die eine verlängerte langsame Freisetzung von Wirkstoffen sowie eine gute Stabilität im Verbraucherprodukt aufweisen.

In einer bevorzugten Variante des erfindungsgemäßen Verfahrens ist das Polyisocyanat ein aliphatisches Polyisocyanat.

Der Begriff "aliphatisches Polyisocyanat" bezieht sich auf jedes PolyisocyanatMolekül, das nicht aromatisch ist. Darüber hinaus umfasst das Molekül mindestens zwei Isocyanatgruppen, d.h. mindestens 2, 3, 4, 5, 6, 7, 8, 9, 10, 11, 12, 13, 14, 15, 20, 50, 100, 200 oder mehr Isocyanatgruppen, die direkt an eine entsprechende Anzahl verschiedener C-Atome desselben aliphatischen Moleküls gebunden sind, und Derivate solcher Verbindungen.

Das aliphatische Polyisocyanatmolekül, das mindestens zwei Isocyanatgruppen, d.h. mindestens 2, 3, 4, 5, 6, 7, 8, 9, 10, 11, 12, 13, 14, 15, 20, 50, 100, 200 oder mehr Isocyanatgruppen aufweist, kann ferner linear, verzweigt oder cyclisch sein und kann beliebige Substitutionen aufweisen, einschließlich beispielsweise aliphatische Substituenten, aromatische Substituenten, ein oder mehrere Heteroatome, wie Stickstoff, Sauerstoff, Phosphor und/oder Schwefel, Halogene wie Fluor, Chlor, Brom und/oder Iod und/oder andere funktionelle Gruppen, wie beispielsweise Alkoxygruppen.

Das lineare aliphatische Polyisocyanatmolekül ist vorzugsweise ausgewählt aus C2- bis C20- linearem Alkyl, vorzugsweise C3- bis C15- linearem Alkyl, C4- bis C12- linearem Alkyl, C5- bis C10- linearem Alkyl, C6- bis C9- linearem Alkyl oder C7-bis C8- linearem Alkyl. Vorzugsweise umfasst das lineare aliphatische Molekül keine aromatische Struktur.

Das verzweigte aliphatische Polyisocyanatmolekül ist vorzugsweise ausgewählt aus C2- bis C20- verzweigtem Alkyl, vorzugsweise C3- bis C15-verzweigtem Alkyl verzweigtem Alkyl, C4- bis C12- verzweigtem Alkyl, C5- bis C10-verzweigtem Alkyl, C6- bis C9- verzweigtem Alkyl, C7- bis C8- verzweigtem Alkyl.

Je kürzer die Kohlenstoffkette des Polyisocyanatmoleküls, desto höher ist die Reaktionsgeschwindigkeit im Vergleich zu längerkettigen Analoga.

Das cyclische aliphatische Polyisocyanatmolekül umfasst mindestens 1, d.h. 1, 2, 3, 4 oder mehr nichtaromatische Ringstrukturen, wobei die Ringstruktur selbst vorzugsweise nur aus C-Atomen besteht. Selbstverständlich können die C-Atome der Ringstruktur geeignete Substituenten tragen. Die mindestens 1-Ringstrukturen bestehen vorzugsweise unabhängig voneinander aus 3, 4, 5, 6, 7 oder 8-gliedrigen Ringen. Vorzugsweise umfasst das cyclische aliphatische Molekül 2 bis 20 C-Atome, wie 3 bis 15 C-Atome, 4 bis 12 C-Atome, 5 bis 10 C-Atome, 6 bis 9 C-Atome oder 7 bis 8 C-Atome.

In einer weiteren Variante des erfindungsgemäßen Verfahrens ist das Polyisocyanat ein aromatisches Polyisocyanat. Der Begriff "aromatisches Polyisocyanat" bezieht sich auf jede Polyisocyanat-Verbindung, bei denen zwei oder mehr Isocyanatgruppen direkt an aromatische C-Atome gebunden und beispielsweise eine Phenyl-, Tolyl-, Xylyl-, Naphthyl- oder Diphenyleinheit als aromatische Komponente umfasst, sowie Derivate solcher Polyisocyanat-Verbindungen.

Aromatische Polyisocyanate reagieren deutlich schneller als aliphatische Polyisocyanate und werden daher in dem erfindungsgemäßen Verfahren bevorzugt eingesetzt.

Das lineare, verzweigte oder cyclische aliphatische oder aromatische Polyisocyanat kann als Monomer bzw. Polymer vorliegen. Ein monomeres Polyisocyanat ist ein Molekül, das nicht mit einem anderen Molekül verbunden ist, insbesondere nicht durch ein oder mehrere Vernetzungsmittel. Ein polymeres Polyisocyanat umfasst mindestens zwei Monomere, die durch ein oder mehrere Vernetzungsmittel miteinander verbunden sind. Die mindestens zwei Monomere müssen nicht notwendigerweise die gleichen Monomere sein, sondern können auch unterschiedlich sein. Ein polymeres Polyisocyanat umfasst vorzugsweise mindestens 2 oder mehr Monomere, d. h. mindestens 2, 3, 4, 5, 10, 20, 30, 40, 50, 100 oder mehr Monomere, die durch mindestens ein Vernetzungsmittel miteinander verbunden sind.

Das lineare, verzweigte oder cyclische aliphatische oder aromatische Polyisocyanat weist vorzugsweise eine begrenzte Größe/ein begrenztes Molekulargewicht auf, was eine Reaktivität mit dem einen oder den mehreren Vernetzungsmitteln ermöglicht. Beispiele für geeignete Molekulargewichte umfassen vorzugsweise ca. 100 g/mol bis 5 · 10⁴ g/mol, vorzugsweise 120 g/mol bis 2 · 10⁴ g/mol, 140 g/mol bis 10⁴ g/mol, 160 g/mol bis 5 · 10³ g/mol, 180 g/mol bis 2 · 10³ g/mol, 200 g/mol bis 10³ g/mol, 220 g/mol bis 900 g/mol, 240 g/mol bis 800 g/mol, 260 g/mol bis 700 g/mol, 280 g/mol bis 600 g/mol, 300 g/mol bis 500 g/mol, 320 g/mol bis 450 g/mol oder 340 g/mol bis 400 g/mol.

Es können beliebig viele verschiedene lineare, verzweigte und/oder cyclische aliphatische und/oder aromatische Polyisocyanate eingesetzt werden. Beispielsweise wird/werden mindestens ein, d. h. mindestens 1, 2, 3, 4, 5, 6, 7, 8, 9 oder 10 verschiedene lineare aliphatische Polyisocyanate verwendet. Beispielsweise wird/werden mindestens ein, d. h. mindestens 1, 2, 3, 4, 5, 6, 7, 8, 9 oder 10 verschiedene verzweigte aliphatische Polyisocyanate verwendet. Beispielsweise wird/werden mindestens ein, d. h. mindestens 1, 2, 3, 4, 5, 6, 7, 8, 9 oder 10 verschiedene verzweigte cyclische Polyisocyanate verwendet.

Vorzugsweise werden Derivate der linearen, verzweigten und/oder cyclischen aliphatischen Polyisocyanate eingesetzt. Ein Derivat, wie es hier verwendet wird, wird in seiner weitesten Bedeutung als eine Verbindung verstanden, die durch eine chemische Reaktion von einer Verbindung abgeleitet wird. Beispiele für Derivate umfassen Oligomere und/oder Addukte der oben genannten linearen oder verzweigten aliphatischen Polyisocyanate. Bevorzugte Oligomere sind Biurets, Isocyanurate, Uretdione, Iminooxadiazindione und bevorzugte Addukte sind Trimethylolpropan-Addukte. Diese Oligomere/Addukte sind im Stand der Technik gut bekannt und beispielsweise in US 4855490 A oder US 4144268 A offenbart.

Vorzugsweise liegt das aliphatische Polyisocyanat nur in monomerer Form und/oder dimerisierter Form (als Isocyanat) oder in oligomerer Form vor.

Die Derivate der linearen, verzweigten oder cyclischen Polyisocyanate und/oder Gemische davon können auch durch Reaktion der Polyisocyanate mit Polyalkoholen (z. B. Glycerin), Polyaminen, Polythiolen (z. B. Dimercaprol) erhalten werden.

Die Isocyanat-Verbindungen gemäß der obigen Definition umfassen ausdrücklich die verschiedenen Isomere, falls vorhanden, allein oder in Kombination. Beispielsweise umfasst Methylenbis-(cyclohexylisocyanat) (H12MDI) 4,4'-Methylenbis- (cyclohexylisocyanat), 2,4'-Methylenbis-(cyclohexylisocyanat) und/oder 2,2'-Methylenbis-(cyclohexylisocyanat).

Beispielhafte aliphatische Polyisocyanate umfassen solche, die im Handel erhältlich sind, z. B. BAYHYDUR N304 und BAYHYDUR N3Q5, die aliphatische wasserdispergierbare Polyisocyanate auf der Basis von Hexamethylendiisocyanat sind, DESMODUR N3400, DESMODUR N3600, DESMODUR N3700 und DESMODUR N3900, die niedrigviskose, polyfunktionale aliphatische Polyisocyanate auf der Basis von Hexamethylendiisocyanat sind, und DESMODUR 3600 und DESMODUR N100, die aliphatische Polyisocyanate auf der Basis von Hexamethylendiisocyanat sind, von denen jedes von der Bayer Corporation, Pittsburgh, PA, erhältlich ist.

Gemäß einer anderen bevorzugten Variante der vorliegenden Erfindung ist oder sind die linearen oder verzweigten aliphatischen Polyisocyanate aus der Gruppe ausgewählt, die aus Pentamethylendiisocyanat (PDI, wie Stabio D-370N oder D-376N von Mitsui Chemicals Inc., Japan, besteht ), Hexamethylendiisocyanat (HDI), Ethylester-Lysin-Triisocyanat, Lysin-Diisocyanat-Ethylester und Derivate davon, vorzugsweise wobei jedes der Derivate mehr als eine Isocyanatgruppe umfasst und gegebenenfalls ferner eine oder mehrere Gruppen umfasst, ausgewählt aus der Gruppe bestehend aus Biuret, Isocyanurat , Uretdion-, Iminooxadiazindion- und Trimethylolpropan-Addukt und/oder wobei das oder die cyclischen aliphatischen Polyisocyanate aus der Gruppe ausgewählt sind oder sind, die aus Isophorondiisocyanat (IPDI), 1,3-Bis (isocyanatomethyl) cyclohexan (H6XDI, wie Takenate) besteht oder ausgewählt ist 600 von Mitsui Chemicals Inc., Japan), 1,2-Bis (isocyanatomethyl) cyclohexan, 1,4-Bis (isocyanato-methyl) cyclohexan, Methylenbis (cyclohexylisocyanat) ) (H12MDI) und Derivate davon, vorzugsweise wobei jedes der Derivate mehr als eine Isocyanatgruppe umfasst und gegebenenfalls ferner eine oder mehrere Gruppen umfasst, ausgewählt aus der Gruppe bestehend aus Biuret, Isocyanurat, Uretdion, Iminooxadiazinion und Trimethylolpropan-Addukt (wie TMP-Addukt) von H6XDI, insbesondere Takenate D-120N von Mitsui Chemicals Inc., Japan).

Besonders bevorzugt sind aliphatische Polyisocyanate, die aus nachwachsenden Rohstoffen wie PDI (Stabio D-370N oder D-376N von Mitsui Chemicals Inc., Japan) gewonnen werden. Es wurde gefunden, dass solche aliphatischen Polyisocyanate, die aus nachwachsenden Rohstoffen erhalten werden, die Qualität/Eigenschaften der Kern-Schale-Kapseln nicht beeinträchtigen.

Weitere geeignete im Handel erhältliche Polyisocyanate umfassen LUPRANAT M20 (BASF), wobei der Durchschnitt n 0,7 beträgt; PA PI 27 (Dow Chemical), wobei der Durchschnitt n 0,7 beträgt; MONDUR MR (Bayer), wo der Durchschnitt n 0,8 beträgt; MONDUR MR Light (Bayer) mit einem durchschnittlichen n von 0,8; MONDUR 489 (Bayer), wo der Durchschnitt n 1,0 beträgt; Poly-[(phenylisocyanat)-co-formaldehyd (Aldrich Chemical, Milwaukee, WI), andere Isocyanatmonomere wie DESMODUR N3200 (Bayer) und TAKENATE D1 10-N (Mitsui Chemicals Corporation, Rye Brook, NY). Weitere repräsentative Polyisocyanate sind Polyisocyanate mit der Bezeichnung TAKENATE D-1 10N (Mitsui), DESMODUR L75 (Bayer) und DESMODUR IL (Bayer).

In einer bevorzugten Variante wird das Polyisocyanat, das bei der Herstellung der Polyharnstoff/Polyurethan-Mikrokapseln gemäß der vorliegenden Erfindung verwendet wird, als alleinige Polyisocyanat-Komponente, d.h. ohne die Beimischung einer anderen, davon verschiedenen Polyisocyanat-Komponente eingesetzt.

Beispiele für die erfindungsgemäß einsetzbaren monomeren Polyisocyanate, welche mindestens zwei Polyisocyanat-Gruppen enthalten, sind:
Ethylendiisocyanat, Trimethylendiisocyanat, 1,4-Tetramethylendiisocyanat, 1,6-Hexamethyldiisocyanat, Ethylendiisothiocyanat, Tetramethylendiisothiocyanat, Hexamethylendiisothiocyanat, Cyclobutan-1,3-diisocyanat, Cyclohexan-1,3-diisocyanat, Cyclohexan-1,4-diisocyanat, 1,3-Phenylendiisocyanat, 1,4-Phenylendiisocyanat, Gemische von 1,3-Phenylendiisocyanat und 1,4-Phenylendiisocyanat, p-Phenylendiisothiocyanat, Xylylen-1,4-diisothiocyanat, 2,4-Toluylendiisocyanat, 2,6-Toluylendiisocyanat, Gemische von 2,4-Toluylendiisocyanat und 2,6-Toluylendiisocyanat, Xylylen-1,4-diisocyanat, Xylylen-1,3-diisocyanat sowie Gemische von Xylylen-1,4-diisocyanat und Xylylen-1,3-diisocyanat, 2,4-Hexahydrotoluylendiisocyanat, 2,6-Hexahydrotoluylendiisocyanat, Gemische von 2,4-Hexahydrotoluylendiisocyanat und 2,6-Hexahydrotoluylendiisocyanat, Hexahydro-1,3-phenylendiisocyanat, Hexahydro-1,4-phenylendiisocyanat, Gemische von Hexahydro-1,4-phenylendiisocyanat und Hexahydro-1,4-phenylendiisocyanat, 1,3-Diisocyanatobenzol, 1,3,5-Trimethylbenzol-2,4-diisocyanat, 1,3,5-Triisopropylbenzol-2,4-diisocyanat, Diphenylmethan-4,4'-diisocyanat, 3,3'-Dimethyldiphenylmethan-4,4'-diisocyanat, 4,4'-Diphenylpropandiisocyanat, Naphthylen-1,4-diisocyanat, Naphthylen-1,5-diisocyanat, Triphenylmethan-4,4',4"-triisocyanat, Toluylen-2,4,6-triisocyanat, Dimethyldiphenylmethan-2,2',5,5'-tetraisocyanat oder Mischungen der vorgenannten Verbindungen.

Als wenigstens zwei Polyisocyanat-Gruppen aufweisende polymerisationsfähige Verbindungen werden vorzugsweise großtechnisch hergestellte Di- und Polyisocyanate bevorzugt, beispielsweise TDI: Toluylendiisocyanat (Isomerengemisch von 2,4- und 2,6-Toluylendiisocyanat im Verhältnis von 80 : 20), HDI: Hexamethylendiisocyanat-(1,6), IPDI: Isophorondiisocyanat oder DMDI: Diphenylmethan-4,4'-diisocyanat.

Weitere besonders bevorzugte monomere Polyisocyanat-Verbindungen sind: Diisocyanate wie 1,4-Diisocyanatobutan, 1,6-Diisocyanatohexan, 1,5-Diisocyanato-2,2-dimethylpentan, 2,2,4- und 2,4,4-Trimethyl-1,6-diisocyanatohexan, 1,10-Diisocyanatodecan, 1,3- und 1,4-Diisocyanatocyclohexan, 1-Polyisocyanato-3,3,5-trimethyl-5-polyisocyanatomethylcyclohexan (Isophorondiisocyanat), 4,4'-Diisocyanatodicyclohexylmethan, 2,4- und 2,6-Diisocyanatomethylcyclohexan und deren Gemische. Prinzipiell können auch aromatische Polyisocyanate, z. B. Toluylendiisocyanate oder 4,4'-Diisocyanatodiphenylmetha Verwendung finden.

Weitere spezifische Beispiele für Diisocyanate umfassen beispielsweise 1,5-Naphthylendiisocyanat, 4,4'-Diphenylmethandiisocyanat (MDI), hydriertes MDI (H12MDI), Xylylendiisocyanat (XDI), Tetramethylxyloldiisocyanat (TMXD1) 4,4'-Diphenyldimethylmethandiisocyanat, Di- und Tetraalkyldiphenylmethandiisocyanat, 4,4'-Dibenzyldiisocyanat, 1,3-Phenylendiisocyanat, 1,4-Phenylendiisocyanat, die Isomere von Toluylendiisocyanat (TDI), gegebenenfalls in einer Mischung, 1-Methyl-2,4-diisocyanatocyclohexan, 1,6-Diisocyanato-2,2,4-trimethylhexan, 1,6-Diisocyanato-2,4,4-trimethylhexan, 1 -Isocyanatomethyl-3-isocyanato-1,5,5-trimethylcyclohexan, chlorierte und bromierte Diisocyanate, phosphorhaltige Diisocyanate, 4,4'-Diisocyanatophenylperfluorethan, Tetramethoxybutan-1,4-diisocyanat, Butan-1,4-Diisocyanat, (HDI), Dicyclohexylmethandiisocyanat, Cyclohexan-1,4-diisocyanat, Ethylendiisocyanat, Phthalsäurebisisocyanatoethylester, auch Polyisocyanate mit reaktiven Halogenatomen wie 1-Chlormethylphenyl-2,4-diisocyanat-1,2-br 3,3-Bischlormethylether-4,4'-diphenyldiisocyanat.

Es hat sich überraschend gezeigt, dass insbesondere die Verwendung von längerkettigen aliphatischen Diisocyanaten mit sechs, sieben, acht, neun, zehn oder sogar mehr Kohlenstoffatomen zur Bildung stabilerer Kapselhüllen bzw. Kapselwände führt.

In einer besonders bevorzugten Ausführungsform, umfasst die interne nicht-wässrige Phase eine Mischung aus zwei oder mehreren verschiedenen polymerisationsfähigen Polyisocyanaten, beispielsweise Polyisocyanaten mit unterschiedlicher Kettenlänge, welche Mischpolymerisate ausbilden können.

Anteilig können auch Derivate von Polyisocyanaten, die durch Modifizierung der oben genannten Diisocyanate oder deren Mischungen nach bekannten Verfahren darstellbar sind und z. B. Uretdion-, Urethan-, Isocyanurat-, Biuret- und/oder Allophanat-Gruppen enthalten, in dem erfindungsgemäßen Verfahren mit eingesetzt werden.

Ganz besonders bevorzugt ist eine Kombination aus mindestens zwei unterschiedlichen, vorzugsweise aliphatischen, Polyisocyanaten oder eine Kombination aus mindestens einem aliphatischen und mindestens einem aromatischen Polyisocyanat.

Bei einer solchen Kombination wird die unterschiedliche Reaktionsgeschwindigkeit der Polyisocyanate ausgenutzt: Aromatische Polyisocyanate reagieren deutlich schneller als aliphatische Polyisocyanate und bei den kurzkettigen aliphatischen Polyisocyanaten, d.h. aliphatischen Polyisocyanaten mit einem bis fünf Kohlenstoffatomen, vorzugsweise drei bis fünf Kohlenstoffatomen, ist die Reaktionsgeschwindigkeit höher im Vergleich zu längerkettigen Analoga.

In einer weiter bevorzugten Weiterbildung der Erfindung weisen daher die unterschiedlichen aliphatischen und/oder aromatischen Polyisocyanate auch unterschiedliche Kettenlängen auf. Längerkettige Polyisocyanate weisen in diesem Zusammenhang vorzugsweise sechs, sieben, acht, neun, zehn, elf, zwölf, dreizehn, vierzehn, 20, fünfundzwanzig oder mehr Kohlenstoffatome auf, noch bevorzugter weisen sie allerdings sechs bis zwölf Kohlenstoffatome und besonders bevorzugt sechs bis acht Kohlenstoffatome auf. Unter kürzerkettigen Polyisocyanaten sind Polyisocyanate mit einem bis fünf Kohlenstoffatomen und bevorzugt Polyisocyanate mit drei bis fünf Kohlenstoffatomen zu verstehen.

Erfindungsgemäß bevorzugt ist eine Kombination aus einem kurzkettigen aliphatischen Polyisocyanat (C1, C2, C3, C4, C5) und einem langkettigen aliphatischen Polyisocyanat (C6, C7, C8, C9, C10, C11, C12, C13, C14, C15, C20, C25 oder mehr) oder eine Kombination aus einem kurzkettigen aliphatischen Polyisocyanat (C1, C2, C3, C4, C5) (C1, C2, C3, C4, C5) mit einem langkettigen aromatischen Polyisocyanat (C6, C7, C8, C9, C10, C11, C12, C13, C14, C15, C20, C25 oder mehr) oder eine Kombination aus einem langkettigen aliphatischen Polyisocyanat (C6, C7, C8, C9, C10, C11, C12, C13, C14, C15, C20, C25 oder mehr) (C6, C7, C8, C9, C10, C11, C12, C13, C14, C15, C20, C25 oder mehr) mit einem kurzkettigen aromatischen Polyisocyanat.

Besonders bevorzugt ist in diesem Zusammenhand die Verwendung einer Mischung unterschiedlicher aliphatischer Polyisocyanate mit zwei oder mehreren Isocyanat-Gruppen mit Kettenlängen von einem bis zwölf Kohlenstoffatomen in der Kette, bevorzugt drei bis acht Kohlenstoffatomen und besonders bevorzugt vier bis sieben Kohlenstoffatomen, für die Herstellung der erfindungsgemäßen bioabbaubaren Mikrokapseln.

Aliphatische Polyisocyanate sind in diesem Zusammenhang aufgrund ihrer chemischen Verwandtschaft zu biobasierenden Systemen besonders zu bevorzugen. So zeigen beispielsweise sowohl Lysin als auch 1,5-Diisocyanatopentan dasselbe Abbauprodukt, 1,5-Diaminopentan, und eignen sich daher besonders für die Verwendung in der Herstellung von biobasierten und bioabbaubaren Mikrokapseln unter Berücksichtigung umwelttechnischer Gesichtspunkte.

Primäre Ausführungen umfassen Mischungen von längerkettigen und kürzerkettigen Diisocyanaten in beliebigen Mischungsverhältnissen. Bevorzugter Weise liegt das Mischungsverhältnis von längerkettigen Diisocyanaten zu kürzerkettigen Diisocyanaten in einem Bereich von 4: 1 bis 1 : 4 und besonders bevorzugt von 2 : 1 bis 1 : 2.

Beispiele für bevorzugte spezifische Mischungen von mindestens einem aliphatischen Polyisocyanat und von mindestens einem aromatischen Polyisocyanat sind eine Mischung aus einem Biuret aus Hexamethylendiisocyanat mit einem Trimethylol-Addukt aus Xylylendiisocyanat, eine Mischung aus einem Biuret aus Hexamethylendiisocyanat mit einem Polyisocyanurat aus Diisocyanat oder eine Mischung aus einem Biuret von Hexamethylendiisocyanat mit einem Trimethylolpropan-Addukt aus Toluoldiisocyanat.

Erfindungsgemäß noch mehr bevorzugt ist, wenn in der zuvor beschriebenen Kombination aus einem kurzkettigen aliphatischen Polyisocyanat und einem langkettigen aliphatischen Polyisocyanat oder in der Kombination aus einem kurzkettigen aliphatischen Polyisocyanat und einem langkettigen aromatischen Polyisocyanat oder in der Kombination aus einem langkettigen aliphatischen Polyisocyanat mit einem kurzkettigen aromatischen Polyisocyanat, die Polyisocyanate in einer Mischung aus monomerer oder oligomerer bzw. polymerer Form vorliegen.

Vorzugsweise ergeben sich daraus für die Verwendung in dem erfindungsgemäßen Verfahren folgende Kombinationen:
- kurzkettiges aliphatisches Polyisocyanat (Monomer oder Oligomer bzw. Polymer) und kurzkettiges aliphatisches Polyisocyanat (Monomer oder Oligomer bzw. Polymer);
- kurzkettiges aliphatisches Polyisocyanat (Monomer oder Oligomer bzw. Polymer) und langkettiges aliphatisches Polyisocyanat (Monomer oder Oligomer bzw. Polymer);
- kurzkettiges aliphatisches Polyisocyanat (Monomer oder Oligomer bzw. Polymer) und kurzkettiges aromatisches Polyisocyanat (Monomer oder Oligomer bzw. Polymer);
- kurzkettiges aliphatisches Polyisocyanat (Monomer oder Oligomer bzw. Polymer) und langkettiges aromatisches Polyisocyanat (Monomer oder Oligomer bzw. Polymer);
- langkettiges aliphatisches Polyisocyanat (Monomer oder Oligomer bzw. Polymer) und kurzkettiges aliphatisches Polyisocyanat (Monomer oder Oligomer bzw. Polymer);
- langkettiges aliphatisches Polyisocyanat (Monomer oder Oligomer bzw. Polymer) und langkettiges aliphatisches Polyisocyanat (Monomer oder Oligomer bzw. Polymer);
- langkettiges aliphatisches Polyisocyanat (Monomer oder Oligomer bzw. Polymer) und kurzkettiges aromatisches Polyisocyanat (Monomer oder Oligomer bzw. Polymer);
- langkettiges aliphatisches Polyisocyanat (Monomer) und langkettiges aromatisches Polyisocyanat (Oligomer bzw. Polymer);
mit den zuvor beschriebenen Definitionen für kurzkettig und langkettig.

Es konnte beobachtet werden, dass die Wahl von mindestens zwei aliphatischen Polyisocyanaten unterschiedlicher Kettenlänge und unterschiedlichem Polymerisationsgrad oder die Wahl von Mischungen aus aliphatischen und aromatischen Polyisocyanaten aufgrund der unterschiedlichen Reaktionsgeschwindigkeit der Polyisocyanat-Komponenten, Dissoziationen und Vernetzungsstrukturen zu einem deutlich Gewinn an Stabilität und Performance (Duftstofffreisetzung im Falle von Duftstoffkapseln) führen.

Mit den zuvor genannten Polyisocyanat-Kombinationen oder PolyisocyanatGemischen aus zwei unterschiedlichen aliphatischen oder einem aliphatischen und einem aromatischen Polyisocyanat lassen sich besonders stabile und bessere, d. h. dichter verzweigte Vernetzungen innerhalb der Kapselhülle erzeugen.

So lassen sich mit dem hierin beschriebenen Verfahren performante (Duftstofffreisetzung) Mikrokapseln herstellen, die entweder aus einem Gemisch aus einem aliphatischen und aromatischen Polyisocyanat oder aus einem Gemisch aus zwei unterschiedlichen aliphatischen Polyisocyanaten hergestellt werden. Derartige Mikrokapseln sind sehr stabil und zeichnen sich durch eine herausragende Duftspeicher-Eigenschaften aus, was sich wiederum in einer besseren Performance (Duftstofffreisetzung) der Kapseln, beispielsweise im Bereich der Duftstoffstoffverkapselung, widerspiegelt.

Der Einsatz von zwei verschiedenen Polyisocyanaten ergibt Mikrokapseln, die die Stabilität von Mikrokapseln aus Einzelpolyisocyanat-Systemen nochmals übersteigt, wie dies in den nachfolgenden Ausführungsbeispielen veranschaulicht ist.

Die Mikrokapsel aus einem aliphatisch-aliphatischen Polyisocyanat-Gemisch ist genauso gut, wie eine Mikrokapsel aus einem aliphatisch-aromatischen Polyisocyanat-Gemisch, wie dies in den nachfolgenden Ausführungsbeispielen veranschaulicht wird. Demnach ist grundsätzlich in der vorliegenden Erfindung die Kombination mindestens zweier unterschiedlicher polymerisationsfähiger (vorzugsweise aliphatischer und/oder aromatischer) Polyisocyanate zu bevorzugen.

Der Gehalt an Polyisocyanat zur Herstellung der erfindungsgemäßen Mikrokapsel beträgt 0,1 bis 10,0 Gew.-%, vorzugsweise 0,5 bis 3,0 Gew.-%, bezogen auf das Gesamtgewicht der internen nicht-wässrigen Phase.

Der Anteil der Polyisocyanat-Komponente zu der internen nicht-wässrigen Phase beträgt vorzugsweise zwischen 1 : 50 und 1 : 20, noch mehr bevorzugt zwischen 1 : 40 und 1 : 30.

Aufgrund des geringen Anteils der Polyisocyanat-Komponente ist es gemäß der vorliegenden Erfindung möglich, Polyharnstoff/Polyurethan-Mikrokapseln herzustellen, in denen der absolute Polyisocyanat-Anteil lediglich ein 1/50-stel der gesamten Kapsel, welche mindestens einen zu verkapselnden lipophilen Wirkstoff umfasst, beträgt. So können mit dem erfindungsgemäßen Verfahren Polyharnstoff/Polyurethan-Mikrokapseln hergestellt werden, die einen Polyisocyanat-Anteil von nur 0,6 Gew.-% aufweisen, bezogen auf das Gesamtgewicht der Kapselwand. Vorzugsweise liegt der Polyisocyanat-Anteil bei etwa 1,8 Gew.-% der Kapselwand. Trotz des geringen Polyisocyanat-Gehalts zeichnen sich die erfindungsgemäßen Mikrokapseln dennoch durch eine hohe Stabilität aus.

Bei dem Schritt (a1) des erfindungsgemäßen Verfahrens zur Herstellung der Mikrokapseln wird zunächst das mindestens eine polymerisationsfähige Polyisocyanat, das mindestens zwei oder mehrere funktionelle Isocyanat-Gruppen umfasst, zusammen mit mindestens einem oder mehreren zu verkapselnden Wirkstoff(en), im Wesentlichen in einem inerten, nicht-wässrigen Lösungsmittel oder einem Lösungsmittelgemisch aus inerten, nicht-wässrigen Lösungsmitteln, gelöst. Unter "im Wesentlichen gelöst" wird verstanden, dass mindestens 90 Gew.-%, vorzugsweise mindestens 98 Gew.-%, noch mehr bevorzugt 99,9 Gew.-%, der zuvor genannten Bestandteile in dem Lösungsmittel oder in dem Lösungsmittelgemisch gelöst sind, um sie im vorliegenden Verfahren verwenden zu können. Bevorzugter sind das mindestens eine Polyisocyanat und der mindestens eine zu verkapselnde Wirkstoff in dem Lösungsmittel oder in dem Lösungsmittelgemisch vollständig gelöst. Sollte ein Lösungsmittel keine ausreichende Löslichkeit der Isocyanate gewährleisten, besteht die Möglichkeit, diesen Nachteil durch Verwendung geeigneter Löslichkeitsförderer zu überwinden.

Bevorzugte Lösungsmittel für die interne nicht-wässrige Phase sind nicht mit Wasser mischbar und reagieren nicht mit der/den Isocyanatkomponente(n) oder der/den Wirkstoffkomponente(n) und weisen in den verwendeten Mengen wenig oder keinen Geruch auf.

Der Ausdruck "Lösungsmittel" im Zusammenhang mit der vorliegenden Erfindung umfasst alle Arten von Ölkörpern oder Ölkomponenten, insbesondere Pflanzenöle wie z. Rapsöl, Sonnenblumenöl, Sojaöl, Olivenöl und dergleichen, modifizierte Pflanzenöle, z.B. alkoxylierte Sonnenblumen- oder Sojaöl, synthetische (Tri) Glyceride wie z.B. technische Gemische von Mono-, Di- und Triglyceriden von C6- bis C22-Fettsäuren, Fettsäurealkylester, z.B. Methyl- oder Ethylester von Pflanzenölen (Agnique^{®} ME 18 RD-F, Agnique^{®} ME 18 SD-F, Agnique^{®} ME 12C-F, Agnique^{®} ME1270), Fettsäurealkylester auf Basis dieser C6- bis C22-Fettsäuren, Mineralöle und deren Gemische. Beispiele geeigneter und bevorzugter lipophiler Lösungsmittel sind: Guerbetalkohole auf der Basis von Fettalkoholen mit 6 bis 18, vorzugsweise 8 bis 10, Kohlenstoffatomen, Estern linearer C6- bis C22-Fettsäuren mit linearem oder verzweigtem C6- bis C22-Fettalkohole oder -ester von verzweigten C6-bis C13-Carbonsäuren mit linearen oder verzweigten C6- bis C22-Fettalkoholen, wie beispielsweise Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylstearat, Stearylerucat, Isostearylmyristat, Isostearylpalmitat, Isostearylstearat, Isostearylisostearat, Isostearyloleat, Isostearylbehenat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat.

Geeignet sind auch Ester von linearen C6- bis C22-Fettsäuren mit verzweigten Alkoholen, insbesondere 2-Ethylhexanol, Ester von C18- bis C38-Alkylhydroxycarbonsäuren mit linearen oder verzweigten C6- bis C22-Fettsäuren, insbesondere Dioctylalat, Ester von linearen oder verzweigten Fettsäuren mit mehrwertigen Alkoholen (wie beispielsweise Propylenglykol, Dimerdiol oder Trimertriol) und/oder Guerbetalkohole, Triglyceride auf Basis von C6- bis C10-Fettsäuren, flüssige Mono-/Di-/Triglyceridmischungen von C6- bis C18-Fettsäuren, Ester von C6- bis C22-Fettalkoholen und/oder Guerbetalkoholen mit aromatischen Carbonsäuren, insbesondere Benzoesäure, Ester von C2- bis C12-Dicarbonsäuren mit linearen oder verzweigten Alkoholen mit 1 bis 22 Kohlenstoffatomen oder Polyole mit 2 bis 10 Kohlenstoffatomen und 2 bis 6 Hydroxylgruppen, Pflanzenöle, verzweigte primären Alkohole, substituierte Cyclohexane, lineare oder verzweigte C6- bis C22-Fettalkoholcarbonate, wie beispielsweise Dicaprylylcarbonat (Cetiol^{®} CC); GuerbetCarbonate, basierend auf Fettalkoholen mit 6 bis 18, vorzugsweise 8 bis 10 Kohlenstoffatomen, Benzoesäureester mit linearen oder verzweigten C6- bis C22-Alkoholen, lineare oder verzweigte, symmetrische oder asymmetrische Dialkylether mit 6 bis 22 Kohlenstoffatomen pro Alkylgruppe, wie zum Beispiel Dicaprylylether, Ringöffnungsprodukte von epoxidierten Fettsäureestern mit Polyolen, Siliconöle (Cyclomethicone, Siliconmethiconqualitäten usw.), aliphatische oder naphthenische Kohlenwasserstoffe, wie zum Beispiel Squalan, Squalen oder Dialkylcyclohexane und / oder Mineralöle.

Bevorzugte Lösungsmittel sind insbesondere auch Ester linearer C6- bis C22-Fettsäuren mit verzweigten Alkoholen, Ester von C18- bis C38-Alkylhydroxycarbonsäuren mit linearen oder verzweigten C6- bis C22-Fettalkoholen, lineare oder verzweigte C6- bis C22-Fettalkohole, insbesondere Dioctylmalate, Ester von linearen oder verzweigten Fettsäuren mit mehrwertigen Alkoholen, wie z. B. Propylenglykol, Dimerdiol oder Trimertriol, und/oder Guerbetalkohole, Triglyceride auf Basis von C6- bis C10-Fettsäuren, flüssige Mono-/Di-/ Triglyceridmischungen auf Basis von C6- bis C18-Fettsäuren, Ester von C6- bis C22-Fettalkoholen und/oder Guerbetalkoholen mit aromatischen Carbonsäuren, insbesondere Benzoesäure, Ester von C2- bis C12-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, Pflanzenöle, verzweigte primäre Alkohole, substituierte Cyclohexane, lineare und verzweigte C6- bis C22-Fettalkoholcarbonate, wie z.B. Dicaprylylcarbonat (Cetiol TM CC), Guerbetcarbonate auf der Basis von Fettalkoholen mit 6 bis 18, vorzugsweise 8 bis 10, Kohlenstoffatomen, Estern der Benzoesäure mit linearen oder verzweigten C6- bis C22-Alkoholen, lineare oder verzweigte, symmetrische oder asymmetrische Dialkylether mit 6 bis 22 Kohlenstoffatomen pro Alkylgruppe, wie z.B. Dicaprylylether (Cetiol TM OE), Ringöffnungsprodukte von epoxidierten Fettsäureestern mit Polyolen, Siliconölen (Cyclomethicone, Siliciummethicon-Typen usw.) und/oder aliphatischen oder naphthenischen Kohlenwasserstoffen, wie z.B Squalan, Squalen oder Dialkylcyclohexane.

Weiterhin können flüssige lineare und/oder verzweigte und/oder gesättigte oder ungesättigte Kohlenwasserstoffe oder beliebige gewünschte Gemische davon im Rahmen der vorliegenden Erfindung als Lösungsmittel verwendet werden. Dies können z.B. Alkane mit 4 bis 22, vorzugsweise 6 bis 18 Kohlenstoffatomen, oder beliebige Gemische davon sein.

Als inerte Lösungsmittel für die interne nicht-wässrige Phase eignen sich besonders vorteilhaft alkylaromatische Kohlenwasserstoffe wie beispielsweise Diisopropylnaphthalin oder substituierte Biphenyle, chloriertes Diphenyl, Paraffine, chloriertes Paraffin, natürliche Pflanzenöle wie beispielsweise Baumwollsamenöl, Erdnussöl, Palmöl, Trikresylphosphat, Silikonöl, Dialkylphthalate, Dialkyladipate, teilhydrierte Terphenyl, alkyliertes Biphenyl, alkyliertes Naphthalin, Diarylether, Arylalkylether und höher alkyliertes Benzol, Benzylbenzoat, Isopropylmyristat sowie beliebige Mischungen dieser hydrophoben Lösungsmittel und Mischungen einzelner oder mehrerer dieser hydrophoben Lösungsmittel mit Kerosin, Paraffinen und/oder Isoparaffinen. Bevorzugt werden Pflanzenöle wie Sonnenblumenöl, Triglyceride, Benzylbenzoat oder Isopropylmyristat als Lösungsmittel für die Bereitstellung der internen nicht-wässrigen Phase verwendet.

Die zuvor genannten Lösungsmittel werden in dem erfindungsgemäßen Verfahren entweder einzeln oder als Mischung aus zwei oder mehreren Lösungsmitteln verwendet.

In einer alternativen und bevorzugten Variante des erfindungsgemäßen Verfahrens wird das mindestens eine Polyisocyanat direkt in einer Lösung des mindestens einen Wirkstoffs, vorzugsweise eines oder mehrerer Duft- oder Aromastoffs/Duft- oder Aromastoffe oder eines Parfümöls, gelöst, so dass im Wesentlichen kein Lösungsmittel, wie oben beschrieben, im Kern der erfindungsgemäßen Mikrokapsel vorhanden ist. Die Vermeidung eines Lösungsmittels im Mikrokapselkern ist insofern vorteilhaft, um die Herstellungskosten zu senken und Umweltaspekte zu berücksichtigen.

Die Duft- oder Aromastoffe sind insbesondere in solchen Lösungsmitteln gelöst, die üblicherweise in der Parfüm- oder Aromaindustrie verwendet werden. Das Lösungsmittel ist vorzugsweise kein Alkohol, da Alkohole mit den Isocyanaten regieren. Beispiele geeigneter Lösungsmittel sind Diethylphthaloat, Isopropylmyristat, Abalyn^{®} (Kolophoniumharze, erhältlich von Eastman), Benzylbenzoat, Ethylcitrat, Limonen oder andere Terpene oder Isoparaffine. Vorzugsweise ist das Lösungsmittel sehr hydrophob. Vorzugsweise enthält die Duft- oder Aromastofflösung weniger als 30 % Lösungsmittel. Bevorzugter umfasst die Duft- oder Aromastofflösung weniger als 20 % und noch bevorzugter weniger als 10 % Lösungsmittel, wobei alle diese Prozentsätze durch das Gewicht relativ zum Gesamtgewicht der Duft- oder Aromastofflösung definiert sind. Am meisten bevorzugt ist der Duft oder das Aromat im Wesentlichen frei von Lösungsmitteln.

Als zu verkapselnder Wirkstoff bzw. als Kernmaterial zur Herstellung der erfindungsgemäßen Mikrokapseln kommt grundsätzlich jedes Material infrage, das für den Einschluss in Mikrokapseln geeignet ist. Die zu verkapselnden Materialien sind lipophile, wasserunlösliche oder mit Wasser nicht mischbare Flüssigkeiten oder Feststoffe sowie Suspensionen. Damit wird gewährleistet, dass sich der zu verkapselnde Wirkstoff bei der Herstellung der erfindungsgemäßen Mikrokapsel in der internen nicht-wässrigen Phase befindet und sich nicht mit der externen wässrigen Phase vermischt, da sich andernfalls keine Emulsion bilden und keine Abscheidung des Kapselwandmaterials auf der Tröpfchenoberfläche erfolgen kann. Dies führt dazu, dass der bei der anschließenden Emulgierung und Vernetzung der Kapselwandbestandteile der lipophile Wirkstoff vollständig im Inneren der Mikrokapsel als Kernmaterial eingeschlossen wird. Die so gebildete interne nicht-wässrige Phase zeichnet sich durch ihren organisch hydrophoben, öligen Charakter aus.

In einer besonders bevorzugten Variante der vorliegenden Erfindung ist der mindestens eine lipophile bzw. hydrophobe Wirkstoff insbesondere ein lipophiler bzw. hydrophober Duft- oder Aromastoff bzw. ein lipophiles bzw. hydrophobes Parfümöl oder Aroma (Duft- oder Aromastoffmischung), ein Cooling Agent, ein TRPV1 oder ein TRPV3-Modulator, eine Substanz, die einen scharfen Geschmack oder eine Wärme oder Hitzeempfindung auf Haut oder Schleimhäuten oder ein Prickel- bzw. Kribbelgefühl im Mund- oder Rachenraum hervorruft oder ein Wirkstoff mit stechender oder beißender oder adstringierender Wirkung, ein Pestizid, ein Biozid, ein Insektizid, eine Substanz aus der Gruppe der Repellentien, ein Lebensmittel-Additiv, ein kosmetischer Wirkstoff, ein pharmazeutischer Wirkstoff, ein Farbstoff, ein Farbstoff-Precursor, eine Leuchtfarbe, eine Agrochemikalie, ein optischer Aufheller, ein Lösungsmittel, ein Wachs, ein Silikonöl, ein Schmierstoffe, Substanzen für eine Druckbeschichtung für Papier, oder eine Mischung aus zwei oder mehreren der vorgenannten Wirkstoffen.

In einer bevorzugten Variante der vorliegenden Erfindung kommen als lipophile Wirkstoffe insbesondere lipophile Duftstoffe bzw. Duftstoffmischungen aus zwei oder mehreren Duftstoffen (Parfümöle) oder Aromastoffe oder Aromastoffmischungen aus zwei oder mehreren Aromastoffen (Aromen) oder auch biogene Prinzipien in Betracht.

Es ist besonders bevorzugt, dass der Kern einen oder mehrere Duftstoff(e) oder Aromastoff(e) umfasst, die aus der Gruppe ausgewählt sind, die besteht aus: Extrakten natürlicher Rohstoffe und auch Fraktionen davon oder daraus isolierten Bestandteilen; einzelnen Duftstoffen aus einer Gruppe von Kohlenwasserstoffen; aliphatischen Alkoholen; aliphatischen Aldehyden und Acetalen; aliphatischen Ketone und Oximen; aliphatischen schwefelhaltigen Verbindungen; aliphatischen Nitrilen; Estern aliphatischer Carbonsäuren; Formiaten, Acetaten, Propionaten, Isobutyraten, Butyraten, Isovaleraten, Pentanoaten, Hexanoaten, Crotonaten, Tiglinaten und 3-Methyl-2-butenoaten von acyclischen Terpenalkoholen; acyclischen Terpenaldehyden und Ketonen sowie deren Dimethyl- und Diethylacetalen; Formiaten, Acetaten, Propionaten, Isobutyraten, Butyraten, Isovaleraten, Pentanoaten, Hexanoaten, Crotonaten, Tiglinaten und 3-Methyl-2-butenoaten von cyclischen Terpenalkoholen; cyclischen Terpenaldehyden und Ketonen; cyclischen Alkoholen; cyclischen und cycloaliphatischen Ethern; cyclischen und makrocyclischen Ketonen; cycloaliphatischen Aldehyden; cycloaliphatischen Ketonen; Estern von cyclischen Alkoholen; Estern von cycloaliphatischen Alkoholen; Estern von cycloaliphatischen Carbonsäuren; aromatischen Kohlenwasserstoffen; araliphatischen Alkoholen; Estern von araliphatischen Alkoholen und aliphatischen Carbonsäuren; araliphatischen Ethern; aromatischen und araliphatischen Aldehyden; aromatischen und araliphatischen Ketonen; aromatischen und araliphatischen Carbonsäuren und deren Ester; stickstoffhaltigen aromatischen Verbindungen; Phenylethern und Phenylestern; heterocyclischen Verbindungen; Lactonen; und Mischungen aus den vorgenannten Wirkstoffen, wobei die Schale gegenüber dem oder den Duftstoffen vollständig oder im Wesentlichen undurchlässig ist.

Geeignete Duftstoffe und Aromen zur Herstellung der erfindungsgemäßen Kapseln sind vorzugsweise beschrieben beispielsweise. in "Riechstoffe [Fragrances]", in Steffen Arctander, in "Perfume and Flavor Chemicals", Eigenverlag, Montclair, N.J. 1969; H. Surburg, J. Panten, in "Common Fragrance and Flavor Materials", 5. Auflage, Wiley-VCH, Weinheim 2006.

Vorzugsweise weisen die erfindungsgemäßen Mikrokapseln ein Kernmaterial in Form eines hydrophoben Einzel-Duftstoffes bzw. Einzel-Aromastoffes auf, wobei das Kernmaterial mindestens einen Einzel-Duftstoff bzw. Einzel-Aromastoff umfasst, ausgewählt aus einer oder mehreren der folgenden Gruppen:
- Kohlenwasserstoffe, wie beispielsweise 3-Caren; alpha-Pinen; beta-Pinen; alpha-Terpinen; gamma-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
- Aliphatische Alkohole, wie beispielsweise Hexanol; Octanol; 3-Octanol; 2,6-Dimethylheptanol; 2-Methylheptanol, 2-Methyloctanol; (E)-2-Hexenol; (E)- und (Z)-3-Hexenol; 1-Octen-3-ol; Gemisch von 3,4,5,6,6-Pentamethyl-3,4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
- Aliphatische Aldehyde und deren Acetale, wie beispielsweise Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxypropoxy)-(E/Z)-3-Hexen;
- Aliphatische Ketone und deren Oxime, wie beispielsweise 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
- Aliphatische schwefelhaltige Verbindungen, wie beispielsweise 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
- Aliphatische Nitrile, wie beispielsweise 2-Nonensäurenitril; 2-Tridecensäurenitril; 2,12-Tridecensäurenitril; 3,7-Dimethyl-2,6-octadiensäurenitril; 3,7-Dimethyl-6-octensäurenitril;
- Aliphatische Carbonsäuren und deren Ester, wie beispielsweise (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; insbesondere Ethyl-2-trans-4-cis-decadienoat; Methyl-2-octinat; Methyl-2-noninat; Allyl-2-isoamyloxyacetat; Methyl-3,7-dimethyl-2,6-octadienoat; 4-Methyl-2-pentylcrotonat;
- Acyclische Terpenalkohole, wie beispielsweise Citronellol; Geraniol; Nerol; Linalool; Lavandulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol; 2,6-Dimethyl-2,5,7-octatrien-I-ol; sowie deren Formiate, Acetate, Propionate, Isobutyrate, Butyrate, Isovalerianate, Pentanoate, Hexanoate, Crotonate, Tiglinate oder 3-Methyl-2-butenoate;
- Acyclische Terpenaldehyde und -ketone, wie beispielsweise Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal; insbesondere die Dimethyl- und Diethylacetale von Geranial, Neral und 7-Hydroxy-3,7-diemthylactanal;
- Cyclische Terpenalkohole, wie beispielsweise Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate oder 3-Methyl-2-butenoate;
- Cyclische Terpenaldehyde und -ketone, wie beispielsweise Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; beta-Iron; alpha-Damascenon; beta-Damascenon; gamma-Damascenon; delta-Damascenon; gamma-Damascenon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; 2-Methyl-4-(2,6,6-trimethyl-1 -cyclohexen-1-yol)-2-butenal; Nootkaton; Dihydronootkaton; 4,6,8-Metgastigmatrien-3-on; alpha-Sinensal; beta-Sinensal; acetyliertes Cedernholzöl (Methylcedrylketon);
- Cyclische Alkohole, wie beispielsweise 4-tert-Butylcyclohexanol; 3,3,5-Trimethylcyclohexanol; 3-Isocamphylcyclohexanol; 2,6,9-Trimethyl-(Z2,Z5,E9)cyclododecatrien-1-ol; 2-Isobutyl-4-methyltetrahydro-2H-pyran-4-ol; aus der Gruppe der cycloaliphatischen Alkohole wie z. B. 3,3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1 -yl)-pentan-2-ol; 3-Methyl-5-(2,2,3-trimethyl-3-cyclopent-1 -yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
- Cyclische und cycloaliphatische Ether, wie beispielsweise Cineol; Cedrylmethylether; Cyclododecylmethylether; 1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyl-dodecahydronaph-tho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
- Cyclische und macrocyclische Ketone, wie beispielsweise 4-tert-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert-Pentylcyclohexanon; 5-Cyclohexadecen-1-on; 6,7-Dihydro-1,1,2,3,3-pentamethyl-4(5H)-indanon; 8-Cyclohexadecen-1-one; 7-cyclohexadecen-1-on; (7/8)-Cyclohexadecen-1-on; 9-Cycloheptadecen-1-on; Cyclopentadecanon; Cyclohexadecanon;
- Cycloaliphatische Aldehyde, wie beispielsweise 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
- Cycloaliphatische Ketone, wie beispielsweise 1-(3,3-Dimethylcyclohexyl)-4-penten-1-on; 2,2-Dimethyl-1-(2,4-dimethyl-3-cyclohexen-1-yl)-1-1propanon; 1-(5,5-Dimethyl-2-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert-Butyl-(2,4-dimethyl-3-cyclohexen-1-yl)keton;
- Ester cyclischer Alkohole, wie beispielsweise 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat; 2-Cyclopentylcyclopentyl-crotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5- bzw. -6-indenylacetat; 4,7-Methano-3a,4,5,6,7,7a-hexahydro-5- bzw. -6-indenylpropionat; 4,7-Methano-3a,4,5,6,7,7a-hexahydro-5-bzw. -6-indenylisobutyrat; 4,7-Methanooctahydro-5- bzw. -6-indenylacetat;
- Ester cycloaliphatischer Alkohole, wie beispielsweise 1-Cyclohexylethylcrotonat;
- Ester cycloaliphatischer Carbonsäuren, wie beispielsweise Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
- Aromatische Kohlenwasserstoffe, wie beispielsweise Styrol und Diphenylmethan;
- Araliphatische Alkohole, wie beispielsweise Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenyl-ethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
- Ester von araliphatischen Alkoholen und aliphatischen Carbonsäuren, wie z. B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; α-Trichlormethylbenzylacetat; a,a-Dimethylphenylethylacetat; a,a-Dimethylphenylethylbutyrat; Cinnamylacetat; 2-Phenoxyethylisobutyrat; 4-Methoxybenzylacetat;
- Araliphatische Ether, wie beispielsweise 2-Phenylethylmethylether; 2-Phenylethylisoamylether; 2-Phenylethyl-1-ethoxyethylether; Phenylacetaldehyddimethylacetal; Phenylacetaldehyd-diethylacetal; Hydratropaaldehyd-dimethylacetal; Phenylacetaldehyd-glycerinacetal; 2,4,6-Trimethyl-4-phenyl-1,3-dioxane; 4,4a,5,9b-Tetrahydroindeno[1,2-d]-m-dioxin; 4,4a,5,9b-Tetrahydro-2,4-dimethylindeno[1,2-d]-m-dioxin;
- Aromatische und araliphatische Aldehyde, wie beispielsweise Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)-propanal; 2-Methyl-3-(4-tert-butylphenyl)propanal; 3-(4-tert-Butylphenyl)propanal; Zimtaldehyd; a-Butylzimtaldehyd; a-Amylzimtaldehyd; a-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;

- Aromatische und araliphatische Ketone, wie beispielsweise Acetophenon; 4-Methylacetophenon; 4-Methoxyacetophenon; 4-tert-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert-Butyl-1,1-dimethyl-4-indanylmethyl-keton; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
- Aromatische und araliphatische Carbonsäuren und deren Ester, wie z. B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethyl-phenylacetat; Methylcinnamat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
- Stickstoffhaltige aromatische Verbindungen, wie beispielsweise 2,4,6-Trinitro-1,3-dimethyl-5-tert-butylbenzol; 3,5-Dinitro-2,6-dimethyl-4-tert-butylacetophenon; Zimtsäurenitril; 5-Phenyl-3-methyl-2-pentensäurenitril; 5-Phenyl-3-methylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert-butylphenyl)propanal oder 2,4-Dimethyl-3-cyclohexencarbaldehyd; 6-Isopropylchinolin; 6-Isobutylchinolin; 6-sec-Butylchinolin; Indol; Skatol; 2-Methoxy-3-isopropylpyrazin; 2-Isobutyl-3-methoxypyrazin; 4-(4,8-Dimethyl-3,7-nonadienyl)-pyridin;
- Phenole, Phenylether und Phenylester, wie z. B. Estragol; Anethol; Eugenol; Eugenylmethylether; Isöugenol; Isöugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat; aus der Gruppe der heterocyclischen Verbindungen wie z. B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
- Lactone, wie z. B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin;
sowie die Stereoisomere, Enantiomere, Stellungsisomere, Diastereomere, cis/trans-Isomere bzw. Epimere der zuvor genannten Substanzen; und Mischungen aus den vorgenannten Substanzen.

In einer weiteren Variante des erfindungsgemäßen Verfahrens lassen sich auch Aromastoffe als Kernmaterial in Form eines Einzelaromas verkapseln, wobei das Kernmaterial als Wirkstoff mindestens einen Einzelaromastoff oder Mischungen daraus umfasst.

Typische Beispiele für Aromastoffe oder Aromen, die im Sinne der Erfindung verkapselt werden können, sind ausgewählt aus der Gruppe, die besteht aus: Acetophenon; Allylcapronat; alpha-Ionon; beta-Ionon; Anisaldehyd; Anisylacetat; Anisylformiat; Benzaldehyd; Benzothiazol; Benzylacetat; Benzylalkohol; Benzylbenzoat; beta-Ionon; Butylbutyrat; Butylcapronat; Butylidenphthalid; Carvon; Camphen; Caryophyllen; Cineol; Cinnamylacetat; Citral; Citronellol; Citronellal; Citronellylacetat; Cyclohexylacetat; Cymol; Damascon; Decalacton; Dihydrocumarin; Dimethylanthranilat; Dimethylanthranilat; Dodecalacton; Ethoxyethylacetat; Ethylbuttersäure; Ethylbutyrat; Ethylcaprinat; Ethylcapronat; Ethylcrotonat; Ethylfuraneol; Ethylguajakol; Ethylisobutyrat; Ethylisovalerianat; Ethyllactat; Ethylmethylbutyrat; Ethylpropionat; Eucalyptol; Eugenol; Ethylheptylat; 4-(p-Hydroxyphenyl)-2-butanon; gamma-Decalacton; Geraniol; Geranylacetat; Geranylacetat; Grapefruitaldehyd; Methyldihydrojasmonat (z. B. Hedion^{®}); Heliotropin; 2-Heptanon; 3-Heptanon; 4-Heptanon; trans-2-Heptenal; cis-4-Heptenal; trans-2-Hexenal; cis-3-Hexenol; trans-2-Hexensäure; trans-3-Hexensäure; cis-2-Hexenylacetat; cis-3-Hexenylacetat; cis-3-Hexenylcapronat; trans-2-Hexenylcapronat; cis-3-Hexenylformiat; cis-2-Hexylacetat; cis-3-Hexylacetat; trans-2-Hexylacetat; cis-3-Hexylformiat; para-Hydroxybenzylaceton; Isoamylalkohol; Isoamylisovalerianat; Isobutylbutyrat; Isobutyraldehyd; Isoeugenolmethylether; Isopropylmethylthiazol; Laurinsäure; Leavulinsäure; Linalool; Linalooloxid; Linalylacetat; Menthol; Menthofuran; Methylanthranilat; Methylbutanol; Methylbuttersäure; 2-Methylbutylacetat; Methylcapronat; Methylcinnamat; 5-Methylfurfural; 3,2,2-Methylcyclopentenolon; 6,5,2-Methylheptenon; Methyldihydrojasmonat; Methyljasmonat; 2-Methylmethylbutyrat; 2-Methyl-2-Pentenolsäure; Methylthiobutyrat; 3,1-Methylthiohexanol; 3-Methylthiohexylacetat; Nerol; Nerylacetat; trans,trans-2,4-Nonadienal; 2,4-Nonadienol; 2,6-Nonadienol; 2,4-Nonadienol; Nootkaton; delta-Octalacton; gamma-Octalacton; 2-Octanol; 3-Octanol; 1,3-Octenol; 1-Octylacetat; 3-Octylacetat; Palmitinsäure; Paraldehyd; Phellandren; Pentandion; Phenylethylacetat; Phenylethylalkohol; Phenylethylalkohol; Phenylethylisovalerianat; Piperonal; Propionaldehyd; Propylbutyrat; Pulegon; Pulegol; Sinensal; Sulfurol; Terpinen; Terpineol; Terpinolen; 8,3-Thiomenthanon; 4,4,2-Thiomethylpentanon; Thymol; delta-Undecalacton; gamma-Undecalacton; Valencen; Valeriansäure; Vanillin; Acetoin; Ethylvanillin; Ethylvanillinisobutyrat (3-Ethoxy-4-isobutyryloxybenzaldehyd); 2,5-Dimethyl-4-hydroxy-3(2H)-furanon und dessen Abkömmlinge (vorzugsweise Homofuraneol (2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon); Maltol und Maltol-Abkömmlinge (vorzugsweise Ethylmaltol); Cumarin und Cumarin-Abkömmlinge; gamma-Lactone (vorzugsweise gamma-Undecalacton, gamma-Nonalacton, gamma-Decalacton); delta-Lactone (vorzugsweise 4-Methyldeltadecalacton, Massoilacton, Deltadecalacton, Tuberolacton); Methylsorbat; Divanillin; 4-Hydroxy-2(oder 5)-ethyl-5(oder 2)-methyl-3(2H)furanon; 2-Hydroxy-3-methyl-2-cyclopentenon; 3-Hydroxy-4,5-dimethyl-2(5H)-furanon; Essigsäureisoamylester; Buttersäureethylester; Buttersäure-n-butylester; Buttersäureisoamylester; 3-Methyl-buttersäureethylester; n-Hexansäureethylester; n-Hexansäureallylester; n-Hexansäure-n-butylester; n-Octansäureethylester; Ethyl-3-methyl-3-phenylglycidat; Ethyl-2-trans-4-cis-decadienoat; 4-(p-Hydroxyphenyl)-2-butanon; 1,1-Dimethoxy-2,2,5-trimethyl-4-hexan; 2,6-Dimethyl-5-hepten-1-al; Phenylacetaldehyd; 2-Methyl-3-(methylthio)furan; 2-Methyl-3-furanthiol; bis(2-Methyl-3-furyl)disulfid; Furfurylmercaptan; Methional; 2-Acetyl-2-thiazolin; 3-Mercapto-2-pentanon; 2,5-Dimethyl-3-furanthiol; 2,4,5-Trimethylthiazol; 2-Acetylthiazol; 2,4-Dimethyl-5-ethylthiazol; 2-Acetyl-1-pyrrolin; 2-Methyl-3-ethylpyrazin; 2-Ethyl-3,5-dimethylpyrazin; 2-Ethyl-3,6-dimethylpyrazin; 2,3-Diethyl-5-methylpyrazin; 3-Isopropyl-2-methoxypyrazin; 3-Isobutyl-2-methoxypyrazin; 2-Acetylpyrazin; 2-Pentylpyridin; (E,E)-2,4-Decadienal; (E,E)-2,4-Nonadienal; (E)-2-Octenal; (E)-2-Nonenal; 2-Undecenal; 12-Methyltridecanal; 1-Penten-3-on; 4-Hydroxy-2,5-dimethyl-3(2H)-furanon; Guajakol; 3-Hydroxy-4,5-dimethyl-2(5H)-furanon; 3-Hydroxy-4-methyl-5-ethyl-2(5H)-furanon; Zimtaldehyd; Zimtalkohol; Methylsalicylat; Isopulegol sowie die vorliegend nicht explizit genannten Stereoisomere, Enantiomere, Stellungsisomere, Diastereomere, cis/trans-Isomere bzw. Epimere dieser Substanzen; und Mischungen aus den vorgenannten Substanzen.

Von den zuvor genannten Einzel-Duftstoffen, die im Sinne der vorliegenden Erfindung verkapselt werden können, kommen bevorzugt Duftstoffe zum Einsatz, die eine Aldehyd-, Carbonsäure- oder Ester-Funktionalität aufweisen.

Aldehydische Duftstoffe, welche auch die entsprechenden Acetale sowie Ester und Lactone umfassen, lassen sich in die folgenden Gruppen unterteilen, nämlich
(i) aliphatische Aldehyde und deren Acetale;
(ii) cycloaliphatische Aldehyde;
(iii) aromatische oder araliphatische Aldehyde;
(iv) aliphatische, aromatische oder araliphatische Ester; und
(v) Lactone;
sowie jeweils deren Gemische.

Die zuvor genannten Duftstoffe mit Aldehyd-, Carbonsäure- oder Ester-Funktionalität sowie Mischungen daraus sind ausgewählt aus einer oder mehreren der folgenden Gruppen:
- Aliphatische Aldehyde und deren Acetale, wie z. B. Hexanal; Heptanal; Octanal; Nonanal; Decanal; Undecanal; Dodecanal; Tridecanal; 2-Methyloctanal; 2-Methylnonanal; (f)-2-Hexenal; (Z)-4-Heptenal; 2,6-Dimethyl-5-heptenal; 10-Undecenal; (f)-4-Decenal; 2- Dodecenal; 2,6,10-Trimethyl-5,9-undecadienal; Heptanal-diethylacetal; 1,1-Dimethoxy-2,2,5-trimethyl-4-hexen; Citronellyloxyacetaldehyd;
- Cycloaliphatische Aldehyde, wie z. B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
- Aromatische und araliphatische Aldehyde, wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)- propanal; 2-Methyl-3-(4-te/t-butylphenyl)propanal; 3-(4-te/t-Butylphenyl)propanal; Zimtaldehyd; α-Butylzimtaldehyd; α-Amylzimtaldehyd; α-Hexylzimtaldehyd; 3-Methyl-5-phenylpentanal; 4-Methoxybenzaldehyd; 4-Hydroxy-3-methoxybenzaldehyd; 4-Hydroxy-3ethoxybenzaldehyd; 3,4-Methylendioxybenzaldehyd; 3,4-Dimethoxybenzaldehyd; 2-Methyl- 3-(4-methoxyphenyl)-propanal; 2-Methyl-3-(4-methylendioxyphenyl)-propanal;
- Aliphatische Carbonsäureester, wie z. B. (E)- und (Z)-3-Hexenylformiat; Ethylacetoacetat; Isoamylacetat; Hexylacetat; 3,5,5-Trimethylhexylacetat; 3-Methyl-2-butenylacetat; (f)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3- Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;
- Ester cyclischer Alkohole, wie z. B. 2-te/t-Butylcyclohexylacetat; 4-te/t-Butylcyclohexylacetat; 2-ieri-Pentylcyclohexylacetat; 4-te/t-Pentylcyclohexylacetat; Decahydro-2-naphthylacetat; 3-Pentyltetrahydro-2/-/- pyran-4-ylacetat; Decahydro-2,5,5,8a-tetramethyl-2-naphthylacetat; 4,7-Methano-3a,4,5,6,7,7a-hexahydro-5- bzw. -6-indenylacetat; 4,7-Methano-3a,4,5,6,7,7a-hexahydro-5- bzw. -6-indenylpropionat; 4,7-Methano-3a,4,5, 6,7,7a-hexahydro-5- bzw. -6-indenylisobutyrat; 4,7-Methanooctahydro-5- bzw. - 6-indenylacetat;
- Ester von araliphatischen Alkoholen und aliphatischen Carbonsäuren, wie z. B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; a-Trichlormethylbenzylacetat; α,α-Dimethylphenylethylacetat;α,α-Dimethylphenylethylbutyrat; Cinnamylacetat; 2-Phenoxyethylisobutyrat; 4-Methoxybenzylacetat;
- Ester cycloaliphatischer Carbonsäuren, wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6- tetramethyl-2-cyclohexencarboxylat; Ethyl-2-methyl-I,3-dioxolan-2-acetat;
- Aromatische und araliphatische Carbonsäureester, wie z. B. Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnamat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat.

Nachfolgend sind Aldehyde, Acetale, Ester und Lactone mit ihren kommerziellen Bezeichnungen aufgelistet, die im Sinne des erfindungsgemäßen Verfahrens als Vertreter der Gruppen (i) bis (v) besonders bevorzugt sind:
Aldehyde: 2-Methylpentanal; Aldehyd C12 MNA HM; Aldehyde C 4; Aldehyde C 5; Aldehyde C 6; Aldehyde C 7; Aldehyde C 8; Aldehyde C 9; Aldehyde C 10; Aldehyde C 11 ISO; Aldehyde C 11 MOA PURE; Aldehyde C 11 UNDECANAL; Aldehyde C 11 UNDEYLENIC; Aldehyde C 12; ; Aldehyde C 12 MNA; Aldehyde C 13; ALDEHYDE MADARINE; AMYL CINNAMIC ALDEHYDE ALPHA; ANISALALDEHYDE-O; ANISYL ALDEHYDE; BENZALDEHYDE NAT.; BERGAMAL; BORONAL; BOURGENOAL; CAMPHONELIC ALDEHYDE; CITRAL; CITRONELLAL HM; CITRONELLYL OXYACET ALDEHYDE; CITRYLAL; CITROYLAL E HM; CORTEX ALDEHYDE; CORTEX ALDEHYDE 50 PCT PEMOSA; CROTONIC ALDEHYDE; CUMINAL ALDEHYDE; CYCLAMEN ALDEHYDE; DECADIENAL TRANS, TRANS-2,4, DECANAL CIS-4; DECANAL TRANS-2; DECANAL TRANS-2 NAT; DECANAL TRANS-4; DECANAL-9,1; DODECANIENAL 2,6; DODECANAL TRANS-2; DUPICAL; EPOXYDECENAL-4,5-2 10% TRI; ETHYL HEXANAL; FARENAL^{®}; FLORHYDRAL; GERALDEHYDE; HELIONAL; HELIOPAN; HELIOTROPIN; HEPTADIENAL TRANS, TRANS,2-4; HEPTENAL CIS-4; HEPTENAL TRANS-2; HEXENAL TRANS-2; HEXYL CINNAMIC ALDEHYDE ALPHA; HYDRATROPIC ALDEHYDE; HYDROXY CITRONELLAL; INTRELEVEN ALDEHYDE SPEC.; ISONONYL ALDEHYDE; ISOVALERIC ALDEHYDE; LEMON ALDEHYDE H&R JS I; LILIAL; LINOLAL; LYRAL; MAJANTAL; MANDRINAL; MANDRAINE ALDEHYDE 10% IN TEC BHT; MEFRANAL; MELONAL^{®}; METHODY CITRONELLAL; METHYL BUTYRALDEHYDE; METHYL CINNAMIC ALDEHYDE ALPHA; METHYL PHENYLPENTENAL-4,2,2; METHYL THIO PROPANAL-3; METHYL TRIDECANAL-12 10% VT; METHYL-3-BUTEN-2-AL; METHYL-5-PHENYL-2-HEXEN-2-AL; MUGENAL 50 DPG; NEOCYCLO CITRAL; NONADIENAL; TRANS, CIS-2,6; NONENAL CIS-6; NONENAL TRANS-2; ONCIDAL^{®} 3/060251; PENTENAL TRANS-2; PERILLA ALDEHYDE; PHENYLACET ALDEHYDE; PHENYLBUTENAL TRANS-2,2; PHENYLPROPYL ALDEHYDE; PINOACET ALDEHYDE; PROFRANESAL; PROPIONALALDEHYD 2-(P-TOLYL); PROPIONIC ALDEHYDE; PS-IRALDEIN X NEU; SAFRANAL; SALICYLIC ALDEHYDE FG; SILVIAL; TETRAHYDRO CITRAL; TIGLIC ALDEHYDE-2,2; TOLYL ALDEHYDE PARA FG; TRIDECENAL TRANS-2; TRIFERNAL; UNDECADIENAL-2,4; UNDECENAL TRANS-2; VERNALALDEHYDE; VERTOCITRAL; VERTOMUGAL; VERTIPRENAL; VETRAL ROH; ZIMTALDEHYD NAT. HM; Acetale: FLOROPAL; HEPTANAL DIETHYL ACETAL; NONANDIENAL DIETHYL ACETAL; OKOUMAL; PHENYLACET ALD. GLYCERIN ACETAL; PHENYLACETALDEYHDEDIMETHYLACETAL; Ester: JASMAL; JESSEMAL; KHARISMAL; TIRAMISONE^{®}.

In einer alternativen Ausführungsform kommt in den erfindungsgemäßen Polyharnstoff/Polyurethan-Mikrokapseln als zu verkapselnder Wirkstoff bzw. als Kernmaterial eine Duftstoffmischung bzw. ein Parfümöl oder eine Aromastoffmischung bzw. ein Aroma zum Einsatz. Dabei handelt es sich um Zusammensetzungen, die mindestens einen Duftstoff oder einen Aromastoff enthalten, und die zur Herstellung solcher Parfümöle oder Aromen verwendet werden können. Solche Kompositionen, insbesondere Duftstoffmischungen bzw. Parfümöle, umfassen vorzugsweise zwei, drei, vier, fünf, sechs, sieben, acht, neun, zehn oder noch mehr Duftstoffe. Die Duftstoffmischungen bzw. Parfümöle sind vorzugsweise ausgewählt aus der Gruppe, die besteht aus Extrakten natürlicher Rohstoffe; ätherischen Ölen, Concretes, Absolüs, Resine (Harze), Resinoide, Balsame, Tinkturen, wie beispielsweise Ambra-Tinktur; Amyrisöl; Angelikasamenöl; Angelika-wurzelöl; Anisöl; Armoiseöl; Baldrianöl; Basilikumöl; Baummoos Absolüs; Bayöl; Beifussöl; Benzoinharz; Bergamotteöl; Bienenwachs-Absolü; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolü; Castoreum-Absolü; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolü; Eichenmoos-Absolü; Elemiöl; Estragonöl; Eukalyptus-Citriodoraöl; Eukalyptusöl; Fenchelöl; Fichtennadelöl; Tannennadelöl; Galbanumöl; Galbanumresin; Geraniumöl; Grapefruitöl; Guajakholzöl; Gurjunbalsam; Gurjunbalsamöl, Helichrysum-Absolü; Helichrysumöl; Ingweröl; Iriswurzel-Absolü; Iriswurzelöl; Jasmin-Absolü; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolü; Labdanumresin; Lavandin-Absolü; Lavandinöl; Lavendel-Absolü; Lavendelöl; Lemongrasöl; Liebstocköl; Limettenöl destilliert; Limettenöl gepresst; Linalööl; Litsea-Cubeba-öl; Loorbeeröl; Lorbeerblätteröl; Macisöl; Majoranöl; Mandarinenöl; Massoirindenöl; Mimosa-Absolü; Moschuskörneröl; Moschustinktur; Muskateller-Salbei-öl; Muskatnussöl; Myrrhen-Absolü; Myrrhenöl; Myrtenöl; Nelkenblätteröl; Nelkenblütenöl; Neroliöl; Olibanum-Absolü; Olibanumöl; Opopanaxöl; Orangenblüten-Absolü; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolü; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-Tree-öl; Terpentinöl; Thymianöl; Tolubalsam; TonkaAbsolü; Tuberosen-Absolü; Vanilleextrakt; Veilchenblätter-Absolü; Verbenaöl; Vetiveröl; Wacholderbeeröl; Weinhefenöl; Wermutöl; Wintergrünöl; Ylang-Ylang-Öl; Ysopöl; Zibet-Absolü; Zimtblätteröl; Zimtrindenöl; sowie Fraktionen davon bzw. daraus isolierte Inhaltsstoffe.

Beispielhafte Cooling Agents (Kühlmittel), die als liphophile Wirkstoffe bei der Herstellung der erfindungsgemäßen Mikrokapseln verwendet werden, umfassen eines oder mehrere von Menthol und Mentholderivaten (zum Beispiel L-Menthol, D-Menthol, racemisches Menthol, Isomenthol, Neoisomenthol, Neomenthol), Menthylether (zum Beispiel (1-Menthoxy)-2-propandiol, (1-Menthoxy)-2-methyl-1,2-propandiol, 1-Menthylmethylether), Menthylester (zum Beispiel Menthylformiat, Menthylacetat, Menthylisobutyrat, Menthyllactat, L-Menthyl-L-Lactat, L-Menthyl-D-Lactat, Menthyl- (2-methoxy)-acetat, Menthyl-(2-methoxyethoxy)-acetat, Menthylpyroglutamat), Menthylcarbonate (zum Beispiel Menthylpropylenglycolcarbonat, Menthylethylenglycolcarbonat, Menthylglycerincarbonat oder Gemische davon), die Semiester von Menthol mit einer Dicarbonsäure oder deren Derivaten (zum Beispiel Monomenthylsuccinat, Monomenthylglutarat, Monomenthylmalonat, O-Menthylsuccinat-N,N-(dimethyl) amid, O-Menthylsuccinamid), Menthancarboxamide (zum Beispiel Menthancarbonsäure-N-ethylamid [WS3], N-alpha-(methancarbonyl) glycinethylester [WS5], Menthancarbonsäure-N-(4-cyanophenyl)-amid, Menthancarbonsäure-N-(alkoxyalkyl)amid), Menthon und Menthonderivate (zum Beispiel L-Menthonglycerolketal), 2,3-Dimethyl-2-(2-propyl)-butansäurederivate (zum Beispiel 2,3-Dimethyl-2-(2-propyl)-butansäure-N-methylamid [WS23]), Isopulegol oder dessen Ester (1-(-)-Isopulegol, 1-(-)-Isopulegolacetat), Menthanderivate (zum Beispiel p-Menthan-3,8-diol), Cubebol oder synthetische oder natürliche Mischungen, die Cubebol enthalten, Pyrrolidonderivate von Cycloalkyldionderivaten (zum Beispiel 3-Methyl)-2-(1-Pyrrolidinyl)-2-cyclopenten-1-on) oder Tetrahydropyrimidin-2-one (zum Beispiel Icilin oder verwandte Verbindungen, wie in der WO 2004/026840 beschrieben). Weitere Cooling Agents sind Menthol (L-Menthol, D-Menthol, racemisches Menthol, Isomenthol, Neoisomenthol, Neomenthol), L-Menthylmethylether, Menthylformiat, Menthylacetat), Menthon, Isopulegol, L-(-)-Isopulegolacetat) und Cubebol, die einen kühlenden Geschmackseffekt aufweisen. Geeignete Kühlmittel sind auf dem Fachgebiet wohlbekannt und sind beispielsweise in US 2017/216802 (A1), US 2010/273887 (A1), EP 2 033 688 (A2) und EP 1 958 627 (A2) beschrieben.

In einer alternativen Variante kommt in den erfindungsgemäßen Polyharnstoff/Polyurethan-Mikrokapseln als zu verkapselnder Wirkstoff bzw. als Kernmaterial ein TRPV1 oder ein TRPV3-Modulator zum Einsatz. TRPV1 und TRPV3-Modulatoren sind im Stand der Technik bekannt und beziehen sich auf TRP-Kanäle (Transient Receptor Potential channels) der Vanilloid (TRPV)-Unterfamilie. TRPV1-Modulatoren vermitteln einen scharfen Geschmack und das mit Capsaicin und Piperin verbundene heiße Gefühl. Das TRPV3-Protein gehört zur Familie nichtselektiver Kationenkanäle, die in einer Vielzahl von Prozessen funktionieren, einschließlich Temperaturempfindung und Vasoregulation. Der TRPV3-Kanal wird direkt durch verschiedene natürliche Verbindungen wie Carvacrol, Thymol und Eugenol aktiviert. Einige andere Monoterpenoide, die entweder ein Wärmegefühl verursachen oder Hautsensibilisatoren sind, können den Kanal ebenfalls öffnen. Monoterpenoide induzieren auch eine Agonisten-spezifische Desensibilisierung von TRPV3-Kanälen auf eine calciumunabhängige Weise.

In einer weiteren Variante kommt in den erfindungsgemäßen Polyharnstoff/Polyurethan-Mikrokapseln als zu verkapselnder Wirkstoff bzw. als Kernmaterial ein Wirkstoff zum Einsatz, der ausgewählt ist aus der Gruppe, die besteht aus Substanzen, die einen scharfen Geschmack oder eine Wärme oder Hitzeempfindung auf Haut oder Schleimhäuten oder ein Prickel- bzw. Kribbelgefühl im Mund- oder Rachenraum hervorrufen oder Wirkstoffe mit stechender oder beißender oder adstringierender Wirkung.

Die Wärme verursachenden oder scharfen Wirkstoffe sind vorzugsweise ausgewählt aus der Gruppe bestehend aus: Paprikapulver, Chili-Pfeffer-Pulver, Extrakte aus Paprika, Extrakte aus Pfeffer, Extrakte aus Chili-Pfeffer, Extrakte aus Ingwerwurzeln, Extrakte aus Paradieskörnern (Aframomum melegueta), Extrakte aus Parakresse (Jambu-Oleoresin; Spilanthes acmella, bzw. Spilanthes oleracea), Extrakte aus Japanischem Pfeffer (Zanthoxylum piperitum), Extrakte aus Kaempferia galanga, Extrakte aus Alpinia galanga, Extrakte aus Wasserpfeffer (Polygonium hydropiper), Capsaicinoiden, insbesondere Capsaicin, Dihydrocapsaicin oder Nonivamid; Gingerolen, insbesondere Gingerol- [6], Gingerol-[8], oder Gingerol-[10]; Shogaolen, insbesondere Shogaol-[6], Shogaol-[8], Shogaol-[10]; Gingerdionen, insbesondere Gingerdion-[6], Gingerdion-[8] oder Gingerdion-[10]; Paradolen, insbesondere Paradol-[6], Paradol-[8] oder Paradol-[10]; Dehydrogingerdionen, insbesondere Dehydrogingerdion-[6], Dehydrogingerdion-[8] oder Dehydrogingerdion-[10]; Piperin; Piperinderivate; Ethyl-2-(4-hydroxy-3-methoxy-phenyl)acetat und 3-Phenylpropyl-2-(4-hydroxy-3-methoxy-phenyl)acetat und deren Gemische.

Die als stechend oder beißend wahrnehmbaren Wirkstoffe sind vorzugsweise ausgewählt aus der Gruppe bestehend aus: aromatischen Isothiocyanaten, insbesondere Phenylethylisothiocyanat, Allylisothiocyanat, Cyclopropylisothiocyanat, Butylisothiocyanat, 3-Methylthiopropylisothiocyanat, 4-Hydroxybenzylisothiocyanat, 4-Methoxybenzylisothiocyanat und deren Gemische.

Die ein Kribbelgefühl (tingling) hervorrufenden Wirkstoffe sind vorzugsweise ausgewählt aus der Gruppe bestehend aus 2E,4E-Decadiensäure-N-isobutylamid (trans-Pellitorin), insbesondere solche wie beschrieben in der WO 2004/043906; 2E,4Z-Decadiensäure-N-isobutylamid (cis-Pellitorin), insbesondere solche wie beschrieben in der WO 2004/000787; 2Z,4Z-Decadiensäure-N-isobutylamid; 2Z,4E-Decadiensäure-N-isobutylamid; 2E,4E-Decadiensäure-N-([2S]-2-methylbutyl)amid; 2E,4E-Decadiensäure-N-([2S]-2-methylbutyl)amid; 2E,4E-Decadiensäure-N-([2R]-2-methylbutylamid); 2E,4Z- Decadiensäure-N-(2-methylbutyl)amid; 2E,4E-Decadiensäure-N-piperid (Achilleamid); 2E,4E-Decadiensäure-N-piperid (Sarmentin); 2E-Decensäure-N-isobutylamid; 3E-Decensäure-N-isobutylamid; 3E-Nonensäure-N-isobutylamid; 2E,6Z,8E-Decatriensäure-N-isobutylamid (Spilanthol); 2E,6Z,8E-Decatriensäure-N-([2S]-2-methylbutyl)amid (Homospilanthol); 2E,6Z,8E-Decatriensäure-N-([2R]-2-methylbutyl)amid; 2E-Decen-4-insäure-N-isobutylamid; 2Z-Decen-4-insäure-N-isobutylamid; 2E,6Z,8E, 10E-Dodecatetraensäure-N-(2-methylpropyl)amid (alpha-Sanshool); 2E,6Z,8E,10E-Dodecatetraensäure-N-(2-hydroxy-2-methylpropyl)amid (alpha-Hydroxysanshool); 2E,6E,8E,10E-Dodecatetraensäure-N-(2-hydroxy- 2-methylpropyl)amid (gamma-Hydroxysanshool); 2E,4E,8Z,10E, 12E-Tetradecapentaensäure-N-(2-hydroxy-2-methylpropyl)amid (gamma-Hydroxysanshool); 2E,4E,8E,10E,12E-Tetradecapentaensäure-N-(2-hydroxy-2-methylpropyl)-amid (gamma-Hydroxyisosanshool); 2E,4E,8Z,10E,12E-Tetradecapentaensäure-N-(2-methyl-2-propenyl)amid (gamma-Dehydrosanshool); 2E,4E,8Z,10E,12E-Tetradecapentaensäure-N-(2-methylpropyl)amid (gamma-Sanshool); 2E,4E,8Z,11Z-Tetradecatetraensäure-N-(2-hydroxy-2-methylpropyl)amid (Bungeanool); 2E,4E,8Z,11E-Tetradecatetraensäure-N-(2-hydroxy-2-methylpropyl)amid (Isobungeanool); 2E,4E,8Z-Tetradecatriensäure-N-(2-hydroxy-2-methylpropyl)amid (Dihydrobungeanool) und 2E,4E-Tetradecadiensäure-N-(2-hydroxy-2-methylpropyl)amid (Tetrahydrobungeanool) und deren Gemische.

Wirkstoffe mit adstringierender Wirkung sind vorzugsweise ausgewählt aus der Gruppe, bestehend aus: Catechine, insbesondere Epicatechine, Gallocatechine, Epigallocatechine sowie deren jeweiligen Gallussäureester, insbesondere Epigallocatechingallat oder Epicatechingallat, deren Oligomere (Procyanidine, Proanthocyanidine, Prodelphinidine, Procyanirine, Thearubigenine, Theogalline) sowie deren C- und O-Glycoside; Dihydroflavonoide wie Dihydromyricetin, Taxifolin, sowie deren C- und O- Glycoside, Flavonole wie Myricetin, Quercetin sowie deren C- und O- Glycoside wie Quercetrin, Rutin, Gallussäaureester von Kohlenhydraten wie Tannin, Pentagalloylglucose oder deren Reaktionsprodukte wie Elligatannin, Aluminiumsalze, z.B. Alaun, und deren Gemische.Beispielhafte Heating Agents, die als liphophile Wirkstoffe bei der Herstellung der erfindungsgemäßen Mikrokapseln verwendet werden, umfassen

In einer weiteren Variante des erfindungsgemäßen Verfahrens lassen sich auch biogene Prinzipien als Kernmaterial verkapseln, wobei das Kernmaterial mindestens ein biogenes Prinzip oder Mischungen daraus umfasst.

Unter biogenen Prinzipien sind Wirkstoffe mit biologischer Aktivität zu verstehen, beispielsweise Tocopherol, Tocopherolacetat, Tocopherolpalmitat, Ascorbinsäure, Carnotin, Carnosin, Koffein, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, sowie Vitaminkomplexe.

In einer weiteren Variante des erfindungsgemäßen Verfahrens kommen auch Substanzen für Druckbeschichtungen für Papier als zu verkapselnder Wirkstoff bzw. als Kernmaterial zum Einsatz, wie sie beschrieben werden in der US 2800457A, deren diesbezügliche Offenbarung durch die Inbezugnahme in vollem Umfang die vorliegende Beschreibung übernommen wird.

Der Gehalt an lipophilem Wirkstoff oder lipophiler Wirkstoffmischung zur Herstellung der erfindungsgemäßen Mikrokapsel beträgt 90,0 bis 99,9 Gew.-%, vorzugsweise 97,0 bis 99,5 Gew.-%, bezogen auf das Gesamtgewicht der internen nicht-wässrigen Phase.

Der Anteil der einen oder der mehreren Wirkstoff-Komponente(n) zu der internen nicht-wässrigen Phase beträgt vorzugsweise zwischen 50 : 1 und 20 : 1, noch mehr bevorzugt zwischen 40 : 1 und 30 : 1.

Somit ist es möglich, mit dem erfindungsgemäßen Verfahren, eine hohe Beladung der erfindungsgemäßen Mikrokapseln mit Wirkstoff zu erreichen.

Darüber hinaus umfasst der erste Polymerisations- und/oder Vernetzungsschritt (a) des erfindungsgemäßen Verfahrens das Bereitstellen einer externen wässrigen Phase, die mindestens ein Schutzkolloid und optional einen Emulgator umfasst (a2).

Hierzu werden das Schutzkolloid und gegebenenfalls der Emulgator in der externen wässrigen Phase, vorzugsweise einem wässrigen Lösungsmittel, gelöst. Geeignete Lösungsmittel sind Wasser oder Gemische von Wasser mit mindestens einem mit Wasser mischbaren organischen Lösungsmittel. Geeignete organische Lösungsmittel sind beispielsweise Glycerin, 1,2-Propandiol, 1,3-Propandiol, Ethandiol, Diethylenglycol, Triethylenglycol und weitere Analoga. Vorzugsweise ist das Lösungsmittel jedoch Wasser.

Ein Schutzkolloid ist ein Polymersystem, das in Suspension oder Dispersion ein Zusammenklumpen (Agglomeration, Koagulation, Flockung) der emulgierten, suspendierten oder dispergierten Komponenten verhindert. Während der Solvatisierung binden schützende Kolloide große Mengen Wasser und erzeugen in wässrigen Lösungen je nach Konzentration hohe Viskositäten. Das Schutzkolloid lagert sich bei der Herstellung von Öl-in-Wasser-Emulsionen mit seinem hydrophoben Teil an die Primärpartikel an und wendet seinen polaren, d. h. hydrophilen Molekülteil, der wässrigen Phase zu. Durch diese Anlagerung an der Grenzfläche erniedrigt es die Grenzflächenspannung und verhindert die Agglomeration der Primärteilchen. Zudem stabilisiert es die Emulsion und begünstigt dabei die Bildung vergleichsweise kleinerer Tröpfchen und damit auch entsprechender Mikrokapseln.

Im Rahmen des erfindungsgemäßen Verfahrens weist das Schutzkolloid neben den o.g. Eigenschaften auch emulgierende Eigenschaften auf. Bei ausreichenden emulgierenden Eigenschaften des Schutzkolloids, wie beispielsweise Carboxymethylcellulose, säuremodifizierte Stärke, Polyvinylalkohol, Ammoniumderivate des Polyvinylalkohols, Polystyrolsulfonate, Polyvinylpyrollidone, Polyvinylacrylate, kann in dem erfindungsgemäßen Verfahren dadurch vorteilhafterweise sogar auf die Verwendung eines Emulgators verzichtet werden.

Das in dem erfindungsgemäßen Verfahren verwendete Schutzkolloid ist ausgewählt aus der Gruppe, bestehend aus
- Diolen, insbesondere Ethandiol, 1,2-Propandiol, 1,3-Propandiol, 1,2-Butandiol, isomeres Butandiol, 1,2-Pentandiol, 1,2-Hexandiol, 1,2-Octandiol, 1,2-Decandiol, 1,2-Dodecandiol, und
- Polyolen, vorzugsweise Triole, insbesondere Glycerin sowie dessen Ethoxylierungs- und Propoxylierungsprodukte, Trimethylolpropan sowie dessen Ethoxylierungs- und Propoxylierungsprodukte, Polyvinylalkohol (PVOH) und dessen Derivate, insbesondere Ammonium- oder Sulfonat-funktionalisierte Polyvinylalkohole, Polyphenole, vorzugsweise 1,3,5-Trihydroxybenzol, Polysaccharide, insbesondere Glucose, Stärken oder chemisch, mechanisch und/oder enzymatisch modifizierte Stärken, Cellulosederivate wie Hydroxyethylcellulose, insbesondere quaternierte Hydroxyethylcellulose, oder Carboxymethylcellulose,
- Polyvinylpyrrolidon, Maleinsäurevinylcopolymeren, Natriumlignosulfonaten, Maleinsäurenanhydrid/Styrolcopolymeren, Ethylen/Maleinsäureanhydridcopolymeren, Copolymeren von Ethylenoxid, Propylenoxid und Säureester von polyethoxyliertem Sorbit, Natriumdodecylsulfat,
- tierische und pflanzliche Polymere, insbesondere Gummi Arabicum (Typ Senegal und Typ Seyal), Proteine, Gelatine, Olibanumharz, Schellack, Lignin, Chitosan, Saponin
sowie Mischungen aus den vorgenannten Verbindungen.

Bevorzugt umfasst die externe wässrige Phase mindestens ein Schutzkolloid, ausgewählt aus Polyvinylpyrrolidonen, Polyvinylalkoholen und Gemischen davon. Polyvinylpyrrolidone sind besonders bevorzugt. Kommerzielle Standard-Polyvinylpyrrolidone haben Molmassen im Bereich von ca. 2500 bis 750000 g/mol. Besonders bevorzugt wird/werden zur Herstellung der erfindungsgemäßen Mikrokapseln Polyvinylalkohol oder seine Ammonium-Derivate, 1,3,5-Trihydroxybenzol oder Stärken, insbesondere modifizierte Stärken, oder tierische oder pflanzliche Polymere als Schutzkolloid verwendet.

Stärken, insbesondere modifizierte Stärken, oder tierische oder pflanzliche Polymere sind natürlich vorkommende Substanzen, welche biologisch abbaubar sind. In Kombination mit den hierin beschriebenen Polyisocyanaten können mit dem vorliegenden Verfahren somit biobasierte und biologisch abbaubare Kapselhüllen bereitgestellt werden. In dem erfindungsgemäßen Verfahren fungieren die Stärke und die tierischen und pflanzlichen Polymere daher auch als sogenannter Bio-Vernetzer.

Die in dem erfindungsgemäßen Verfahren verwendete Stärke ist ausgewählt aus der Gruppe, bestehend aus Maisstärke, Kartoffelstärke, Roggenstärke, Weizenstärke, Gerstenstärke, Haferstärke, Reisstärke, Erbsenstärke, Tapiokastärke sowie Mischungen daraus.

Bei den chemisch modifizierten Stärken handelt es sich vorzugsweise um säuremodifizierte Stärken, alkalimodifizierte Stärken, oxidierte Stärken, acetylierte Stärken, succinierte Stärken oder ocentylsuccinierte Stärken.

Gemäß der vorliegenden Erfindung können auch Kombinationen von zwei oder mehr verschiedenen Schutzkolloiden zur Herstellung der erfindungsgemäßen Mikrokapsel eingesetzt werden.

Als besonders vorteilhaft hat sich in dem erfindungsgemäßen Verfahren erwiesen, wenn in der externen wässrigen Phase eine Kombination aus einem der o.g. Schutzkolloide mit Stärke als weiterem Schutzkolloid verwendet wird. Durch eine derartige Kombination wird aufgrund der hohen Anzahl der funktionellen Hydroxylgruppen die Emulsion stabilisiert und zum anderen eine Reaktion zwischen dem Schutzkolloid und dem/den Polyisocyanat(en) begünstigt, wobei das Reaktionsgleichgewicht bei der Reaktion der Schutzkolloide mit den Polyisocyanat(en) auf die Seite der Produkte, d.h. der Polyurethane, geschoben wird. Die große Anzahl an funktionellen Hydroxylgruppen bei Stärke ermöglicht darüber hinaus die Ausbildung räumlich besonders ausgeprägter Vernetzungen.

Je nach Anzahl der funktionellen Gruppen und/oder Größe des Schutzkolloids weisen die o.g. Schutzkolloide unterschiedliche Reaktionsgeschwindigkeiten mit den Isocyanat-Gruppen des mindestens einen Polyisocyanats auf. Beispielsweise reagiert Glycerin aufgrund seiner Größe schneller mit den Isocyanat-Gruppen als beispielsweise Stärke. Daher lässt sich durch die Auswahl des Schutzkolloids die Vernetzung des Schutzkolloids mit den Isocyanat-Gruppen des Polyisocyanats steuern.

Als besonders vorteilhafte Kombination hat sich Glycerin mit Stärke oder mit modifizierter Stärke oder die Kombination Glycerin mit quaternierter Hydroxyethylcellulose oder Gummi Arabicum Typ Seyal erwiesen; mit solchen Kombinationen macht man sich die zuvor beschriebenen Eigenschaften beider Schutzkolloide zunutze: hohe Reaktionsgeschwindigkeit des Glycerins auf der einen Seite und Anzahl an polymerisierbaren funktionellen Gruppen der Stärke andererseits.

Die in dem erfindungsgemäßen Verfahren verwendeten Schutzkolloide weisen eine Doppelfunktion auf, indem sie zum einen als Schutzkolloid wirken und somit die Agglomeration der emulgierten, suspendierten oder dispergierten Komponenten verhindern, die anschließend gebildete Emulsion stabilisieren, die Bildung von kleinen Tröpfchen begünstigen und die letztlich gebildete Mikrokapsel-Dispersion stabilisieren.

Zum anderen vernetzt das Schutzkolloid aufgrund von polymerisationsfähigen Eigenschaften, beispielsweise funktionellen Gruppen, insbesondere von OH-Gruppen, mit dem mindestens einen oder den mehreren Polyisocyanat(en) unter Polymerisation. Durch die Vernetzung mit dem mindestens einen Polyisocyanat wird bereits während des Emulgierschrittes (a3) eine Polymerschicht ausgebildet, die zum Aufbau der Kapselwand beiträgt und zum Bestandteil dieser wird.

Überraschend wurde festgestellt, dass sich bereits beim Emulgieren bzw. Suspendieren der internen nicht-wässrigen Phase in der externen wässrigen Phase in Anwesenheit des Schutzkolloids, vorzugsweise eines Polyols, an den Grenzflächen der emulgierten bzw. suspendierten zu verkapselnden hydrophoben Wirkstoff-Öltropfen, die den Kern der erfindungsgemäßen Mikrokapsel bilden und der äußeren externen Phase, durch Grenzflächenpolymerisation eine Polymerisation und/oder Vernetzung in Kernnähe ausbildet. Die Polymerisation und/oder Vernetzung beruht auf der Polyadditionsreaktion des Polyisocyanats mit dem Schutzkolloid, vorzugsweise eines Polyols, unter Ausbildung einer Kapselhülle bzw. Kapselwand aus Polyurethan gemäß der nachfolgenden Formel:

n O=C=N-R¹-N=C=O + n HO-R²-OH- → (-R²-O-CO-NH-R¹-NH-CO-O-)ₙ

Dies zeigt sich durch Gasentwicklung und Freisetzung von Kohlendioxid.

Durch die Ausbildung einer Polymerschicht bereits während des Emulgierschritts werden der/die zu verkapselnden Wirkstoff(e), insbesondere Wirkstoffe mit Aldehyd-, Carbonsäure- oder Ester-Funktionalitäten, geschützt, so dass gegebenenfalls eine Deprotonierung, Oxidation oder Verseifung, insbesondere in dem anschließenden Verfahrensschritt, verhindert oder zumindest minimiert, und damit einhergehend ein Verlust an liphophilen Wirkstoff(en) verringert oder eliminiert werden kann; derartige Abbauprodukte tragen üblicherweise auch zur Instabilität der Emulsion bei.

Das Verhältnis der verwendeten Menge des Schutzkolloids oder der Schutzkolloide zu der wässrigen Phase liegt gemäß der vorliegenden Erfindung vorzugsweise in einem Bereich von 1 : 50 bis 1 : 10, weiter bevorzugt in einem Bereich von 1 : 40 bis 1 : 30.

Das Verhältnis von Schutzkolloid in der externen wässrigen Phase zu Polyisocyanat in der internen nicht-wässrigen Phase liegt in einem Bereich von 1 : 5 bis 1 : 2, vorzugsweise in einem Bereich von 1 : 2 bis 1 : 1.

Die verwendete Menge des Schutzkolloids oder die verwendete Menge einer Kombination von Schutzkolloiden liegt somit in einem Bereich von 1 bis 8 Gew.-%, vorzugsweise in einem Bereich von 2 bis 4 Gew.-%, noch mehr bevorzugt in einem Bereich von 3 bis 4 Gew.-%, bezogen auf das Gesamtgewicht der externen wässrigen Phase.

Das mindestens eine Schutzkolloid kann, muss aber kein Bestandteil der Kapselhülle sein. Insbesondere werden Schutzkolloide mit einer höheren Reaktivität, wie oben beschrieben, schneller oder leichter mit den Isocyanat-Gruppen der Polyisocyanat-Komponente reagieren und somit Polyurethan-Vernetzungseinheiten ausbilden die Teil der Kapselhülle bzw. Kapselwand sind, und zwar in Mengen von 0,1 bis maximal 15 Gew.-%, vorzugsweise jedoch im Bereich von 1 bis 5 Gew.-% und noch mehr bevorzugt von 1,5 bis 3 Gew.-%, bezogen auf das Gewicht der Kapseln.

Um die Bildung einer Emulsion aus interner nicht-wässriger Phase und externer wässriger Phase zu erleichtern, die gebildete Emulsion zu stabilisieren und eine Entmischung der internen nicht-wässrigen (öligen/organischen/hydrophoben) Phase und der externen wässrigen (hydrophilen) Phase zu verhindern, wird in dem erfindungsgemäßen Verfahren der externen wässrigen Phase optional ein Emulgator oder eine Emulgierhilfe zugesetzt. Der Zusatz eines Emulgators erfolgt optional dann, wenn das Schutzkolloid keine oder nur geringe, d.h. nicht ausreichende, emulgierende Eigenschaften aufweist. Wenn ein emulgierendes Schutzkolloid verwendet wird, kann in dem erfindungsgemäßen Verfahren vorteilhafterweise auf die Verwendung eines Emulgators verzichtet werden.

In dem erfindungsgemäßen Verfahren werden als Emulgatoren vorzugsweise O/W-Emulgatoren eingesetzt, die eine homogene Verteilung der Öltröpfchen der internen nicht-wässrigen Phase in der externen wässrigen Phase ermöglichen und die Emulsion stabilisieren. Ähnliches gilt für die Aufmischung von festen, nicht löslichen Wirkstoffen in der externen wässrigen Phase, um die so erhaltene Dispersion zu stabilisieren.

Als Emulgatoren kommen beispielweise nichtionogene Tenside aus mindestens einer der folgenden Gruppen in Betracht:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Rizinusöl und/oder gehärtetes Rizinusöl;
- Anlagerungsprodukte von 15 bis 60 Mol Ethylenoxid an Rizinusöl und/oder gehärtetes Rizinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid, vorzugsweise Cremophor^{®};
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin;
- Mono-, Di- und Trialkylphosphate sowie Mono-, Di- und/oder Tri-PEGalkylphosphate und deren Salze;
- Wollwachsalkohole;
- Polysiloxan-Polyalkyl-Polyether-Copolymere bzw. entsprechende Derivate; Block-Copolymere z.B. Polyethylenglycol-30 Dipolyhydroxystearate; Polymeremulgatoren, z.B. Pemulen-Typen (TR-1,TR-2) von Goodrich oder Cosmedia^{®} SP von Cognis;
- Polyalkylenglycole sowie Glycerincarbonat.

Typische anionische Emulgatoren, die in dem erfindungsgemäßen Verfahren zur Herstellung der Isocyanat-basierten Mikrokapseln verwendet werden können, sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

Weiterhin können in dem erfindungsgemäßen Verfahren zur Herstellung der Isocyanat-basierten Mikrokapseln als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat.

Ebenfalls geeignete Emulgatoren sind ampholytische Tenside. Unter ampholytischen Tensiden werden solche oberflächenaktiven Verbindungen verstanden, die außer einer C8/18-Alkyl- oder Acylgruppe im Molekül mindestens eine freie Amino-Gruppe und mindestens eine -COOH- oder -SO3H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C12/18-Acylsarcosin.

Schließlich kommen auch Kationtenside als Emulgatoren in Betracht, wobei solche vom Typ der Esterquats, vorzugsweise methylquaternierte Difettsäuretriethanolaminester-Salze, quaternierte Hydroxyethylcellulose, modifiziertes Chitosan mit Propylenglykol und quaterniert mit Epichlorhydrin, Distearyldimethylammonium-Chlorid (DSDMAC), Benzalkoniumchlorid, Benzethoniumchlorid, Cetylalkoniumchlorid, Cetylpyridiniumchlorid, Cetyltrimethylammoniumbromid (Cetrimoniumbromid), Dequaliniumchlorid. besonders bevorzugt sind.

Die Emulgatoren können der externen wässrigen Phase in einer Menge von etwa 0,5 bis etwa 10 Gew.-% und vorzugsweise etwa 1 bis etwa 5 Gew.-%, jeweils bezogen auf das Gesamtgewicht der externen wässrigen Phase, zugesetzt werden.

Die wässrige Schutzkolloid-Emulgator-Lösung wird vorzugsweise unter Rühren hergestellt, indem man der externen wässrigen Phase nacheinander das Schutzkolloid und optional den Emulgator oder umgekehrt zusetzt, oder das Schutzkolloid und optional den Emulgator gleichzeitig der externen wässrigen Phase zusetzt.

Es kann vorteilhaft sein, wenn zur Herstellung der Polyharnstoff/Polyurethan-Mikrokapseln gemäß der vorliegenden Erfindung die externe wässrige Phase gegebenenfalls Stabilisatoren entweder gelöst oder dispergiert enthält, um eine Entmischung der internen nicht-wässrigen (öligen) Phase und der externen wässrigen Phase zu verhindern.

Die bevorzugten Stabilisatoren zur Herstellung der Isocyanat-basierten Mikrokapseln gemäß der vorliegenden Erfindung sind vor allem Acrylcopolymere, die über Sulfonatgruppen verfügen. Ebenfalls geeignet sind Copolymere von Acrylamiden und Acrylsäure, Copolymere von Alkylacrylaten und N-Vinylpyrrolidon wie beispielsweise LUVISKOL^{®} K15, K30 oder K90 (BASF); Natriumpolycarboxylate, Natriumpolystyrolsulfonate, Vinyl- und Methylvinylether-Maleinsäureanhydrid-Copolymere sowie Ethylen-, Isobutylen- oder Styrol-Maleinsäureanhydrid-Copolymere, mikrokristalline Cellulose, die beispielweise unter der Bezeichnung VIVAPUR^{®} im Handel ist, Diutan Gum, Xanthan Gum oder Carboxymethylcellulosen.

Die Einsatzmenge der Stabilisatoren kann im Bereich von 0,01 bis 10 Gew.-% und insbesondere im Bereich von 0,1 bis 3 Gew.-%, jeweils bezogen auf die externe wässrige Phase, liegen.

Die Herstellung der Öl-in-Wasser-Emulsion erfolgt durch Vermischen der internen nicht-wässrigen Phase und der externen wässrigen Phase. Das Gewichtsverhältnis von internen nicht-wässriger Phase zu externer wässriger Phase liegt vorzugsweise in einem Bereich von 2 : 1 bis 1 : 10, noch bevorzugter in einem Bereich von 1 : 2 bis 1 : 4.

Die Emulsionsbildung bei flüssigen Wirkstoffen bzw. Dispersionsbildung im Falle fester Wirkstoffe, d. h. das Emulgieren bzw. Dispergieren der internen nichtwässrigen bzw. öligen Phase in der externen wässrigen bzw. hydrophilen Phase erfolgt unter hoher Turbulenz oder starker Scherung. Durch die die Stärke der Turbulenz oder der Scherung kann der Durchmesser der erhaltenen Mikrokapseln bestimmt eingestellt werden. Die Tröpfchengröße kann durch Lichtstreuungsmessungen oder Mikroskopie überprüft werden. Die Herstellung der Mikrokapseln kann dabei kontinuierlich oder diskontinuierlich erfolgen. Mit steigender Viskosität der wässrigen Phase oder mit fallender Viskosität der öligen Phase nimmt in der Regel die Größe der resultierenden Kapseln ab.

Das erfindungsgemäße Verfahren zur Herstellung der Polyharnstoff/Polyurethan-Mikrokapseln kann beispielsweise über eine Zwangsdosierpumpe nach der "Inline"-Technik erfolgen oder aber auch in üblichen Dispersionsapparaturen bzw. Emulgiervorrichtungen unter Rühren.

Das Emulgieren bzw. Dispergieren der internen nicht-wässrigen Phase in der externen wässrigen Phase erfolgte für die Herstellung von erfindungsgemäßen Mikrokapseln mittels einer Emulgierturbine (IKA Eurostar 20 Highspeedrührer). Der Prozess des Emulgierens im Schritt (a3) des erfindungsgemäßen Verfahrens wird vorteilhafter Weise für eine Zeit von 30 Sekunden bis 20 Minuten, vorzugsweise von 1 bis 4 Minuten, bei einer Rührgeschwindigkeit von 1000 U/min bis 5000 U/min, vorzugsweise bei 3000 U/min bis 4000 U/min, durchgeführt.

Nach Abschluss des Emulgier- bzw. Dispergierschrittes (a3) liegt eine Öl-in-Wasser-Emulsion bzw. -Dispersion vor, bei der die interne ölige Phase mit den zu verkapselnden Wirkstoffen in Form von Tröpfchen fein dispergiert oder emulgiert in der externen wässrigen Phase vorliegt.

In einem darauffolgenden Schritt (a4) des erfindungsgemäßen Verfahrens wird ebenfalls unter Rühren eine erste Polymerisation und/oder Vernetzung des Materials der Kapselhülle bzw. Kapselwand durchgeführt. Die erste Vernetzung erfolgt durch Zugabe mindestens einer ersten Aminosäure oder mindestens eines Aminosäure-Hydrochlorids, vorzugsweise in Form einer wässrigen Lösung, in Anwesenheit eines Katalysators. Die Zugabe der Aminosäure oder des Aminosäure-Hydrochlorids und des Katalysators erfolgt vorzugsweise bei einer Temperatur von 20 bis 30 °C.

Die mindestens eine erste Aminosäure ist ausgewählt aus der Gruppe, die besteht aus Arginin, Histidin, Lysin, Tryptophan, Ornithin sowie Mischungen daraus.

Häufig ist es vorteilhaft, die Aminosäure als Hydrochlorid einzusetzen. Die Hydrochloride der vorgenannten Aminosäuren sind leichter in Wasser und damit leichter in der externen wässrigen Phase löslich. Außerdem wird durch die Verwendung der Aminosäure als Hydrochlorid der pH-Wert der Reaktionsmischung ins Saure verschoben, wodurch neben einer verbesserten Löslichkeit auch eine erhöhte Reaktivität zwischen dem mindestens einen Polyisocyanat und der ersten Aminosäure, und damit eine erhöhte Polymerisation und/oder Vernetzung zwischen diesen beiden Komponenten zu erwarten ist.

Das mindestens eine Aminosäure-Hydrochlorid ist ausgewählt aus der Gruppe, die besteht aus Arginin-Hydrochlorid, Histidin-Hydrochlorid, Lysin-Hydrochlorid, Tryptophan-Hydrochlorid, Ornithin-Hydrochlorid und Mischungen daraus.

Bei den Aminosäuren Arginin, Lysin, und Ornithin bzw. den entsprechenden Aminosäure-Hydrochloriden handelt es sich um Verbindungen mit jeweils zwei Amino-Gruppen in der Seitenkette. Die Aminosäuren Histidin und Tryptophan bzw. die entsprechenden Hydrochloride weisen jeweils eine Amino-Gruppe und eine NH-Funktionalität in der Seitenkette auf. Die zuvor genannten Aminosäuren bzw. deren Aminosäuren-Hydrochloride weisen damit eine Multifunktionalität für die Polymerisation mit dem mindestens einen Polyisocyanat auf.

Aufgrund der Vernetzung zwischen den funktionellen Gruppen des mindestens einen Polyisocyanats und der mindestens einen Aminosäure oder des Aminosäure-Hydrochlorids werden erste Vernetzungseinheiten bzw. eine erste Vernetzungsmatrix ausgebildet, die Bestandteil der Kapselhülle bzw. der Kapselwand wrden/wird.

Von den zuvor genannten Aminosäuren ist die basisch reagierende Aminosäure Arginin oder ihr Hydrochlorid-Analog als Vernetzungsmittel besonders bevorzugt wegen seiner Wasserlöslichkeit, seiner hohen Reaktivität und seines pH-Wertes, sowohl als Aminosäure als auch als Hydrochlorid.

Die Verwendung von Aminosäuren bzw. Aminosäure-Hydrochloriden als Vernetzungsmittel sind unter umwelttechnischen Gesichtspunkten in Bezug auf Bioabbaubarkeit und Biokompatibilität besonders vorteilhaft.

Die Aminosäure bzw. das Aminosäure-Hydrochlorid, d.h. das erste Vernetzungsmittel, wird der Emulsion entweder als solches, beispielsweise als Feststoff, oder bevorzugt in Form einer wässrigen Lösung zugesetzt. Die Aminosäure bzw. das Aminosäure-Hydrochlorid ist in der wässrigen Lösung in einer Konzentration von 0,5 bis 2 Mol/l, vorzugsweise von 1 Mol/l, enthalten.

Die Menge an der mindestens einen Aminosäure bzw. dem mindestens einen Aminosäure-Hydrochlorid wird so eingeteilt, dass für jedes Mol Isocyanat-Gruppe 1 bis 3 Mol Amino-Gruppen, vorzugsweise 1 bis 3 Mol Amino-Gruppen, zugegeben werden.

Die erste Vernetzung in dem erfindungsgemäßen Verfahren wird für eine Zeitdauer von etwa 10 Minuten bis 20 Minuten durchgeführt, vorzugsweise für eine Zeitdauer von 12 bis 18 Minuten und am meisten bevorzugt für eine Zeitdauer von etwa 15 Minuten.

Die Menge an der mindestens einen Aminosäure bzw. dem mindestens einen Aminosäure-Hydrochlorid wird typischerweise so eingestellt, dass für jedes Mol Isocyanat-Gruppe 1 bis 3 Mol Amino-Gruppen, vorzugsweise 1 bis 2 Mol Amino-Gruppen, zugegeben werden.

Es ist keine spezifische Wirkung erforderlich, um die Polymerisation zwischen dem mindestens einen Polyisocyanat oder den mehreren Polyisocyanaten und der ersten Aminosäure bzw. dem Aminosäure-Hydrochlorid zu induzieren. Die Reaktion beginnt unmittelbar nach Zugabe der Aminosäure oder des Aminosäure-Hydrochlorids zu der Öl-in-Wasser-Emulsion bzw. -Dispersion unter Ausbildung von ersten Vernetzungseinheiten bzw. einer ersten Vernetzungsmatrix. Da die Reaktion zwischen dem mindesten einen Polyisocyanat oder den mehreren Polyisocyanaten und der Aminosäure bzw. Aminosäure-Hydrochlorid schnell genut ist, erfordert sie keinen Katalysator.

Die Ausbildung der ersten Vernetzungseinheiten in dem erfindungsgemäßen Verfahren beruht auf der Polyadditionsreaktion des Polyisocyanats oder der Polyisocyanate mit der Aminosäure bzw. dem Aminosäure-Hydrochlorid. Die ersten Vernetzungseinheiten, die die Kapselhülle bzw. Kapselwand ausbilden, basieren auf einer Polyharnstoff-Struktur. Die Bildung der Polyharnstoff-Verknüpfung bzw. Polyharnstoff-Struktur erfolgt durch Polyaddition der Amino-Gruppe(n) (-NH₂) der mindestens einen Aminosäure oder des mindestens einen Aminosäure-Hydrochlorids an die Isocyanat-Gruppe des mindestens einen Polyisocyanats:

n O=C=N-R¹-N=C=O + n H₂N-R²- & (-O-NH-R¹-NH-CO-NH-R²-)ₙ

Bei dem erfindungsgemäßen Verfahren bildet sich durch Grenzflächenpolymerisation an der Grenzfläche der emulgierten bzw. suspendierten Öltröpfchen, die den zu verkapselnden lipophilen Wirkstoff umfassen, eine erste Vernetzungsmatrix bzw. erste Vernetzungseinheiten, insbesondere Polyharnstoff-Vernetzungseinheiten, für den Aufbau einer Kapselhülle bzw. Kapselwand aus.

Durch die Ausbildung der ersten Vernetzungsmatrix bzw. von ersten Vernetzungseinheiten werden an der Grenzfläche die emulgierten oder dispergierten Öltröpfchen mit dem Kernmaterial, d. h. den eingekapselten Wirkstoffen, von der Vernetzungsmatrix oder den Vernetzungseinheiten auf der Außenseite umschlossen und so eine Kapselwand generiert, wodurch eine Diffusion des eingekapselten Wirkstoffs erschwert wird.

Die Zugabe eines Katalysators zu der Emulsion bzw. Dispersion beschleunigt die Reaktion zwischen Polyisocyanaten und der Aminosäure bzw. dem Aminosäure-Hydrochlorid und katalysiert die Reaktion zugunsten der Bildung einer Polyharnstoff-Vernetzungsmatrix.

Bei dem Katalysator, der in dem erfindungsgemäßen Verfahren zugegeben wird, handelt es sich vorzugsweise um Diazabicyclo[2.2.2]octan (DABCO), auch Triethylendiamin (TEDA) genannt, ein bicyclisches, tertiäres Amin. DABCO wird im Allgemeinen als Katalysator zur Herstellung von Polyurethan-Kunststoffen verwendet. Das tertiäre Amin mit freien Elektronenpaaren begünstigt die Reaktion zwischen dem mindestens einen polymerisationsfähigen Polyisocyanat in der internen nicht-wässrigen Phase und den Amino-Gruppen der Aminosäure bzw. dem Aminosäure-Hydrochlorid.

Neben DABCO kommen zur Katalyse der ersten Vernetzung beispielsweise auch Katalysatoren auf Bismut- oder Zinn-Basis zum Einsatz, wie beispielsweise Katalysatoren auf Basis von Bismut(II)-Salzen oder Bismut(III)-Salze, wie sie beschrieben sind in K.C. Frisch & L.P. Rumao, Catalysis in Isocyanate Reactions, Polymer Reviews, 1970, 5:1, Seiten 103 - 149, DOI: 10.1080/15583727008085365, deren diesbezügliche Offenbarung in vollem Umfang in die vorliegende Beschreibung übernommen wird.

Besonders bevorzugt ist Diazabicyclo[2.2.2]octan (DABCO) als Katalysator.

Erfindungsgemäß bevorzugt ist eine Kombination aus DABCO und einem der o.g. Katalysatoren. Eine derartige Mischung führt zu einer Multiplikation der Reaktivität, wie dies beschrieben ist in K.C. Frisch & L.P. Rumao, Catalysis in Isocyanate Reactions, Polymer Reviews, 1970, 5:1, Seiten 103 - 149, DOI: 10.1080/15583727008085365, deren diesbezügliche Offenbarung in vollem Umfang in die vorliegende Beschreibung übernommen wird.

DABCO und die zuvor genannten Katalysatoren katalysieren in dem erfindunsgemäßen Verfahren vorzugsweise die Polyurethan-Reaktion zwischen dem mindestens einen polymerisationsfähigen Polyisocyanat mit zwei oder mehreren loscyanat-Gruppen und den Diolen oder Polyolen.

Die Menge, in der der Katalysator der Emulsion bzw. Dispersion zugesetzt wird, liegt in einem Bereich von 0,01 bis 1 Gew.-% und vorzugsweise in einem Bereich von 0,05 bis 0,2 Gew.-%, bezogen auf das Gesamtgewicht der Emulsion bzw. Dispersion. Im Falle einer träge ablaufenden Polymerisationsreaktion kann die benötigte Menge an Katalysator entsprechend angepasst werden.

Das Verhältnis von Katalysator in der Emulsion bzw. Dispersion zu dem mindestens einen Polyisocyanat bzw. Isothiocyanat in der internen nicht-wässrigen Phase vorzugsweise in einem Bereich von 1 : 20 bis 1 : 50.

Es hat sich als vorteilhaft gezeigt, dass der Katalysator zunächst in Wasser dispergiert oder gelöst wird und anschließend der Emulsion bzw. Dispersion unter Rühren zugesetzt wird.

Die Zugabe der Aminosäure bzw. des Aminosäure-Hydrochlorids und des Katalysators erfolgt vorzugsweise bei einer Rührgeschwindigkeit von 500 U/min bis 2000 U/min, besonders bevorzugt bei 1000 U/min bis 1500 U/min und bei Temperaturen von 20 °C bis 30 °C, vorzugsweise bei Temperaturen von 22 °C bis 26 °C.

Die erste Polymerisation und/oder Vernetzung in dem erfindungsgemäßen Verfahren wird für eine Zeitdauer von etwa 10 Minuten bis 20 Minuten durchgeführt, vorzugsweise für eine Zeitdauer von 12 bis 18 Minuten und am meisten bevorzugt für eine Zeitdauer von etwa 15 Minuten.

Überraschend war, dass die Zugabe des Katalysators nach dem Emulgier-oder Suspendierschritt zu einer deutlichen Erhöhung der Kapselstabilität führt. So hergestellte Kapseln weisen eine deutlich höhere Stabilität, selbst nach 10 Tagen bei 50 °C, und eine deutliche Reduzierung des freien Parfümöls gegenüber Vergleichskapseln auf, verglichen mit Mikrokapseln, die ohne die Zugabe eines Katalysators hergestellt wurden.

Besonders stabile Kapseln konnten mit dem Katalysator Diazabicyclo[2.2.2]octan (DABCO) hergestellt werden.

Dem ersten Polymerisations- und/oder Vernetzungsschritt (a) in dem erfindungsgemäßen Verfahren folgt ein weiterer, d.h. zweiter, Polymerisations-und/oder Vernetzungsschritt (b) durch Zugabe mindestens eines Hydroxylgruppen-Donors zu der in Verfahrensschritt (a4) erhaltenen Öl-in-Wasser-Emulsion bzw. - Dispersion, um die Kapselhülle bzw. Kapselwand weiter aufzubauen.

Der mindestens eine Hydroxylgruppen-Donor ist vorzugsweise ein Polyol, das zwei oder mehr funktionelle Hydroxyl-Gruppen, mit guter bis sehr guter Wasserlöslichkeit bei Temperaturen von über 40 °C, aufweist.

Der Hydroxylgruppen-Donor ist ausgewählt aus der Gruppe, die besteht aus Glycerin, Propylenglycol, 1,3,5-Trihydroxybenzol, Stärken, modifizierte Stärken, Cellulosederivate wie Hydroxyethylcellulose, insbesondere quaternierte Hydroxyethylcellulose, oder Carboxymethylcellulose, Gummi Arabicum (Typ Senegal und Typ Seyal) und Mischungen daraus. Bevorzugt sind Glycerin und Stärke; am meisten bevorzugt ist Glycerin.

Die in dem erfindungsgemäßen Verfahren verwendete Stärke ist ausgewählt aus der Gruppe, bestehend aus Maisstärke, Kartoffelstärke, Roggenstärke, Weizenstärke, Gerstenstärke, Haferstärke, Reisstärke, Erbsenstärke, Tapiokastärke sowie Mischungen daraus.

Be den modifizierten Stärken handelt es sich vorzugsweise um chemisch modifizierte Stärke, nämlich säuremodifizierte Stärken, alkalimodifizierte Stärken, oxidierte Stärken, acetylierte Stärken, succinierte Stärken oder ocentylsuccinierte Stärken.

Gemäß der vorliegenden Erfindung kann auch eine Kombination aus zwei verschiedenen der o.g. Hydroxylgruppen-Donoren zur Herstellung der erfindungsgemäßen Mikrokapsel eingesetzt werden.

Je nach Größe weisen die o.g. Hydroxylgruppen-Donoren unterschiedliche Reaktionsgeschwindigkeiten mit den Isocyanat-Gruppen des mindestens einen Polyisocyanats auf. Beispielsweise reagiert Glycerin aufgrund seiner Größe schneller mit den Isocyanat-Gruppen als beispielsweise Stärke.

Als besonders vorteilhafte Kombination hat sich daher Glycerin mit Stärke oder mit modifizierter Stärke oder die Kombination Glycerin mit quaternierter Hydroxyethylcellulose oder Gummi Arabicum Typ Seyal erwiesen; mit solchen Kombinationen macht man sich die zuvor beschriebenen Eigenschaften beider Hydroxylgruppen-Donoren: hohe Reaktionsgeschwindigkeit des Glycerins auf der einen Seite und Anzahl an polymerisierbaren funktionellen Gruppen der Stärke andererseits.

Durch die Reaktion des mindestens einen Polyisocyanats und/oder Isothiocyanats mit den Hydroxylgruppen des Hydroxylgruppen-Donors bildet sich eine weitere, d.h. zweite, Vernetzungsmatrix bzw. zweite Vernetzungseinheiten zum Aufbau bzw. zur Ausbildung der Kapselhülle bzw. Kapselwand, deren Struktur sich analog zu den ersten Vernetzungseinheiten aus Polyisocyanaten und Schutzkolloid, wie dies oben beschrieben wurde, ableiten lassen.

Die Polyadditionsreaktion des mindestens einen Polyisocyanats mit dem Hydroxylgruppen-Donor, resultiert in einer Bildung von sogenannten Urethan-Brücken (-NH-CO-C-) durch Addition der Hydroxylgruppen des Hydroxylgruppen-Donors (-OH) an das Kohlenstoffatom der Kohlenstoff-Stickstoff-Bindung der Polyisocyanat-Gruppen (-N=C=O).

Durch die Ausbildung derartiger weiterer Polyurethan-Vernetzungseinheiten werden die im ersten Polymerisations- und/doer Vernetzungsschritt (a4) gebildeten ersten Polyharnstoff-Vernetzungseinheiten weiter vernetzt und verdichtet.

Um besonders effiziente, dichte und stabile Vernetzungen zu erhalten, erfolgt der zweite Polymerisations- und/oder Vernetzungsschritt (b) mit dem Hydroxylgruppen-Donor bei Temperaturen zwischen 40 °C und 60 °C, und vorzugsweise bei Temperaturen zwischen 45 °C und 55 °C noch bevorzugter bei Temperaturen zwischen 45 °C und 50 °C.

Weiterhin ist es bevorzugt, dass der Schritt der weiteren Vernetzung durch Zugabe des Hydroxylgruppen-Donors bei Rührgeschwindigkeiten von 900 U/min bis 1700 U/min, bevorzugt von 1000 U/min bis 1300 U/min, durchgeführt wird.

Es hat sich in diesem Kontext herausgestellt, dass eine Zugabe des Hydroxylgruppen-Donors in wässriger Form zu besonders stabilen Vernetzungen und daher besonders stabilen Kapselhüllen bzw. Kapselwänden führt. Die Konzentration des Hydroxylgruppen-Donors in der wässrigen Lösung beträgt vorzugsweise 10 % bis 70 % und noch bevorzugter beträgt die Konzentration des Hydroxylgruppen-Donors in der wässrigen Lösung 40 % bis 60 %.

Dem zweiten Vernetzungsschritt (b) in dem erfindungsgemäßen Verfahren folgt ein weiterer, d.h. dritter, Polymerisations- und/oder Vernetzungsschritt (c). In diesem dritten Vernetzungsschritt wird der in Vernetzungsschritt (b) erhaltenen Öl-in-Wasser-Emulsion mindestens eine weitere, d.h. eine zweite, Aminosäure gesetzt.

Die mindestens eine zweite Aminosäure ist ausgewählt aus der Gruppe, die besteht aus Arginin, Histidin, Asparaginsäure, Lysin, Glycin, Alanin, Prolin, Cystein, Glutamin, Leucin, Serin, Tryptophan, Valin, Threonin, Ornithin, Harnsäure sowie Mischungen daraus.

Bei den zuvor genannten Aminosäuren handelt es sich um Verbindungen mit mindestens einer Amino-Gruppe in der Seitenkette und weisen damit eine Funktionalität für die Polymerisation und/oder Vernetzung mit dem mindestens einen Polyisocyanat auf.

Von den zuvor genannten Aminosäuren sind die basisch reagierenden Aminosäuren wie Histidin, Lysin oder Arginin oder ihre Hydrochlorid-Analoga besonders bevorzugt wegen ihrer hohen Reaktivität und ihrer pH-Werte sowohl als Aminosäure als auch als Hydrochlorid.

Besonders bevorzugt wird in dem erfindungsgemäßen Verfahren Arginin wegen seiner Wasserlöslichkeit als Vernetzungsmittel verwendet.

Die Verwendung von Aminosäuren bzw. Aminosäure-Hydrochloriden als Vernetzungsmittel sind unter umwelttechnischen Gesichtspunkten in Bezug auf Bioabbaubarkeit und Biokompatibilität besonders vorteilhaft.

Die zweite Aminosäure wird der Emulsion oder Dispersion entweder als solches, beispielsweise als Feststoff, oder bevorzugt in Form einer wässrigen Lösung zugesetzt. Die zweite ist in der wässrigen Lösung in einer Konzentration von 0,5 bis 2 Mol/l, vorzugsweise von 1 Mol/l, enthalten.

Die Menge an der mindestens einen zweiten Aminosäure bzw. dem Aminosäure-Hydrochlorid wird typischerweise so eingestellt, dass für jedes Mol Isocyanat-Gruppe 1 bis 3 Mol Amino-Gruppen, vorzugsweise 1 bis 2 Mol Amino-Gruppen, zugegeben werden.

Die Zugabe der zweiten Aminosäure bzw. des Aminosäure-Hydrochlorids erfolgt vorzugsweise bei einer Rührgeschwindigkeit von 500 U/min bis 2000 U/min, besonders bevorzugt bei 1000 U/min bis 1500 U/min und bei einer Temperatur von 60 bis 80 °C, vorzugsweise von 60 °C.

Durch die Zugabe einer zweiten Aminosäure werden in dem erfindungsgemäßen Verfahren eine dritte Vernetzungsmatrix oder dritte Vernetzungseinheiten zum Aufbau der Kapselhülle bzw. Kaspselwand ausgebildet. Diese dritten Vernetzungseinheiten beruhen auf der Polyadditionsreaktion einzelner Polymere oder Oligomere des Polyisocyanats oder der Polyisocyanate mit der Aminosäure zur Bildung einer Kapselhülle bzw. Kapselwand basierend auf einer Polyharnstoff-Struktur. Die Bildung der Polyharnstoff-Verknüpfung bzw. Polyharnstoff-Struktur erfolgt durch Polyaddition der Amino-Gruppe(n) (-NH₂) der mindestens einen Aminosäure an die Isocyanat-Gruppe des mindestens einen Polyisocyanats:

n O=C=N-R¹-N=C=O + n H₂N-R²- + → (-O-NH-R¹-NH-CO-NH-R²-)ₙ

Durch die Ausbildung derartiger weiterer Polyharnstoff-Vernetzungseinheiten werden die im ersten und zweiten Polymerisations- und/doer Vernetzungsschritt (a4) und (b) gebildeten Polyharnstoff-Vernetzungseinheiten und Polyurethan-Vernetzungseinheiten weiter vernetzt und verdichtet.

Die dritte Vernetzung in dem erfindungsgemäßen Verfahren wird für eine Zeitdauer von etwa 10 Minuten bis 20 Minuten durchgeführt, vorzugsweise für eine Zeitdauer von 12 bis 18 Minuten und am meisten bevorzugt für eine Zeitdauer von etwa 15 Minuten.

Der dritte Vernetzungsschritt wird bei einer Temperatur von 60 bis 80 °C, vorzugsweise von 60 °C, durchgeführt.

Durch die Kombination aus erstem Polymerisations- bzw. Vernetzungsschritt zwischen Polyisocyanat und einer ersten Aminosäure, zweitem Polymerisations- bzw. Vernetzungsschritt zwischen Polyisocyanat und Hydroxylgruppen-Donor und drittem Polymerisations- bzw. Vernetzungsschritt zwischen Polyisocyanat und einer zweiten Aminosäure lassen sich Polyharnstoff- und Polyurethan-Vernetzungseinheiten bzw. Vernetzungsmatrices generieren, die die Kapselhülle bzw. die Kapselwand aufbauen. Die ersten, zweiten und dritten Polyharnstoff- und Polyurethan-Vernetzungseinheiten werden in dem erfindungsgemäßen Verfahren darüber hinaus durch die sequentiellen Vernetzungsschritte räumlich weiter miteinander und untereinander vernetzt.

Je höher die Anzahl an vernetzenden funktionellen Gruppen, desto größer ist die räumliche Vernetzung und desto stabiler ist die resultierende Kapselhülle bzw. Kapselwand der Mikrokapsel. Neben der Anzahl der funktionellen Gruppen, beeinflusst die Kettenlänge der einzelnen Bausteine die mechanischen Eigenschaften, d. h. die Stabilität, der Kapseln, maßgeblich. Beispielsweise ermöglicht die große Anzahl an Hydroxyl-Gruppen bei Stärke die Ausbildung räumlich besonders ausgeprägter Vernetzungen. Längerkettige Kapselhülle bzw. Kapselwand-Bausteine, beispielsweise Polyisocyanate, führ zur Bildung stabilerer Kapselhüllen bzw. Kapselwände.

Während der oben beschriebenen Vernetzungsschritte wird die Rührleistung zurückgenommen, vorzugsweise auf eine Rührgeschwindigkeit von etwa 800 bis 1200 UpM, um die sich bildenden Vernetzungseinheiten zur Ausbildung der Kapselhülle nicht gleich wieder zu zerschlagen.

Nach dem dritten Polymerisations- bzw. Vernetzungsschritt und der vollständigen Vernetzung und Ausbildung der Kapselhülle bzw. Kapselwand liegen die nach dem erfindungsgemäßen Verfahren hergestellten Kapseln als rohe Mikrokapseln in Form einer wässrigen Dispersion bzw. einer Slurry/Aufschlämmung vor.

Nach der Vernetzung weisen die Mikrokapseln in der Slurry noch eine flexible Hülle auf, die keine sonderliche Stabilität besitzt und daher leicht aufbricht. Zu diesem Zweck wird eine Härtung der Hülle der Mikrokapseln durchgeführt. Die Härtung erfolgt bevorzugt dadurch, dass man die Mikrokapsel-Dispersion schrittweise auf eine Temperatur von mindestens 60 °C, vorzugsweise auf eine Temperatur im Bereich von 60 bis 65 °C, bis maximal zum Siedepunkt der Mikrokapsel-Dispersion anhebt. Die Härtung wird üblicherweise über eine Zeitdauer von mindestens 60 Minuten, vorzugsweise 2 bis 4 Stunden durchgeführt.

Zusätzlich vorteilhaft ist es, der externen wässrigen Phase Substanzen zur Härtung zuzusetzen. Für diesen Zweck werden natürliche pflanzliche Gerbstoffe vom Typ der Tannine eingesetzt, bei denen es sich chemisch betrachtet um Proanthocyanidine handelt, wie sie in dikotylen Stauden, Sträuchern und Blättern besonders in den Tropen und Subtropen zu finden sind. Die Terpene weisen in der Regel molekulare Gewichte im Bereich von 500 bis 3000 KDa auf. Ein bevorzugtes Beispiel für ein geeignetes Tannin ist das Corigallin. Zur Härtung wird eine wässerige Zubereitung der Tannine der die rohen Mikrokapseln enthaltenden wässrigen Dispersion zugegeben. Üblicherweise setzt man die Tannine in Mengen von etwa 0,1 bis etwa 2 Gew.-% und vorzugsweise von etwa 0,5 bis etwa 1,5 Gew.-%, bezogen auf die Mikrokapseln, zu.

Nach dem Aushärtungsschritt (d) des erfindungsgemäßen Verfahrens wird der Mikrokapsel-Dispersion bzw. Mikrokapsel-Slurry in einem weiteren Verfahrensschritt (e) mindestens ein Trennmittel zugesetzt, welches sich auf der Mikrokapselhülle bzw. Mikrokapselwand aufzieht oder vorzugsweise in die Mikrokapselhülle bzw. Mikrokapselwand einlagert.

Bei dem Trennmittel handelt es sich herkömmlicherweise um flüssige oder pastöse Substanzen, die eine Haftung zwischen zwei Materialien verhindern. Im Fall der vorliegenden Erfindung handelt es sich um Substanzen, die sich in die Mikrokapselhülle bzw. Mikrokapselwand einlagern und über funktionelle Gruppen, beispielsweise OH-Gruppen oder COOH-Gruppen, mit den bestehenden Vernetzungsstrukturen des Kapselmaterials ionische oder sogar kovalente Bindungen eingehen.

Das mindestens eine Trennmittel, das in dem erfindungsgemäßen Verfahren verwendet wird, ist ausgewählt aus der Gruppe, die besteht aus Fettsäuren, Fettalkoholen, Fettsäureestern und tierischen und pflanzlichen Wachsen.

Fettsäuren sind aliphatische Monocarbonäsuren mit zumeist unverzweigter Kohlenstoffkette. Fettsäuren unterscheiden sich durch die Anzahl der Kohlenstoffatome (Kettenlänge) sowie - bei ungesättigten Fettsäuren - in der Anzahl und Position von Doppelbindungen. Fettsäuren können aufgrund ihrer Kettenlängen in kurzkettige Fettsäuren (bis 6 bis 8 C-Atome), mittelkettige (8 bis 12 C-Atome) langkettige (13 bis 21 C-Atome) eingeteilt werden.

Fettalkohole sind aliphatische, langkettige, einwertige, meist primäre Alkohole. Die Kohlenwasserstoffreste sind bei nativen Fettalkoholen oft unverzweigt, synthetische Fettalkohole sind häufig auch verzweigt. Die Kohlenstoffkette weist 6 bis 30 Kohlenstoffatome auf und kann auch ein- oder mehrfach ungesättigt sein. Fettalkohole finden sich in natürlichen Wachsen, gebunden als Carbonsäureester, z. B. in Wollwachs oder Walrat, und werden oft Wachsalkohole genannt.

Wachs ist eine organische Verbindung, die bei über etwa 40 °C schmilzt und dann eine Flüssigkeit niedriger Viskosität bildet. Wachse können in ihrer chemischen Zusammensetzung und Herkunft sehr unterschiedlich sein. Hauptkomponenten dieser Stoffgemische sind Ester von Fettsäuren mit langkettigen, aliphatischen primären Alkoholen, den sogenannten Fettalkoholen. Diese Ester unterscheiden sich in ihrem Aufbau von den Fetten und fetten Ölen, die Triglyceride mit Fettsäuren sind. Außerdem enthalten diese Wachse noch freie, langkettige, aliphatische Carbonsäuren, Ketone, Alkohole und Kohlenwasserstoffe. Die Wachse können tierischen oder pflanzlichen Ursprungs sein.

Das mindestens eine Trennmittel in dem erfindungsgemäßen Verfahren ist vorzugsweise ausgewählt aus der Gruppe, die besteht aus:
- langkettigen, aliphatischen, linearen oder verzweigten, gesättigten oder ungesättigten Carbonsäuren mit 12 bis 30 C-Atomen (Fettsäuren), insbesondere Laurinsäure (12:0), Tridecansäure (13:0), Myristinsäure (14:0), Pentadecansäure (15:0), Palmitinsäure (16:0), Margarinsäure (17:0), Stearinsäure (18:0), Nonadecansäure (19:0), Arachinsäure (20:0) Heneicosansäure (21:0), Behensäure (22:0), Lignocerinsäure (24:0), Cerotinsäure (26:0), Montansäure (28:0) und Melissinsäure (30:0); Myristoleinsäure (14:1), Palmitoliensäure (16:1), Margaroleinsäure (17:1), Petroselinsäure (18:1), Ölsäure (18:1), Elaidinsäure (18:1), Vaccensäure (18:1), Gadoleinsäure (20:1), Gondoäsure (20:1), Cetoleinsäure (22:1), Erucasäure 822:1), Nervonsäure (24:1), Linolsäure (18:2), alpha-Linolensäure (18:3), gamma-Linolensäure (18:3), Calendulasäure (18:3), Punicinsäure (18:3), alpha-Eloeostearinsäure (18:3), beta-Eloeostearinsäure (18:3), Stearidonsäure (18:4), Arachidonsäure (20:4), Eicosapentaensäure (20:5), Docosatetraensäure (ADA) (22:4), Docosapentaensäure (DPA-3) (22:5), Docosahexaensäure (22:6) und Tetracosahexaensäure (24:6); Phytansäure;
- langkettigen, aliphatischen, linearen oder verzweigten, gesättigten oder ungesättigten primären Alkoholen mit 12 bis 30 C-Atomen (Fettalkohole), insbesondere Laurylalkohol (12:0), Myristylalkohol (14:0), Palmitylalkohol (16:0), Margarylalkohol (17:0), Stearylalkohol (18:0), Arachidylalkohol (20:0) Behenylalkohol (22:0), Lignocerylalkohol (24:0), Cerylalkohol (26:0), Montanylalkohol (28:0) und Melissylalkohol (30:0); Palmitoleylalkohol (16:1), Oleylalkohol (18:1), Elaedylalkohol (18:1), Linoleylalkohol (18:2), gamma-Linolenylalkohl (18:3);
- Estern von langkettigen, aliphatischen gesättigten Carbonsäuren mit 12 bis 30 C-Atomen mit langkettigen, aliphatischen primären Alkoholen mit 12 bis 30 C-Atomen, insbesondere Laurylpalmitat, Myricylpalmitat, Cetylarachinat und Stearylbehenat;
- tierischen und pflanzlichen Wachsen, insbesondere Wollwachs, Chinawachs, Bienenwachs, Sonnenblumenkernwachs, Reiskleiewachs, Carnaubawachs, Pinovawachs, Rapswachs, Sojawachs, Candelillawachs, Jojobaöl, Korkwachs, Guarumawachs, Baumwollwachs, Flachswachs, Torfwachs, Rosenwachs, Jasminwachs, Peetha-Wachs vom Waschskürbis, sowie Myrtewachs *(Myrica cerifera),* Wachsfeigenwachs, Beerenwachs,
sowie Mischungen aus zwei oder mehreren der vorgenannten Trennmittel.

Von den oben aufgezählten Carbonsäuren sind die gesättigten Fettsäuren bevorzugt. Am meisten bevorzugt in dem erfindungsgemäßen Verfahren ist die Verwendung der oben spezifizierten tierischen und pflanzlichen Wachse aufgrund ihrer niedrigeren Schmelzpunkte, die einen Einbau in die Mikrokapselhülle bzw. Mikrokapselwand erleichtern.

Das mindestens eine Trennmittel weist eine Doppelfunktionalität auf: Die Einlagerung des Trennmittels in die Mikrokapselhülle bzw. Mikrokapselwand und die Ausbildung von ionischen oder kovalenten Bindungen des Trennmittels mit den Vernetzungseinheiten bzw. Vernetzungsmatrices bewirkt zum einen eine weitere Stabilisierung der Mikrokapselhülle. Zum anderen werden durch die Einlagerung des Trennmittels in die Mikrokapselhülle bzw. Mikrokapselwand Sollbruch-Stellen für die Abbaubarkeit der Mikrokapselhülle generiert, d.h. Stellen in der Mikrokapselwand, die so ausgelegt sind, dass dort zuvorderst ein Abbau des Mikrokapselmaterials erfolgt. Dadurch wird die Abbaubarkeit der Mikrokapselhülle bzw. Mikrokapselwand erleichtert. Die Verwendung eines die Mikrokapselhülle bzw. Mikrokapselwand stabilisierenden Trennmittels ermöglicht darüber hinaus eine Reduktion anderer Kapselwandmaterialien, die weniger oder überhaupt nicht biologisch abbaubar sind.

Das Trennmittel wird der Mikrokaspel-Dispersion bzw. Mikrokapsel-Slurry in einer Menge von 1 bis 10 Gew.-%, bezogen auf die Kapselhülle, zugesetzt. Vorzugsweise wird das Trennmittel in einer Menge von 2 bis 5 Gew.-%, bezogen auf die Kapselhülle, zugesetzt.

Das mindestens eine Trennmittel wird der Mikrokapsel-Dispersion bzw. Mikrokapsel-Slurry bei einer Temperatur von mindestens 60 °C bis maximal zum Siedepunkt der Mikrokapsel-Dispersion, vorzugsweise bei einer Temperatur von 80 °C, zugesetzt wird. Bei dieser Temperatur liegt das Trennmittel in einer flüssigen oder geschmolzenen Form vor, so dass es leicht in die bestehenden Vernetzungsstrukturen der Mikrokapselhülle eingeschmolzen und in diese eingelagert werden kann.

Nach der Zugabe des mindestens einen Trennmittels erfolgt ein Schritt des Nach-Härtens der in Schritt (f) erhaltenen Polyharnstoff/Polyurethan-Mikrokapseln, vorzugsweise bei einer Temperatur von mindestens 60 °C bis 100 °C und zwar für eine Zeitdauer von 60 bis 240 Minuten.

Nach der Härtung liegen die nach dem erfindungsgemäßen Verfahren hergestellten Mikrokapseln als Dispersion in Wasser vor, die auch als Mikrokapsel-Dispersion oder Mikrokapsel-Slurry bezeichnet wird. In dieser Form sind die Mikrokapseln grundsätzlich bereits verkaufsfähig.

Um ein Entmischen bzw. Aufrahmen einer solchen Suspension zu verhindern und somit eine hohe Lagerstabilität zu erzielen, hat es sich als vorteilhaft erwiesen, dass die Suspension eine Viskosität von 12 bis 1500 mPas aufweist. Um die gewünschte Viskosität der Suspension zu erhalten wird vorzugsweise ein Verdickungsmittel eingesetzt.

Als Verdickungsmittel werden vorzugsweise Xanthan Gum, Diuthan Gum; Carboxymethylcellulose (CMC), Mikrokristalline Cellulose (MCC) oder Guar Gum verwendet.

Zur Verbesserung der Haltbarkeit wird der Mikrokapsel-Slurry optional ein oder mehrere Konservierungsmittel zugesetzt oder die Mikrokapsel-Slurry wird getrocknet.

Als Konservierungsmittel werden vorzugsweise 1,2-Hexandiol, 1,2-Octandiol oder Parmetol verwendet.

Alternativ dazu werden die Mikrokapseln abgetrennt und zu Konservierungszwecken getrocknet.

Grundsätzlich kommen dafür Verfahren wie die Lyophilisierung in Frage, bevorzugt ist jedoch eine Sprühtrocknung beispielsweise in der Wirbelschicht. Dabei hat es sich als vorteilhaft erwiesen, der Dispersion bei Temperaturen von etwa 20 bis etwa 50 °C und vorzugsweise etwa 40 °C weitere Polysaccharide, vorzugsweise Dextrine und insbesondere Maltodextrine zuzugeben, die den Trockenprozess unterstützen und die Kapseln während dieses Vorgangs schützen. Dabei kann die Einsatzmenge der Polysaccharide etwa 50 bis etwa 150 Gew.-% und vorzugsweise etwa 80 bis etwa 120 Gew.-%, bezogen auf die Kapselmasse, in der Dispersion betragen.

Die Sprühtrocknung selbst kann in konventionellen Sprühanlagen kontinuierlich oder batchweise durchgeführt werden, wobei die Einlasstemperatur bei etwa 170 bis etwa 200 °C und vorzugsweise etwa 180 bis 185 °C liegt und die Austrittstemperatur etwa 70 bis etwa 80 °C und vorzugsweise etwa 72 bis 78 °C beträgt.

Ein für die Einsetzbarkeit der Mikrokapseln wichtiges Kriterium ist das Gewichtsverhältnis von Kernmaterial zu Kapselwandmaterial. Während einerseits ein möglichst hoher Anteil an Kernmaterial angestrebt wird, um einen möglichst hohen Nutzwert der Kapseln zu ermöglichen, ist es auf der anderen Seite notwendig, dass die Kapsel einen noch hinreichenden Anteil an Kapselwandmaterial aufweisen, damit die Stabilität der Kapseln gewährleistet ist.

Erfindungsgemäß hat es ich als besonders vorteilhaft erwiesen, dass die Mikrokapseln so ausgestaltet sind, dass sie ein Gewichtsverhältnis von Kernmaterial zu Kapselwandmaterial aufweisen, das bei 50: 50 bis 90: 10, vorzugsweise bei 70 : 30 bis 80 : 20 liegt.

Die nach dem erfindungsgemäßen Verfahren hergestellten Mikrokapseln lassen sich über den d(0,5)-Wert ihrer Größenverteilung charakterisieren, d. h. dass 50 % der hergestellten Kapseln sind größer, 50 % der Kapseln sind kleiner als dieser Wert.

Die erfindungsgemäßen Mikrokapseln wurden hergestellt aus Hexamethylendiisocyanat und 4,4'-Methyldiphenylendiisocyanat in einem Verhältnis von 75:25. Des Weiteren wurden als erste Aminosäure Lysin*HCl, als Hydroxylgruppen-Donor Glycerin und als zweite Aminosäure Arginin verwendet. Als Katalysator wurde DABCO verwendet und als Schutzkolloid eine modifizierte Stärke; dazu wurde Bienenwachs als Trennmittel gegeben.

Zur Bestimmung der Partikelgröße werden im Rahmen eines dynamischen Prozesses die erfindungsgemäßen Mikrokapseln in Wasser dispergiert und dann die Partikelgröße mittels Laserbeugung bestimmt. Abhängig von der Größe der Kapsel wird der Laserstrahl unterschiedlich gebrochen und kann so zu einer Größe umgerechnet werden. Dafür wurde die Mie-Theorie verwendet. Für die Partikelmessung wurde ein MALVERN Mastersizer 3000 verwendet.

Die erfindungsgemäßen Mikrokapseln sind dadurch gekennzeichnet, dass sie eine Partikelgrößenverteilung bei einem d(0,5)-Wert von 10 µm bis 100 µm, vorzugsweise einen d(0,5)-Wert von 20 µm bis 65 µm aufweisen. Die entsprechenden Partikelgrößenverteilungen von erfindungsgemäßen Mikrokapseln und Mikrokapseln des Standes der Technik sind in Figur 2 veranschaulicht.

Der direkte Vergleich der Mikrokapseln zeigt, dass mit dem erfindungsgemäßen Verfahren genauso gute Mikrokapseln mit derselben Partikelgrößenverteilung wie bei Mikrokapseln des Standes der Technik, basierend auf Polyharnstoff/Polyurethanstrukturen ohne Trennmittel, erzielt werden können.

In Figur 3 sind die IR-Aufnahmen der erfindungsgemäßen Mikrokapseln und von Mikrokapseln des Stands der Technik dargestellt.

Die erfindungsgemäßen Mikrokapseln wurden hergestellt aus Hexamethylendiisocyanat und 4,4'-Methyldiphenylendiisocyanat in einem Verhältnis von 75:25. Des Weiteren wurden als erste Aminosäure Lysin*HCl, als Hydroxylgruppen-Donor Glycerin und als zweite Aminosäure Arginin verwendet. Als Katalysator wurde DABCO verwendet und als Schutzkolloid eine modifizierte Stärke; dazu wurde Bienenwachs als Trennmittel gegeben. Bei den Mikrokapseln des Standes der Technik handelt es sich um Mikrokapseln auf Polyharnstoff/Polyurethan-Basis ohne Trennmittel.

Anhand der Graphik lassen sich deutliche Unterschiede der Banden insbesondere im Fingerprintbereich erkennen. Bei 1050 cm-' ist eine deutlich intensivere Bande im Vergleich zum Stand der Technik zur erkennen, die auf Polyurethane und auch Polyester zurückzuführen ist. Die Polyester stammen zum einen von den Esterbindungen der ocentylsuccinierten Stärke und zum anderen von den Esterbindungen des Trennmittels (beispielsweise von Wachs). Bei 1170 cm-' lässt sich die Bande einem Polyester zuordnen. Die Doppelschwingung im Bereich von 2500 bis 3000 cm-' deutet auf einen erhöhten Anteil an Kohlenstoffketten hin, die von dem Trennmittel resultieren. Der Vergleich der IR-Spektren zeigt darüber hinaus, dass sowohl die erfindungsgemäßen Mikrokapseln als auch die Mikrokapseln des Standes der Technik aus einem Polyharnstoff/Polyurethan-Polymer bestehen.

Das Verfahren gemäß der vorliegenden Erfindung zur Herstellung von Polyharnstoff/Polyurethan-Mikrokapseln zeichnet sich vorteilhafterweise u.a. dadurch aus, dass die einzelnen Vernetzungsschritte pH-Wert unabhängig durchgeführt werden. Damit ist das erfindungsgemäße Verfahren im Vergleich zu Verkapselungsverfahren gemäß dem Stand der Technik einfacher in der Durchführung.

Trotz einfacherer und kürzerer Durchführung lassen sich mit dem Verfahren gemäß der Erfindung Mikrokapseln mit besserer Performance, d.h. ohne Einbuße oder Verlust in der Funktionalität der Mikrokapseln, wie beispielsweise olfaktorische Eigenschaften und positive sekundäre Eigenschaften wie beispielsweise hohe Stabilität, nämlich die Fähigkeit zur Retention des Wirkstoffs, herstellen.

Die pH-Wert unabhängige Durchführung der Vernetzungsschritte erlaubt vorteilhafterweise auch die Verkapselung von lipophilen Wirkstoffen, die eine Aldehyd-, Carbonsäure- oder Ester-Funktionalität aufweisen. Mit Polyisocyanatbasierten Kapselhüllen des Standes der Technik können normalerweise nur ausgewählte Wirkstoffe umhüllt werden. Die Verkapselung mit Polyisocyanaten gemäß dem Stand der Technik eignet sich hingegen nicht für die Verkapselung von Duftstoffen oder Duftölen mit Aldehyd-, Carbonsäure- oder Ester-Funktionalitäten. Bei der Verkapselung bei einem alkalischen pH-Wert werden nämlich Carbonsäuren deprotoniert, oxidierte Aldehyde (Carbonsäuren) analog und Ester verseift, wodurch damit einhergehend ein Verlust der liphophilen Wirkstoffe auftritt und andererseits die erhaltene Emulsion instabil wird. Damit ist der Einsatz einer derartigen Mikroverkapselung hinsichtlich der Wirkstoffe begrenzt und somit beispielsweise nur für ein geringes Spektrum an Duftstoffen bzw. Duftölen geeignet, während derartige Mikroverkapselungs-Verfahren für Duftstoffe oder Duftöle mit Aldehyd-, Carbonsäure-oder Ester-Funktionalitäten ausscheidet. Jedoch zählen gerade Duftstoffe mit Aldehyd-, Carbonsäure- oder Ester-Funktionalitäten zu den wichtigsten Vertretern bei den Duftstoffen bzw. Duftölen. Die pH-Wert unabhängigen Vernetzungsschritte in dem erfindungsgemäßen Verfahren ermöglichen folglich die effektive Verkapselung derartiger lipophiler Parfümstoffe bzw. Parfümöle.

Mit dem erfindungsgemäßen Verfahren ist es möglich, durch Grenzflächenpolymerisation abwechselnd definierte Polyharnstoff- und Polyurethanbasierte Vernetzungseinheiten bzw. Vernetzungsmactrices um den Kern, der den/die lipophilen Wirkstoff(e) umfasst, abzuscheiden und dadurch den Aufbau einer stabilen Kapselwand bzw. Kapselhülle zu erzeugen. Die Hauptkomponenten der Kapselhülle bzw. Kapselwand bilden grundsätzlich Polyharnstoff- oder Polyurethan-Vernetzungsmatrices bzw. -Vernetzungseinheiten. Ferner kann zudem das Schutzkolloid (beispielsweise Stärke) über Polyurethan-Verknüpfungen in der Kapselhülle bzw. Kapselwand vorliegen. Durch die Einlagerung eines Trennmittels in die Kapselhülle bzw. Kapselwand wird die Kapselhülle bzw. Kapselwand weiter stabilisiert.

Weiter zeichnet sich das erfindungsgemäße Verfahren dadurch aus, dass vorzugsweise Schutzkolloid, Aminosäuren, Hydroxylgruppen-Donor als Hauptkomponenten und Polyisocyanate über gezielt katalysierte Mechanismen polymerisiert und/oder vernetzt werden und somit die Herstellung von biobasierenden und biologisch abbaubaren Mikrokapseln auf Basis biokompatibler Polymere ermöglichen. Anders als bei den Mikrokapseln des Standes der Technik, wo die Polyisocyanate einen großen Anteil des Kapselhüll-Materials ausmachen, liegt hier genau das Gegenteil vor: Die Polyisocyanate fungieren bei den erfindungsgemäßen Mikrokapseln nicht mehr als Hauptmaterial, sondern dienen ausschließlich als Vernetzer der Aminosäuren und der anderen o.g. Bestandteile.

Das erfindungsgemäße Verfahren erlaubt somit, einen Teil des Polyisocyanats durch biologisch abbaubare Wandmaterialien wie beispielsweise Schutzkolloid, Aminosäuren, Hydroxylgruppen-Donor und Trennmittel zu ersetzen und damit den Polyisocyanat-Anteil zu senken, ohne dass dadurch eine Einbuße oder ein Verlust in der Funktionalität der Mikrokapseln, wie beispielsweise olfaktorische Eigenschaften und positive sekundäre Eigenschaften wie beispielsweise hohe Stabilität, nämlich die Fähigkeit zur Retention des Wirkstoffs, auftritt. Damit lassen sich mit dem erfindungsgemäßen Verfahren Mikrokapseln herstellen, die einerseits eine ausgezeichnete Funktionalität aufweisen und gleichzeitig biologisch gut abbaubar sind.

Überraschenderweise hat sich herausgestellt, dass sich mit dem erfindungsgemäßen Verfahren Mikrokapseln mit einer verringerten Menge an Polyisocyanat um bis zu 60 % herstellen lassen, ohne dass dadurch eine Einbuße oder ein Verlust der Stabilität der erhaltenen Mikrokapseln auftritt, wie dies in den nachfolgenden Ausführungsbeispielen gezeigt wird. Dadurch können Mikrokapseln mit einer verringerten Menge an Ausgangssubstanz Isocyanat bei gleichbleibender Menge an zu verkapselndem Wirkstoff hergestellt werden.

In einem weiteren Aspekt betrifft die vorliegende Erfindung bioabbaubare Polyharnstoff/Polyurethan-Mikrokapseln, die nach dem erfindungsgemäßen Verfahren herstellt werden.

Die biologisch abbaubaren Polyharnstoff/Polyurethan-Mikrokapseln sind dadurch gekennzeichnet, dass sie aufgebaut sind aus oder umfassen:
(i) einen Kern, der mindestens einen hydrophoben Wirkstoff umfasst; und
(ii) eine Kapselhülle, die umfasst:
   - ein Reaktionsprodukt aus einer Polymerisation und/oder Vernetzung von mindestens einem Polyisocyanat mit zwei oder mehreren Isocyanat-Gruppen, in Anwesenheit mindestens eines Schutzkolloids mit mindestens einer ersten Aminosäure oder eines Aminosäure-Hydrochlorids, einer weiteren Polymerisation und/oder Vernetzung mit mindestens einem Hydroxylgruppen-Donor, und einer weiteren Polymerisation und/oder Vernetzung mit mindestens einer zweiten Aminosäure; und
   - mindestens ein Trennmittel.

Die abwechselnde Polymerisation und/oder Vernetzung von Polyisocyanat-Einheiten mit funktionellen Amino-Gruppen bzw. Hydroxyl-Gruppen resultiert in einer stabilen Kapselwand aus alternierenden definierten und dichten und somit stabilen Vernetzungsmatrices bzw. Vernetzungseinheiten auf Polyurethan- und Polyharnstoff-Basis.

In einer bevorzugten Ausführungsform umfasst oder besteht die Kapselhülle der erfindungsgemäßen bioabbaubaren Polyharnstoff/Polyurethan-Mikrokapsel aus:
(α) eine erste Vernetzungsmatrix bzw. erste Vernetzungseinheiten aus einer Polymerisation und/oder Vernetzung von mindestens einem Polyisocyanat mit zwei oder mehreren Polyisocyanat-Gruppen mit mindestens einem Schutzkolloid und mindestens einer ersten Aminosäure;
(β) eine zweite Vernetzungsmatrix bzw. zweite Vernetzungseinheiten aus einer Vernetzung von mindestens einem Polyisocyanat mit zwei oder mehreren Polyisocyanat-Gruppen mit mindestens einem Hydroxylgruppen-Donor;
(γ) eine dritte Vernetzungsmatrix bzw. dritte Vernetzungseinheiten aus einer Vernetzung von mindestens einem Polyisocyanat mit zwei oder mehreren Polyisocyanat-Gruppen mit mindestens einer zweiten Aminosäure; und
(δ) mindestens ein Trennmittel.

Bei der ersten Vernetzungsmatrix bzw. den ersten Vernetzungseinheiten der Kapselhülle der erfindungsgemäßen Mikrokapsel handelt es sich um ein Netzwerk auf Polyharnstoff-Basis. Bei der zweiten Vernetzungsmatrix bzw. den zweiten Vernetzungseinheiten handelt es sich um ein Netzwerk auf Polyurethan-Basis und bei der dritten Vernetzungsmatrix bzw. den dritten Vernetzungseinheiten handelt es sich um ein weiteres Netzwerk auf Polyharnstoff-Basis. Die Zusammensetzung der Polyharnstoff- und Polyurethan-Vernetzungmatrices bzw. Vernetzungseinheiten ist abhängig von dem eingesetzten Polyisocyanat und dem eingesetzten Vernetzungsmittel, d.h. Schutzkolloid, erste Aminosäure, Hydroxylgruppen-Donor und zweite Aminosäure.

Neben der oben beschriebenen Polyurethan-Bildung und PolyharnstoffBildung entstehen bei den zuvor beschriebenen Vernetzungsschritten aufgrund der Reaktivität der Polyisocyanate Nebenprodukte, beispielsweise Harnstoff, Allophanat, Biuret, Uretidion, Carbodiimid, Uretonimin, etc., wie sie beschrieben sind in M.F. Sonnenschein, Introduction to Polyurethane Chemistry, Polyurethanes: Science, Technology, Markets, and Trends, First Ediiton, 2015, John Wiley & Sons, Seiten 105 bis 126, deren diesbezügliche Offenbarung in vollem Umfang in die vorliegende Beschreibung übernommen wird. Diese Nebenprodukte sind Bestandteil der Kapselhülle bzw. Kapselwand.

Durch den Aufbau der Kapselwand, basierend auf mehreren einzelnen definierten und alternierenden Vernetzungsmatrices bzw. Vernetzungseinheiten, ist es möglich, besonders stabile Mikrokapseln mit hervorragender sensorischer Performance herzustellen, während gleichzeitig eine deutliche Reduzierung der Hüllenbestandteile möglich ist. Schließlich wird die Stabilität der Kapselhülle zudem noch durch die Einlagerung eines Trennmittels begünstigt.

Überraschenderweise weisen Polyharnstoff/Polyurethan-Duftstoffkapseln, welche nach dem erfindungsgemäßen Verfahren hergestellt wurden, eine höhere Stabilität und eine Reduzierung an ungewollt austretendem Parfümöl auf, wie dies in den nachfolgenden Ausführungsbeispielen gezeigt wird, was sich insbesondere auf eine effizientere Verkapselung der Duftstoffe zurückführen lässt.

Überraschenderweise hat sich herausgestellt, dass sich mit dem erfindungsgemäßen Verfahren Mikrokapseln herstellen lassen, die im Vergleich zu den Polyharnstoff/Polyurethan-Mikrokapseln des Standes der Technik eine um den Faktor mindestens 1,5, vorzugsweise mindestens 2, bessere Stabilität ausweisen, wie dies in den nachfolgenden Ausführungsbeispielen veranschaulicht wird.

Die erfindungsgemäßen Mikrokapseln weisen außerdem einen Gehalt an freiem hydrophoben Wirkstoff von 0,5 Gew.-% oder weniger auf, vorzugsweise einen Gehalt von ≤ 0,3 Gew.-% oder weniger, und noch mehr bevorzugt einen Gehalt von ≤ 0,2 Gew.-%.

Die erfindungsgemäßen Polyharnstoff/Polyurethan-Mikrokapseln zeigen darüber hinaus eine deutliche Verbesserung in der sensorischen Performance (Duftstofffreisetzung) gegenüber Kapseln des Standes der Technik, welche sich auf die stabile Wirkstoffverkapselung und die damit verbundenen geringen Wirkstoffverluste zurückführen lassen. Die Mikrokapseln gemäß der Erfindung zeigen daher bei Duftfreisetzung durch Öffnen der Kapseln mittels mechanischer Reibung oder durch Druck eine signifikant höhere sensorische Intensität, wie dies in Figur 5 veranschaulicht ist. Die erfindungsgemäßen Polyharnstoff/Polyurethan-Mikrokapseln zeigen eine deutliche Verbesserung in der sensorischen Performance (Duftstofffreisetzung) gegenüber Kapseln des Standes der Technik um den Faktor mindestens 1,5, vorzugsweise mindestens 1,75, noch mehr bevorzgut um den Faktor mindestens 2.

Mit steigendem Vernetzungsgrad, d.h. mit steigendem Polyisocyanat-Gehalt, erhöht sich die Stabilität der Mikrokapseln, gleichzeitig sinkt aber auch die Fähigkeit zum biologischen Abbau der Kapselhülle, wie dies in den Ausführungsbeispielen veranschaulicht wird. Figur 6 zeigt allgemein die Korrelation zwischen Mikrokapselstabilität, Performance und Biobaubarkeit in Abhängigkeit vom Vernetzungsgrad. Beispielsweise ist bei sehr stabilien Mikrokapseln die Performance, beispielsweise die sensorische Performance, geringer, da die Anzahl der Mikrokapseln, welche durch Reiben, Druck, etc. aufbrechen und die Wirkstoffe freigeben, sinkt.

Die erfindungsgemäßen Polyharnstoff/Polyurethan-Mikrokapseln weisen eine verringerte Menge an Polyisocyanat um bis zu 60 % auf, verglichen mit Polyharnstoff/Polyurethan-Mikrokapseln des Stand des Technik, ohne dass dadurch eine Einbuße oder ein Verlust der Stabilität oder in der Beladung mit zu verkapselndem Wirkstoff der Mikrokapseln auftritt, wie dies in den nachfolgenden Ausführungsbeispielen gezeigt wird. Die Isocyanate fungieren im Gegensatz zu den Mikrokapseln des Standes der Technik nicht mehr als Hauptmaterial der Kapselhülle bzw. Kapselwand, sondern dienen ausschließlich als Vernetzer der Aminosäuren und der anderen Hauptbestandteile der Kapselhülle, wie beispielsweise Schutzkolloid, Hydroxylgruppen-Donor. Der absolute Polyisocyanat-Anteil der hierin beschriebenen Mikrokapseln entspricht lediglich einem 1/60-stel der gesamten Kapsel, welche den oder die Wirkstoffe umfasst. Unter der Annahme, dass die Rohstoffe quantitativ reagieren, kann davon ausgegangen werden, dass bei 100% Wandmaterial genau 1/5 des Wandmaterials aus Polyisocyanat besteht und dieses aufgrund der geringen Menge, in welcher es vertreten sind, lediglich als Vernetzer angesehen werden kann.

Aufgrund des geringeren Polyisocyanat-Anteils in der Kapselhülle bzw. der Kapselwand und der Verwendung eines Trennmittels sind die erfindungsgemäßen Mikrokapseln biologisch besser abbaubar als Kapseln gemäß dem Stand der Technik. Erfindungsgemäße Mikrokapseln, die unter Verwendung eines Trennmittels hergestellt wurden, weisen eine deutlich bessere biologische Abbaubarkeit auf, wie dies in den nachfolgenden Ausführungsbeispielen gezeigt wird.

Die biologische Abbaubarkeit ist das Vermögen organischen Materials, nach einer festgeschriebenen Zeit unter definierten Temperatur-, Sauerstoff- und Feuchtebedingungen in der Anwesenheit von Mikroorganismen oder Pilzen zu Wasser, Kohlendioxid (CO₂) und Biomasse abgebaut zu werden.

Gemäß OECD 301F gilt eine Mikrokapsel als biologisch sofort abbaubar, wenn nach 28 Tagen mehr als 60 % des Wandmaterials abgebaut ist.

Die erfindungsgemäßen Mikrokapseln weisen eine biologische Abbaubarkeit nach OECD 301 F nach 28 Tagen von ≥ 20 % auf, vorzugsweise eine biologische Abbaubarkeit von ≥ 50 %, noch mehr bevorzugt eine biologische Abbaubarkeit von ≥ 70 % und am meisten bevorzugt eine biologische Abbaubarkeit von ≥ 90 %.

Die erfindungsgemäße Mikrokapsel ist darüber hinaus eine universelle Kapsel, mit der nach heutigem Stand ein breites Spektrum an Duft- oder Aromastoffen verkapselt werden kann, selbst Duft- oder Aromastoffen, die eine Aldehyd-, Carbonsäure- oder Ester-Funktionalität aufweisen, so dass keine Restriktionen gegen einzelne Wirkstoffe vorhanden sind.

Aufgrund ihrer vorteilhaften Eigenschaften, insbesondere ihrer Stabilität und der gezielten Freisetzung der Wirkstoffe eignen sich die erfindungsgemäßen Mikrokapseln für ein weites Anwendungsspektrum und insbesondere zur Verwendung in Haushaltsprodukten, Textilpflegeprodukten, Waschmitteln, Weichspülern, Reinigungsmitteln, Scent Boostern, Scent Lotions und Duftverstärkern, Kosmetika, Körperpflegeprodukten, Agrarprodukten, pharmazeutischen Produkten, Druckbeschichtungen für Papier und dergleichen.

Die vorliegende Erfindung betrifft daher in einem weiteren Aspekt die Verwendung der erfindungsgemäßen bioabbaubaren Polyharnstoff/Polyurethan-Mikrokapseln oder einer Dispersion der erfindungsgemäßen Polyharnstoff/Polyurethan-Mikrokapseln zur Herstellung von Haushaltsprodukten, Textilpflegeprodukten, Waschmitteln, Weichspülern, Reinigungsmitteln, Scent Boostern, Scent Lotions oder Duftverstärkern in flüssiger oder fester Form, Kosmetika, Körperpflegeprodukten, Agrarprodukten, pharmazeutischen Produkten, Druckbeschichtungen für Papier und dergleichen.

Schließlich betrifft die vorliegende Erfindung Haushaltsprodukte, Textilpflegepflegprodukte, Waschmittel, Weichspüler, Reinigungsmittel, Scent Booster, Scent Lotions und Duftverstärker, Kosmetika, Körperpflegeprodukte, Agrarprodukte, pharmazeutische Produkte, Druckbeschichtungen für Papier und dergleichen, die die erfindungsgemäßen bioabbaubaren Polyharnstoff/Polyurethan-Mikrokapseln oder eine Dispersion der erfindungsgemäßen Polyharnstoff/Polyurethan-Mikrokapseln umfassen.

### Ausführungsbeispiele

Die erfindungsgemäßen bioabbaubaren Polyharnstoff/Polyurethan-Mikrokapseln und ihre vorteilhaften Eigenschaften werden anhand der nachfolgenden Beispiele näher beschrieben.

### Ausführungsbeispiel Nr. 1 - Wandmaterialien im Vergleich

**Tabelle 1:**

| **Bestandteil** | **Stand der Technik/% *)** | **Erfindungsgemäß *)** |
|---|---|---|
| Isocyanate | 4,8 | 1,8 |
| Schutzkolloid | 0,3 | 2,2 |
| Erste Aminosäure | - | 1,3 |
| Hydroxylgruppendonor | - | 2,7 |
| Zweite Aminosäure/ basisch reagierendes Amin | 1,9 | 0,7 |
| Langkettige Säure/ Alkohole/Ester | - | 3,6 |

| | | |
|---|---|---|
| *) Alle Gehaltsangaben bezogen auf die komplette Mikrokapsel inklusive Öl | | |

Die erfindungsgemäßen Mikrokapseln weisen einen deutlich geringeren Anteil an Polyisocyanat um bis 60 % auf. Die Isocyanate fungieren im Gegensatz zu den Mikrokapseln des Standes der Technik nicht mehr als Hauptmaterial und dienen ausschließlich als Vernetzer der Aminosäuren und anderen Bestandteilen der Kapselmaterialien.

### Ausführungsbeispiel Nr. 2 - Kapselstabilität

Die nachfolgenden Stabilitätsdaten beziehen sich auf einen Test bei 40°C in einer handelsüblichen Formulierung, wie Scent Booster oder Weichspüler.

Als Kapseln des Standes der Technik wurden Kapseln gewählt, deren Kapselwände ausschließlich auf ein Polyharnstoff-Netzwerk zurückzuführen sind. Bei der Herstellung dieser Kapseln wurde in der Regel kein Katalysator verwendet und die Synthese erfolgte bei einem pH-Wert von 9. Als Schutzkolloid wurde Polyvinylalkohol verwendet.

Die freien Öle wurden bestimmt, indem die Slurry in Isopropanol für 30 Sekunden stehen gelassen wurde. Anschließend wurde der Ölgehalt mittels SPME bestimmt.

### Beispiel 1 - Kapselstabilität in Abhängigkeit des Trennmittels

Das Verhalten der freien Öle wurde durch die Variation des Trennmittels bestimmt. Erfindungsgemäße Mikrokapsel wurden hergestellt durch Verwendung eines Isocyanat-Gemisches bestehend aus Hexamethylendiisocyanat und 4,4`-Methyldiphenylendiisocyanat in einem Verhältnis von 75 : 25. Des Weiteren wurden als erste Aminosäure Lysin*HCl, als Hydroxylgruppen-Donor Glycerin und als zweite Aminosäure Arginin verwendet. Als Katalysator wurde DABCO verwendet und als Schutzkolloid eine modifizierte Stärke. Als zu verkapselnde Phase wurde TomCap verwendet. Die verwendeten Wachse sind in der nachfolgenden Tabelle aufgelistet. Die verwendete Stärke liegt in Form eines Succinats vor.

**Tabelle 2:**

| **Wachse** | **Freies Öl/%** |
|---|---|
| Kein Wachs | 0,61 |
| Bienenwachs | 0,09 |
| Carnaubawachs | 0,23 |
| Reiskleiewachs | 0,23 |
| Sonnenblumenkernwachs | 0,27 |

Offensichtlich führt der Einsatz der Wachse zu einer verbesserten Resistenz gegen extraktive Lösungsmittel. Die Kapseln gelten bei einem freien Öl von < 1 % als stabil. Je geringer der Gehalt an freiem Öl ist, umso stabiler ist die Kapsel.

### Beispiel 2 - Kapselstabilität in Abhängigkeit von der Polyisocyanat-Zusammensetzung (Vergleich Einzel-Polyisocyanat und Kombination aus zwei verschiedenen Polyisocyanaten)

Erfindungsgemäße Mikrokapseln wurden unter Verwendung von unterschiedlichen Einzel-Polyisocyanaten oder einer Kombination aus zwei verschiedenen Polyisocyanaten, Guanidiniumcarbonat, Polyvinylalkohol als Schutzkolloid mit TomCap als Parfümöl hergestellt:

**Tabelle 3:**

| **Isocyanate** | **Freies Öl/%** | **3 Tage 50 °C in Weichspüler/%** | **10 Tage 50 °C in Weichspüler/%** |
|---|---|---|---|
| Hexamethylendiisocyanat/ 4, 4'Diphenylmethandiisocyanat 80 : 20 | 0,068 | 80 | 74 |
| Hexamethylendiisocyanat | 0,065 | 43 | 32 |
| Pentamethylendiisocyanat | 0,113 | 72 | 62 |
| 4, 4'Diphenylmethandiisocyanat | 0,111 | 78 | 69 |

Der Einsatz einer Kombination aus zwei verschiedenen Isocyanaten ergab Mikrokapseln, die die Stabilität der Einzelisocyanat-Systeme nochmal übersteigen. Dadurch ist der Einsatz eines Gemisches aus zwei verschiendenen Isocyanaten präferiert.

### Beispiel 3 - Kapselstabilität in Abhängigkeit von der Polyisocyanat-Zusammensetzung (Vergleich aliphatisch-aliphatisches Polyisocyanat-Gemisch und alphatisch-aromatisches Polyisocyanat-Gemisch)

Erfindungsgemäße Mikrokapseln wurden unter Verwendung eines aliphatisch-aliphatischen Polyisocyanat-Gemischs und eines aliphatisch-aromatischen Polyisocyanat-Gemischs wie folgt hergestellt:

Aliphatisch-aliphatisches Polyisocyanat-Gemisch: Pentamethylendiisocyanat und Hexamethylen-diisocyanat in einem Verhältnis von 50 : 50. Aliphatisch-Aromatisches Isocyanat-Gemisch: Hexamethylendiisocyanat und 4,4'-Methyl-diphenylendiisocyanat in einem Verhältnis von 75 : 25.
Des Weiteren wurden als erste Aminosäure Lysin*HCl, als Hydroxylgruppen-Donor Glycerin und als zweite Aminosäure Arginin verwendet. Als Katalysator wurde DABCO verwendet und als Schutzkolloid eine modifizierte Stärke. Als zu verkapselnde Phase wurde TomCap verwendet. Als Wachs wurde Bienenwachs verwendet. Die verwendete Stärke liegt in Form eines Succinats vor.

**Tabelle 4:**

| **Isocyanat-Gemisch** | **Freies Öl/%** |
|---|---|
| aliphatisch-aliphatisch | 0,49 |
| aliphatisch-aromatisch | 0,09 |

Die freien Öle liegen in beiden Proben deutlich unter 1 %, wodurch beide Kapseln als stabil gelten. Die Mikrokapsel aus einem aliphatisch-aliphatischen Polyisocyanat-Gemisch ist genauso stabil, wie eine Mikrokapsel aus einem aliphatisch-aromatischen Polyisocyanat-Gemisch.

### Ausführungsbeispiel Nr. 3 - Bioabbaubarkeit und freies Öl in Bezug auf die Menge Vernetzer (Isocyanate)

Der Gehalt an freiem Öl wurde wie oben beschrieben bestimmt.

Die Bioabbaubarkeit nach OECD 301F wurde wie folgt bestimmt: Abbaubarkeit des Wandmaterials in einem nicht preadaptierten Inokolum, gemessen durch manometrische Respiration (Sauerstoffverbrauch).

**Tabelle 5:**

| **Vernetzer-Menge/%** | **Freies Öl/%** | **Bioabbaubarkeit/% nach OECD 301F innerhalb von 28 Tagen** |
|---|---|---|
| 4,3 | 0,13 | 30 |
| 3,2 | 0,22 | 42 |
| 2,0 | 0,71 | 96 |

Die Bioabbaubarkeit nimmt zu, wenn die Menge an Isocyanat (Vernetzer) abnimmt. Überraschend in diesem Fall ist die sehr geringe Zunahme des freien Öls, wodurch die Kapseln als stabil angenommen werden können.

### Ausführungsbeispiel Nr. 4 - Vergleich Bioabbaubarkeiten mit und ohne Trennmittel

**Tabelle 6:**

| **Bestandteil** | **Mikrokapsel ohne Trennmittel/% *)** | **Mikrokapsel mit Trennmittel/% *)** |
|---|---|---|
| Isocyanate | 1,8 | 1,7 |
| Schutzkolloid | 2,2 | 2,2 |
| Aminosäure | 1,4 | 1,3 |
| Hydroxylgruppendonor | 2,7 | 2,6 |
| Aminosäure | 1,8 | 1,7 |
| Trennmittel | - | 3,5 |
| Biologische Abbaubarkeit Kapselwandmaterial | 6,7 % nach OECD 301F innerhalb von 28 Tagen | 96 % nach OECD 301F innerhalb von 28 Tagen |

| | | |
|---|---|---|
| *) Alle Gehaltsangaben bezogen auf die komplette Mikrokapsel inklusive Öl *) Gehaltsangaben bezogen auf Kapsel inklusive Öl | | |

Die erfindungsgemäßen Kapseln zeigen, dass eine biologische Abbaubarkeit von 96% vorliegt, wodurch das Wandmaterial als sofort biologisch abbaubar angenommen werden kann.

### Ausführungsbeispiel Nr. 6 - Vergleich Bioabbaubarkeit in Bezug auf Natriumsulfat und Toxizitätskontrolle

Mit den erfindungsgemäßen Mikrokapseln von Anwendungsbeispiel Nr. 5 wurde die biologische Abbaubarkeit vom Wandmaterial gemäß OECD 301 F getestet. Dafür wurden als Prozesskontrolle Natriumbenzoat und als Toxizitätskontrolle eine Mischung der erfindungsgemäßen Mikrokapseln und Natriumbenzoat verwendet.
Test replicate 1 und 2 = Erfindungsgemäße Kapsel
Procedure control replicate 1 und 2 = Natriumsulfat
Toxicity control = Kombination aus erfindungsgemäßer Kapsel und Natriumsulfat um
die toxikologische Wirkung zu kontrollieren
Die Testergebnisse sind in Figur 4 gezeigt.

Die erfindungsgemäßen Kapseln zeigen, dass eine biologische Abbaubarkeit von 96% im Mittel von zwei Proben vorliegt, wodurch das Wandmaterial als sofort biologisch abbaubar angenommen werden kann. Die Toxizitätskontrolle zeigt ebenfalls eine Abbaubarkeit, wodurch bewiesen ist, dass das Wandmaterial der erfindungsgemäßen Kapsel nicht persistent ist.

### Ausführungsbeispiel Nr. 7 - Vergleich Bioabbaubarkeiten mit und ohne Wachs

Erfindungsgemäße Mikrokapsel wurden hergestellt durch Verwendung eines Isocyanat-Gemisches bestehend aus Hexamethylendiisocyanat und 4,4`-Methyldiphenylendiisocyanat in einem Verhältnis von 75 : 25. Des Weiteren wurden als erste Aminosäure Lysin*HCl, als Hydroxylgruppen-Donor Glycerin und als zweite Aminosäure Arginin verwendet. Als Katalysator wurde DABCO verwendet und als Schutzkolloid eine modifizierte Stärke. Anschließend erfolgte die Abtrennung des Wandmaterials durch Einsatz einer Zentrifuge, eines Rotationsverdampfers und eines Vakuumtrockenschrankes. Als zu verkapselnde Phase wurde Ethylacetat verwendet. Die verwendeten Wachse werden in der nachfolgenden Tabelle angegeben. Die verwendete Stärke liegt in Form eines Succinats vor.

**Tabelle 7:**

| **Wachse** | **Biologische Abbaubarkeit nach OECD 301 F nach 28 Tagen/%** |
|---|---|
| Kein Wachs | 12 |
| Bienenwachs | 96 |
| Sonnenblumenkernwachs | 72 |

Nach OECD 301F gelten beide Kapseln durch den Einsatz des Wachses als biologisch abbaubar. Der Einsatz der Wachse ist dahingehend überraschend, dass trotz der geringen Menge an Wachs die biologische Abbaubarkeit überproportional ansteigt.

### Ausführungsbeispiel Nr. 8 - Sensorischer Test

Für die sensorische Evaluierung wurden die erfindungsgemäßen Mikrokapseln mit Mikrokapseln aus dem Stand der Technik, d. h. von Mikrokapseln basierend auf Polyharnstoff/Polyurethanstrukturen ohne Trennmittel miteinander verglichen.

Die Mikrokapseln wurden hergestellt aus Hexamethylendiisocyanat und 4,4`-Methyldiphenylendiisocyanat in einem Verhältnis von 75 : 25. Des Weiteren wurden als erste Aminosäure Lysin*HCl, als Hydroxylgruppen-Donor Glycerin und als zweite Aminosäure Arginin verwendet. Als Katalysator wurde DABCO verwendet und als Schutzkolloid eine modifizierte Stärke; dazu wurde Bienenwachs als Trennmittel gegeben.

Die sensorische Evaluierung wurde wie folgt durchgeführt: Die o.g. Mikrokapseln wurden jeweils mit einer Ölkonzentration von 0,2 Gew.-% in einen Weichspüler eingearbeitet und dann verwaschen. Abgerochen wurde auf Mischfaser-Tüchern aus Baumwolle und Polyester.

12 Testpersonen haben auf einer Skala von 1 (kein Geruch) bis 9 (sehr starker Geruch) die Duftintensität von Mischfaser-Tücher nach dem Waschen beurteilt. Die Duftfreigabe erfolgte dabei in drei Schritten. Der erste Schritt beschreibt das Abriechen eines unbehandelten Tuches. Der zweite Schritt beschreibt das Abriechen eines leichtgekneteten Tuches; dafür wurde das Tuch leicht mechanisch belastet, indem es mehrfach zwischen den Händen hin und her bewegt wurde und somit die Kapseln brachen. Der dritte Schritt beschreibt das Abriechen, nachdem die Tücher stark gerieben wurden und somit die Kapseln brachen.

Die erfindungsgemäßen Mikrokapseln haben eine deutlich bessere Performance, wie dies in Figur 5 veranschaulicht ist. Das lässt sich auf die gezielte Modifikation des Systems zurückführen, weil durch die Abscheidung der verschiedenen Polymere der Vernetzungseinheiten und den Einsatz eines Trennmittels eine definierte Hüllstruktur ausgebildet wurde, wodurch die Kapseln besser haften und riechen.

## Patentansprüche

1. Verfahren zur Herstellung einer bioabbaubaren Polyharnstoff/Polyurethan-Mikrokapsel, welches die folgenden Schritte in dieser Reihenfolge umfasst:
(a) Durchführung eines ersten Polymerisations- und/oder Vernetzungsschrittes, umfassend:
(a1) Bereitstellen einer internen nicht-wässrigen Phase, umfassend mindestens ein Polyisocyanat mit zwei oder mehreren Isocyanat-Gruppen und mindestens einen zu verkapselnden lipophilen Wirkstoff;
(a2) Bereitstellen einer externen wässrigen Phase, umfassend mindestens ein Schutzkolloid und optional einen Emulgator;
(a3) Vermischen der internen nicht-wässrigen Phase und der externen wässrigen Phase unter Erhalt einer Öl-in-Wasser-Emulsion;
(a4) Zugabe mindestens einer ersten Aminosäure oder eines Aminosäure-Hydrochlorids und eines Katalysators;
(b) Durchführen eines zweiten Polymerisatons- und/oder Vernetzungsschrittes durch Zugabe mindestens eines Hydroxylgruppen-Donors;
(c) Durchführen eines dritten Polymerisations- und/oder Vernetzungsschrittes durch Zugabe mindestens einer zweiten Aminosäure, insbesondere bei einer Temperatur von mindestens 60 °C, unter Erhalt einer Mikrokapsel-Dispersion;
(d) Aushärten der Mikrokapsel-Dispersion bei einer Temperatur von mindestens 60 °C für eine Zeitdauer von mindestens 60 Minuten;
(e) Zugabe mindestens eines Trennmittels und Einlagerung des Trennmittels in die Mikrokapselhülle;
(f) Nach-Härten der in Schritt (f) erhaltenen Polyharnstoff-Mikrokapsel;
und optional:
(g) Abtrennen der Mikrokapsel von der Mikrokapsel-Dispersion und gegebenenfalls Trocknen der Mikrokapsel.

2. Verfahren nach Anspruch 1, worin das mindestens eine Polyisocyanat mit zwei oder mehreren Isocyanat-Gruppen ausgewählt ist aus der Gruppe, die besteht aus: aliphatischen, cycloaliphatischen, hydroaromatischen, aromatischen oder heterocyclischen Polyisocyanaten deren Substitutionsprodukte sowie Mischungen aus den vorgenannten Verbindungen, insbesondere worin das mindestens eine Polyisocyanat zwei aliphatische Polyisocyanate oder ein aliphatisches und ein aromatisches Polyisocyanat umfasst, insbesondere worin das mindestens eine Polyisocyanat Polyisocyanate in alternierend monomerer, oligomerer oder polymerer Struktur mit unterschiedlicher Kettenlänge umfasst.

3. Verfahren nach einem der vorangehenden Ansprüche 1 oder 2, worin der mindestens eine zu verkapselnde lipophile Wirkstoff ausgewählt ist aus der Gruppe, die besteht aus Duftstoffen, Aromastoffen, Cooling Agents, TRPV1- und TRPV3-Modulatoren, Substanzen, die einen scharfen Geschmack oder eine Wärme oder Hitzeempfindung auf Haut oder Schleimhäuten oder ein Prickel- bzw. Kribbelgefühl im Mund- oder Rachenraum hervorrufen oder Wirkstoffe mit stechender oder beißender oder adstringierender Wirkung, ein Pestizid, ein Biozid, ein Insektizid, eine Substanz aus der Gruppe der Repellentien, ein Lebensmittel-Additiv, ein kosmetischer Wirkstoff, ein pharmazeutischer Wirkstoff, ein Farbstoff, ein Farbstoff-Precursor; eine Agrochemikalie, ein Farbstoff, eine Leuchtfarbe, ein optischer Aufheller, ein Lösungsmittel, ein Wachs, ein Silikonöl, ein Schmierstoffe, Substanzen für eine Druckbeschichtung für Papier, sowie Mischungen aus zwei oder mehreren der vorgenannten Wirkstoffen.

4. Verfahren nach einem der vorangehenden Ansprüche 1 bis 3, worin der mindestens eine zu verkapselnde lipophile Wirkstoff ausgewählt ist aus der Gruppe, die besteht aus Duftstoffen oder Aromastoffen, die eine Aldehyd-, Carbonsäure- oder Ester-Funktionalität aufweisen.

5. Verfahren nach einem der vorangehenden Ansprüche 1 bis 4, worin das Schutzkolloid ausgewählt ist aus der Gruppe, die besteht aus Diolen, insbesondere Ethandiol, 1,2-Propandiol, 1,3-Propandiol, 1,2-Butandiol, isomeres Butandiol, 1,2-Pentandiol, 1,2-Hexandiol, 1,2-Octandiol, 1,2-Decandiol, 1,2-Dodecandiol, und
- Polyolen, vorzugsweise Triole, insbesondere Glycerin sowie dessen Ethoxylierungs- und Propoxylierungsprodukte, Trimethylolpropan sowie dessen Ethoxylierungs- und Propoxylierungsprodukte, Polyvinylalkohol (PVOH) und dessen Derivate, insbesondere Ammonium- oder Sulfonatfunktionalisierte Polyvinylalkohole, Polyphenole, vorzugsweise 1,3,5-Trihydroxybenzol, Polysaccharide, insbesondere Glucose, Stärken oder chemisch, mechanisch und/oder enzymatisch modifizierte Stärken, Cellulosederivate wie Hydroxyethylcellulose, insbesondere quaternierte Hydroxyethylcellulose, oder Carboxymethylcellulose,
- Polyvinylpyrrolidon, Maleinsäurevinylcopolymeren, Natriumlignosulfonaten, Maleinsäurenanhydrid/Styrolcopolymeren, Ethylen/Maleinsäureanhydridcopolymeren, Copolymeren von Ethylenoxid, Propylenoxid und Säureester von polyethoxyliertem Sorbit, Natriumdodecylsulfat,
- tierische und pflanzliche Polymere, insbesondere Gummi Arabicum (Typ Senegal und Typ Seyal), Proteine, Gelatine, Olibanumharz, Schellack, Lignin, Chitosan, Saponin,
sowie Mischungen aus den vorgenannten Verbindungen.

6. Verfahren nach einem der vorangehenden Ansprüche 1 bis 5, worin das Schutzkolloid in Kombination mit Stärke eingesetzt wird.

7. Verfahren nach einem der vorangehenden Ansprüche 1 bis 6, worin die mindestens eine erste Aminosäure ausgewählt ist aus der Gruppe, die besteht aus Arginin, Histidin, Lysin, Tryptophan, Ornithin, Arginin-Hydrochlorid, Histidin-Hydrochlorid, Lysin-Hydrochlorid, Tryptophan-Hydrochlorid, Ornithin-Hydrochlorid und Mischungen daraus.

8. Verfahren nach einem der vorangehenden Ansprüche 1 bis 7, worin der Katalysator ausgewählt ist aus der Gruppe, die besteht aus Diazobicyclo[2.2.2]octan (DABCO), Bismuth-Katalysator, Zinn-Katalysator und Mischungen daraus.

9. Verfahren nach einem der vorangehenden Ansprüche 1 bis 8, worin der Hydroxylgruppen-Donor ein Polyol mit zwei oder mehr funktionellen Hydroxyl-Gruppen ist, insbesondere Glycerin, Propylenglycol, 1,3,5-Trihydroxybenzol, Stärken, modifizierte Stärken, Cellulosederivate wie Hydroxyethylcellulose, insbesondere quaternierte Hydroxyethylcellulose, oder Carboxymethylcellulose, oder Gummi Arabicum (Typ Senegal und Typ Seyal) oder Mischungen daraus.

10. Verfahren nach einem der vorangehenden Ansprüche 1 bis 9, worin die mindestens eine zweite Aminosäure ausgewählt ist aus der Gruppe, die besteht aus Arginin, Histidin, Asparaginsäure, Lysin, Glycin, Alanin, Prolin, Cystein, Glutamin, Leucin, Serin, Tryptophan, Valin, Threonin, Ornithin und Mischungen daraus.

11. Verfahren nach einem der vorangehenden Ansprüche 1 bis 10, worin das mindestens eine Trennmittel ausgewählt ist aus der Gruppe, die besteht aus
- langkettigen, aliphatischen, linearen oder verzweigten, gesättigten oder ungesättigten Carbonsäuren mit 12 bis 30 C-Atomen (Fettsäuren), insbesondere Laurinsäure (12:0), Tridecansäure (13:0), Myristinsäure (14:0), Pentadecansäure (15:0), Palmitinsäure (16:0), Margarinsäure (17:0), Stearinsäure (18:0), Nonadecansäure (19:0), Arachinsäure (20:0) Heneicosansäure (21:0), Behensäure (22:0), Lignocerinsäure (24:0), Cerotinsäure (26:0), Montansäure (28:0) und Melissinsäure (30:0); Myristoleinsäure (14:1), Palmitoliensäure (16:1), Margaroleinsäure (17:1), Petroselinsäure (18:1), Ölsäure (18:1), Elaidinsäure (18:1), Vaccensäure (18:1), Gadoleinsäure (20:1), Gondoäsure (20:1), Cetoleinsäure (22:1), Erucasäure 822:1), Nervonsäure (24:1),
Linolsäure (18:2), alpha-Linolensäure (18:3), gamma-Linolensäure (18:3), Calendulasäure (18:3), Punicinsäure (18:3), alpha-Eloeostearinsäure (18:3), beta-Eloeostearinsäure (18:3), Stearidonsäure (18:4), Arachidonsäure (20:4), Eicosapentaensäure (20:5), Docosatetraensäure (ADA) (22:4), Docosapentaensäure (DPA-3) (22:5), Docosahexaensäure (22:6) und Tetracosahexaensäure (24:6); Phytansäure;
- langkettigen, aliphatischen, linearen oder verzweigten, gesättigten oder ungesättigten primären Alkoholen mit 12 bis 30 C-Atomen (Fettalkohole), insbesondere Laurylalkohol (12:0), Myristylalkohol (14:0), Palmitylalkohol (16:0), Margarylalkohol (17:0), Stearylalkohol (18:0), Arachidylalkohol (20:0) Behenylalkohol (22:0), Lignocerylalkohol (24:0), Cerylalkohol (26:0), Montanylalkohol (28:0) und Melissylalkohol (30:0); Palmitoleylalkohol (16:1), Oleylalkohol (18:1), Elaedylalkohol (18:1), Linoleylalkohol (18:2), gamma-Linolenylalkohl (18:3);
- Estern von langkettigen, aliphatischen gesättigten Carbonsäuren mit 12 bis 30 C-Atomen mit langkettigen, aliphatischen primären Alkoholen mit 12 bis 30 C-Atomen, insbesondere Laurylpalmitat, Myricylpalmitat, Cetylarachinat und Stearylbehenat; und
- tierischen und pflanzlichen Wachsen, insbesondere Wollwachs, Chinawachs, Bienenwachs, Sonnenblumenkernwachs, Reiskleiewachs, Carnaubawachs, Pinovawachs, Rapswachs, Sojawachs, Candelillawachs, Jojobaöl, Korkwachs, Guarumawachs, Baumwollwachs, Flachswachs, Torfwachs, Rosenwachs, Jasminwachs, Peetha-Wachs vom Waschskürbis, sowie Myrtewachs *(Myrica cerifera),* Wachsfeigenwachs, Beerenwachs;
sowie Mischungen aus den vorgenannten Trennmitteln.

12. Bioabbaubare Polyharnstoff/Polyurethan-Mikrokapsel oder Mikrokapsel-Dispersion, erhältlich nach dem Verfahren nach einem oder mehreren der vorangehenden Ansprüche 1 bis 11.

13. Bioabbaubare Polyharnstoff/Polyurethan-Mikrokapsel, umfassend
(i) einen Kern, umfassend mindestens einen hydrophoben Wirkstoff; und
(ii) eine Kapselhülle, umfassend:
- ein Reaktionsprodukt aus einer Polymerisation und/oder Vernetzung von mindestens einem Polyisocyanat mit zwei oder mehreren Isocyanat-Gruppen, in Anwesenheit eines Schutzkolloids mit mindestens einer ersten Aminosäure oder eines Aminosäure-Hydrochlorids, einer weiteren Polymerisation und/oder Vernetzung mit mindestens einem Hydroxylgruppen-Donor, und einer weiteren Polymerisation und/oder Vernetzung mit mindestens einer zweiten Aminosäure; und
- mindestens ein Trennmittel.

14. Bioabbaubare Polyharnstoff/Polyurethan-Mikrokapsel nach Anspruch 13, worin die Kapselhülle umfasst:
(α) eine erste Vernetzungsmatrix bzw. erste Vernetzungseinheiten aus einer Polymerisation und/oder Vernetzung von mindestens einem Polyisocyanat mit zwei oder mehreren Polyisocyanat-Gruppen mit mindestens einem Schutzkolloid und mindestens einer ersten Aminosäure;
(β) eine zweite Vernetzungsmatrix bzw. zweite Vernetzungseinheiten aus einer Vernetzung von mindestens einem Polyisocyanat mit zwei oder mehreren Polyisocyanat-Gruppen mit mindestens einem Hydroxylgruppen-Donor;
(γ) eine dritte Vernetzungsmatrix bzw. dritte Vernetzungseinheiten aus einer Vernetzung von mindestens einem Polyisocyanat mit zwei oder mehreren Polyisocyanat-Gruppen mit mindestens einer zweiten Aminosäure; und
(δ) mindestens ein Trennmittel.

15. Bioabbaubare Polyharnstoff/Polyurethan-Mikrokapsel nach einem der Ansprüche 12 bis 14, die eine biologische Abbaubarkeit nach OECD 301 F nach 28 Tagen von ≥ 20 % aufweist und/oder einen Gehalt an freiem hydrophoben Wirkstoff von ≤ 0,3 % Gew.-% aufweist.

16. Verwendung der bioabbaubaren Polyharnstoff/Polyurethan-Mikrokapsel nach einem der Ansprüche 12 bis 15 oder der Polyharnstoff/Polyurethan-Mikrokapsel-Dispersion nach einem der Ansprüche 12 bis 15 zur Herstellung von Haushaltsprodukten, Textilpflegeprodukten, Waschmitteln, Weichspülern, Reinigungsmitteln, Scent Boostern, Scent Lotions oder Duftverstärkern in flüssiger oder fester Form, Kosmetika, Körperpflegeprodukten, Parfümzusammensetzungen, Agrarprodukten pharmazeutischen Produkten oder Druckbeschichtungen für Papier.

17. Haushaltsprodukte, Textilpflegeprodukte, Waschmittel, Weichspüler, Reinigungsmittel, Scent Booster, Scent Lotions und Duftverstärker, Kosmetika, Körperpflegeprodukte, Parfümzusammensetzungen, Agrarprodukte, pharmazeutische Produkte oder Druckbeschichtungen für Papier, umfassend bioabbaubare Polyharnstoff/Polyurethan-Mikrokapsel nach einem der Ansprüche 12 bis 15 oder eine Polyharnstoff/Polyurethan-Mikrokapsel-Dispersion nach einem der Ansprüche 12 bis 15.
